# EUROPEAN PATENT APPLICATION

(11) **EP 3 447 135 A1**
(43) Date of publication of application: **27.02.2019**
(21) Application number: 17206305.9
(22) Date of filing: 08.12.2017
(51) Int. Cl.: C12N 9/12, C12N 15/82, A01H 5/10

(54) **GENE CONFERRING RESISTANCE TO FUNGAL PATHOGEN**

(30) Priority: 22.08.2017 EP 17187309
(71) Applicant: KWS SAAT SE, 37574 Einbeck (DE); Universität Zürich, 8006 Zürich (CH)
(72) Inventor: Kessel, Bettina, 37574 Einbeck (DE); Scheuermann, Daniela, 37574 Einbeck (DE); Presterl, Thomas, 37574 Einbeck (DE); Ouzunova, Milena, 3707 Göttingen (DE); Keller, Beat, 8006 Zürich (CH); Krattinger, Simon, 8906 Bonstetten (CH); Yang, Ping, 8008 Zurich (CH); Herren, Gerhard, 8625 Gossau (CH); Wicker, Thomas, 8536 Hüttwilen (CH)

(57) **Abstract**

The present invention relates to a nucleic acid molecule encoding a polypeptide conferring resistance to a plant against a fungal pathogen, such as *Helminthosporium turcicum.* The present invention further relates to a plant (or part thereof) comprising the nucleic acid molecule, and methods involving the nucleic acid molecule.

## Description

### Field of the invention

The present invention relates to a nucleic acid molecule encoding a polypeptide conferring resistance to a plant against a fungal pathogen, such as *Helminthosporium turcicum.* The present invention further relates to a plant (or part thereof) comprising the nucleic acid molecule, and methods involving the nucleic acid molecule.

### Background of the invention

In maize (*Zea mays* L.), there are many fungal pathogens which cause leaf diseases. The fungus which can cause by far the most damage under tropical and also under temperate climatic conditions, such as those in large parts of Europe and North America as well as in Africa and India, is known as *Helminthosporium turcicum* or synonymously as *Exserohilum turcicum* (teleomorph: *Setosphaeria turcica*). *H. turcicum* is the cause of the leaf spot disease known as "Northern Corn Leaf Blight" (NCLB), which can occur in epidemic proportions during wet years, attacking vulnerable maize varieties and causing a great deal of damage and considerable losses of yield of 30% and more over wide areas. Since the 1970s, then, natural resistance in genetic material has been sought. Currently, quantitative and qualitative resistances, even if incomplete, are known. While the oligo- or polygenically inherited quantitative resistance appears incomplete and non-specific as regards race in the phenotype and is influenced by additional and partially dominant genes, qualitative resistance seems to be typically race-specific and can be inherited through individual, mostly dominant genes at loci like *HT1, HT2, HT3, Htm1* or *HTN1* (Lipps et al., 1997, "Interaction of Ht and partial resistance to Exserohilum turcicum in maize." Plant Disease 81: 277-282; Welz & Geiger, 2000, "Genes for resistance to northern corn leaf blight in diverse maize populations." Plant Breeding 119: 1-14). Backcrosses in many frequently used inbred maize lines such as W22, A619, B37 or B73 have successfully brought about introgression of the HT loci, where they exhibit a partial dominance and expression as a function of the respective genetic background (Welz, 1998, "Genetics and epidemiology of the pathosystem Zea mays / Setosphaeria turcica" Habilitationsschrift, Institut für Pflanzenzüchtung, Saatgutforschung und Populations-genetik, Universität Hohenheim).

Despite this complex genetic architecture of NCLB resistance in maize, until now, principally, the use of the *HT1* gene located on the long arm of chromosome 2 together with a partial quantitative resistance has been sufficient to control helminthosporiosis in maize (Welz, 1998). The basis for this is that globally, races 0 and 1 of *H. turcicum* are most prevalent (approximately 55%) (Lipps *et al.,* 1997), while other races such as 2N and 23N are only rare and even in geographically restricted areas (Welz, 1998). This race 0 is avirulent with regard to a maize plant with *HT1* (see also Table 1), so that when provided with a suitable quantitative resistance, it exhibits a sufficient general resistance to NCLB. However, many studies have reported an increasing dissemination of the less common races (Jordan et al., 1983, "Occurrence of race 2 of Exserohilum turcicum on corn in the central and eastern United States." Plant Disease 67: 1163-1165; Welz, 1998). The reasons for this are linked to the population dynamic of a pathogen which allows changes in pathogen virulence by new mutations in avirulence genes and new combinations of available virulence genes. This can lead to the occurrence of new, sometimes more aggressive pathogenic races. In Brazil, for example, the *H. turcicum* population already appears to be substantially more diverse with regard to the race composition than, for example, in North America. Already in the 1990s it has been reported that *H. turcicum* races had broken the resistance conferred by the *HT1* gene. In addition, there is the instability of the resistance genes to certain environmental factors such as temperature and light intensity in some climate zones (Thakur et al., 1989, "Effects of temperature and light on virulence of Exserohilum turcicum on corn." Phytopathology 1989, 79: 631-635). As a consequence, the use of novel *HT* resistance genes for the production of commercial maize plants in order to target a broader and more long-lasting resistance to *H. turcicum* in maize is growing in importance.

**Table 1: Overview of resistance (R) and susceptibility (S) of resistance loci against different Helminthosporium turcicum races:**

| **Pathogen** | **Host (Ht) reaction to each race** | | | |
|---|---|---|---|---|
| **Ht Race Designation** | **HT1 locus** | **HT2 locus** | **HT3 locus** | **HTN1 gene** |
| 0 | R | R | R | R |
| 1 | S | R | R | R |
| 2 | R | S | R | R |
| 3 | R | R | S | R |
| N | R | R | R | S |
| 12 | S | S | R | R |
| 23 | R | S | S | R |
| 2N | R | S | R | S |
| 12N | S | S | R | S |
| 23N | R | S | S | S |
| 123N | S | S | S | S |

One source of monogenic *HTN1* resistance is the Mexican landrace "Pepitilla" (Gevers, 1975, "A new major gene for resistance to Helminthosporium turcicum leaf blight of maize." Plant Dis Rep 59: 296-300). *HTN1* introgression lines exhibit a gene mapping on the long arm of chromosome 8. In contrast to the usual HT resistance genes, *HTN1* confers resistance by delaying the onset of sporulation, and thus combats the development of lesions. As a result, fewer, smaller lesions as well as reduced sporulation zones are formed (Simcox & Bennetzen, 1993, "The use of molecular markers to study Setospaeria turcica resistance in maize." Phytopathology 83: 1326-1330). Chlorotic-necrotic lesions such as those which occur with *HT1, HT2* or *HT3*-conferred resistance, are not formed (Gevers, 1975). WO2015/032494 discloses the identification of the causative gene, RLK1, conferring the "Pepitilla" resistance phenotype on bin 8.06 in corn and describes molecular markers which are suitable to benefit from this resistance locus without close-linked, undesired linkage drag leading to a negative impact on the yield potential.

In WO2011/163590 the genotypes PH99N and PH26N have been discloses as alternative sources for NCLB resistance on chromosome 8 bin 5.

With the intention of identifying a resistance gene for NCLB from the maize hybrid DK888, in 2010, Chung *et al.* published a study for fine mapping the bin 8.06 resistance locus (Chung et al. 2010 "Characterization and fine-mapping of a resistance locus for northern leaf blight in maize bin 8.06" Theoretical and Applied Genetics 121(2): 205-227). Investigations on *Helminthosporium* race specificity initially made it clear that functionally, the resistance locus was closely linked with the *HT2* and *HTN1* genes. Genome annotations of a 0.46 Mb-sized chromosome fragment using B73 reference hinted at several putative open reading frames; however, the causative gene has not been identified and a functional verification was not described.

It is thus an objective of the present invention to identify and/or further characterize plant resistance genes encoding polypeptides conferring or increasing resistance against a fungal pathogen, such as *Helminthosporium turcicum.*

### Summary of the invention

The present invention provides a nucleic acid molecule comprising or consisting of a nucleotide sequence having the nucleotide sequence set forth in SEQ ID NO: 1 or SEQ ID NO: 2. Also provided is a nucleic acid molecule comprising or consisting of a nucleotide sequence encoding the amino acid sequence set forth in SEQ ID NO: 3.

Further provided is a nucleic acid molecule comprising or consisting of a nucleotide sequence having at least 96% identity to the nucleotide sequence set forth in SEQ ID NO: 1 or SEQ ID NO: 2 and a nucleic acid molecule comprising or consisting of a nucleotide sequence encoding an amino acid sequence having at least 92% identity to the amino acid sequence set forth in SEQ ID NO: 3.

Also provided is a nucleic acid molecule comprising or consisting of a nucleotide sequence hybridizing with the complementary strand of the nucleotide sequence set forth in SEQ ID NO: 1 or SEQ ID NO: 2 or hybridizing with the complementary strand of the nucleotide sequence encoding the amino acid sequence set forth in SEQ ID NO: 3 under stringent hybridization conditions.

Moreover, provided is a nucleic acid molecule comprising or consisting of a nucleotide sequence encoding a protein, said protein being derived from the amino acid sequence encoded by the nucleotide sequence of SEQ ID NO: 1 or SEQ ID NO: 2 or from the amino acid sequence set forth in SEQ ID NO: 3 by way of substitution, deletion and/or addition of one or more amino acid(s).

The nucleic acid molecule according to the present invention encodes a polypeptide which is a wall associated receptor-like kinases 1 (WAK RLK1) or functionally belongs to the family of wall associated receptor-like kinases.

The nucleic acid molecule of the invention is encoding a polypeptide capable of conferring (or increasing) resistance to a plant disease caused by fungal pathogen, in particular by *Helminthosporium turcicum,* preferably by *Helminthosporium turcicum* races 0, 1 and/or N in a plant, preferably in a plant of the species *Zea mays,* in which the polypeptide is expressed.

In another aspect, provided is a vector or expression cassette comprising the nucleic acid molecule of the present invention. Preferably, in the vector or expression cassette, the nucleotide sequence of the invention is operably linked to a regulatory element allowing expression of the nucleotide sequence in a plant cell.

Further provided is a host cell comprising the nucleic acid molecule of the invention or the vector or expression cassette of the invention.

In another aspect, a polypeptide encoded by the nucleic acid molecule of the invention is provided. The polypeptide is a wall associated receptor-like kinases 1 (WAK RLK1) or functionally belongs to the family of wall associated receptor-like kinases 1. Preferably, the polypeptide is capable of conferring (or increasing) resistance to a plant disease caused by *Helminthosporium turcicum,* preferably by *Helminthosporium turcicum* races 0, 1 and/or N in a plant, preferably in a plant of the species *Zea mays,* in which the polypeptide is expressed.

Additionally, the polypeptide may not be capable of conferring or increasing resistance to a plant disease caused by *Helminthosporium turcicum* races 2 and/or 3 in a plant, preferably a plant of the species *Zea mays,* in which the polypeptide is expressed. The plant may show a susceptible response to infection with *Helminthosporium turcicum* races 2 and/or 3.

Furthermore, provided is a plant, preferably a plant of the species *Zea mays,* comprising the nucleic acid molecule of the invention transgenically (as transgene) or endogenously (as endogenous gene), the vector of the invention, the expression cassette of the invention, or the polypeptide of the invention. Preferably, the plant is resistant to a plant disease caused by *Helminthosporium turcicum,* in particular by *Helminthosporium turcicum* races 0, 1 and/or N. The plant may show a susceptible response to infection with *Helminthosporium turcicum* races 2 and/or 3. The plant may be a transgenic plant or a genetically edited plant. The plant may be a plant comprising the nucleic acid molecule of the invention endogenously, wherein the genomic flanking regions does not contain an A619HT2 or A619HT3 derived interval located between alleles of marker SYN14136 and marker MA0021 or an A619HT2 or A619HT3 derived interval located between alleles of marker MA0022 and marker SYN4196. A part of the plant of the invention, plant cell of the plant of the invention and seed of the plant of the invention is also provided, wherein the seed comprising the nucleic acid molecule of the invention transgenically (as transgene) or endogenously (as endogenous gene), the vector of the invention, the expression cassette of the invention, or the polypeptide of the invention.

According to another aspect, provided is a method or process of identifying or selecting a plant, preferably a plant of the species *Zea mays,* having increased resistance to a plant disease caused by fungal pathogen, preferably by *Helminthosporium turcicum,* more preferably by *Helminthosporium turcicum* races 0, 1 and/or N, or a part, a cell or a seed thereof, comprising the following steps: (a) detecting in the plant, or part, cell or seed thereof (or in a sample of the plant, or part, cell or seed thereof), the presence of the nucleic acid molecule of the present invention as described above or a nucleic acid molecule comprising or consisting of a nucleotide sequence selected from the group consisting of: (i) a nucleotide sequence having at least 60% identity to the nucleotide sequence set forth in SEQ ID NO: 1 or SEQ ID NO: 2, (ii) a nucleotide sequence encoding an amino acid sequence having at least 60% identity to the amino acid sequence set forth in SEQ ID NO: 3, (iii) a nucleotide sequence having at least 85% identity to nucleotide positions 1-920 of the nucleotide sequence set forth in SEQ ID NO: 1 or SEQ ID NO: 2 (Exon 1; SEQ ID NO: 6) and/or having at least 60% identity to nucleotide positions 23252-23288 of the nucleotide sequence set forth in SEQ ID NO: 1 (Exon 2; SEQ ID NO: 7) or to nucleotide positions 921-957 of the nucleotide sequence set forth in SEQ ID NO: 2 (Exon 2; SEQ ID NO: 7) and/or having at least 98% identity to nucleotide positions 23586-24632 of the nucleotide sequence set forth in SEQ ID NO: 1 (Exon 3; SEQ ID NO: 8) or to nucleotide positions 958-2004 of the nucleotide sequence set forth in SEQ ID NO: 2 (Exon 3; SEQ ID NO: 8), (iv) a nucleotide sequence encoding an amino acid sequence having at least 75% identity to the positions 1-306 of amino acid sequence set forth in SEQ ID NO: 3 and/or having at least 60% identity to positions 307-319 of amino acid sequence set forth in SEQ ID NO: 3 and/or having at least 98% identity to positions 320-668 of amino acid sequence set forth in SEQ ID NO: 3, wherein the nucleic acid molecule of any one of (i) to (iv) is encoding a polypeptide capable of conferring or increasing resistance to a plant disease caused by fungal pathogen, preferably by *Helminthosporium turcicum,* more preferably by *Helminthosporium turcicum* races 0, 1 and/or N, in a plant, preferably a plant of the species *Zea mays,* in which the polypeptide is expressed, and wherein preferably the nucleic acid molecule of any one of (i) to (iv) encodes a polypeptide which is or belongs functionally to the family of wall associated receptor-like kinases 1 (WAK RLK1); and (b) identifying or selecting the plant in which or in whose part, cell or seed the nucleic acid molecule as defined in (a) is present, as having a resistance or an increased resistance to a plant disease caused by a fungal pathogen, preferably by *Helminthosporium turcicum,* more preferably by *Helminthosporium turcicum* races 0, 1 and/or N.

Further provided is, a method or process of identifying a plant, preferably a plant of the species *Zea mays,* which is resistant to a plant disease caused by *Helminthosporium turcicum,* in particular by *Helminthosporium turcicum* races 0, 1 and/or N and comprises the nucleic acid molecule of the invention endogenously, the method or process comprising detecting in the plant alleles of at least two markers, wherein at least one of said markers is on or within the chromosomal interval between SYN14136 and the nucleic acid molecule of the invention, and at least one of said markers is on or within the chromosomal interval between the nucleic acid molecule of the invention and SYN4196.

In another aspect, provided is a plant identified or selected by the method or process of identifying or selecting according to the present invention, or progeny thereof.

In another aspect, provided is a method of identifying an allele of a resistance gene conferring or increasing resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, in *Zea mays* and preferably encoding a polypeptide which is or belongs functionally to the family of wall associated receptor-like kinases 1 (WAK RLK1), wherein the method comprises the following steps: (a) conducting sequence comparison using (i) at least one coding nucleotide sequence originating or derived from a *Zea mays* genotype, wherein the nucleotide sequence preferably maps to bin 8.05 resistance locus or to bin 8.06 resistance locus, and (ii) as reference sequence, a nucleotide sequence of the invention, preferably the nucleotide sequence set forth in SEQ ID NO: 1 or SEQ ID NO: 2 or the nucleotide sequence encoding the amino acid sequence set forth in SEQ ID NO: 3, or a part thereof, or a consensus sequence derived from a set of at least two nucleotide sequences wherein one nucleotide sequence is the nucleotide sequence of the invention, preferably the nucleotide sequence set forth in SEQ ID NO: 1 or SEQ ID NO: 2 or the nucleotide sequence encoding the amino acid sequence set forth in SEQ ID NO: 3, or a part thereof, and wherein each nucleotide sequence of the set of at least two nucleotide sequences encodes a polypeptide capable of conferring or increasing resistance to a plant disease caused by fungal pathogen, preferably by *Helminthosporium turcicum,* more preferably by *Helminthosporium turcicum* races 0, 1 and/or N, in *Zea mays* in which the polypeptide is expressed, and preferably maps to bin 8.05 resistance locus or to bin 8.06 resistance locus and preferably encoding a polypeptide which is or belongs functionally to the family of wall associated receptor-like kinases 1 (WAK RLK1); and (b) identifying the allele, if the sequence comparison reveals (i) a sequence identity on nucleotide level of at least 85% identity to nucleotide positions 1-920 of the nucleotide sequence set forth in SEQ ID NO: 1 or SEQ ID NO: 2 and/or of at least 60% identity to nucleotide positions 23252-23288 of the nucleotide sequence set forth in SEQ ID NO: 1 or to nucleotide positions 921-957 of the nucleotide sequence set forth in SEQ ID NO: 2 and/or of at least 98% identity to nucleotide positions 23586-24632 of the nucleotide sequence set forth in SEQ ID NO: 1 or to nucleotide positions 958-2004 of the nucleotide sequence set forth in SEQ ID NO: 2, and/or (ii) a sequence identity of encoded amino acid level of at least 75% identity to the positions 1-306 of amino acid sequence set forth in SEQ ID NO: 3 and/or of at least 60% identity to positions 307-319 of amino acid sequence set forth in SEQ ID NO: 3 and/or of at least 98% identity to positions 320-668 of amino acid sequence set forth in SEQ ID NO: 3.

Further provided is a nucleic acid molecule comprising or consisting of a nucleotide sequence of the allele of a resistance gene identified by the method of identifying an allele of a resistance gene.

In another aspect, provided is a method for conferring or increasing resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, in a plant, preferably a plant of the species *Zea mays,* comprising the following steps: (a) introducing into at least one cell of the plant the nucleic acid molecule of the present invention, including for example a nucleic acid molecule comprising or consisting of a nucleotide sequence of the identified allele, the expression cassette of the invention or the vector of the invention, (b) regenerating the plant from the at least one cell, and (c) causing expression of the nucleic acid molecule in the plant.

Further provided is a method for increasing resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, in a plant of the invention, comprising the step of reducing the level of expression of the nucleic acid molecule of the present invention, including for example a nucleic acid molecule comprising or consisting of a nucleotide sequence of the identified allele, the expression cassette of the invention or the vector of the invention, in the plant or at least one cell of the plant, preferably compared to the expression level of the endogenous gene in a resistant wild type plant.

In a further aspect, provided is a method for producing a plant, preferably a plant of the species *Zea mays,* having (increased) resistance to a plant disease caused by fungal pathogen, preferably by *Helminthosporium turcicum,* more preferably by *Helminthosporium turcicum* races 0, 1, 2, 3, N, 12, 23, 2N, 12N, 23N and/or 123N, or a part, a cell or a seed thereof, comprising the following steps: (a) introducing into the plant or at least one cell of the plant the nucleic acid molecule of the present invention, including for example a nucleic acid molecule comprising or consisting of a nucleotide sequence of the identified allele, the expression cassette of the invention or the vector of the invention, (b) optionally, regenerating the plant from the at least one cell, and (c) causing expression of the nucleic acid molecule in the plant.

In another aspect, provided is a plant produced by the methods for producing a plant according to the present invention, or progeny thereof.

Further provided is a method for controlling infestation of a fungal pathogen, preferably *Helminthosporium turcicum,* more preferably *Helminthosporium turcicum* races 0, 1, 2, 3, N, 12, 23, 2N, 12N, 23N and/or 123N, in a population of plants, preferably plants of the species *Zea mays,* comprising the following steps: (a) growing plants of the present invention on agricultural and horticultural fields, and (b) causing expression of the nucleic acid molecule of the present invention, including for example a nucleic acid molecule comprising or consisting of a nucleotide sequence of the identified allele, the expression cassette of the invention or the vector of the invention, in the plants.

Another aspect of the invention is an oligonucleotide having a length of at least 15, 16, 17, 18, 19 or 20, preferably at least 21, 22, 23, 24 or 25, more preferred at least 30, 35, 40, 45, 50, 100, 200, 300 or 500 nucleotides, wherein the oligonucleotide is able to hybridize or anneal to (i) the nucleic acid molecule of the invention, (ii) the nucleic acid molecule comprising the nucleotide sequence of SEQ ID NO: 4 or SEQ ID NO: 5, or (iii) nucleic acid molecule complementary to (i) or (ii).

Further provided is a pair of oligonucleotides or a kit comprising said oligonucleotides, wherein the oligonucleotides are suitable to anneal as forward primer and reverse primer to a region in the plant genome, preferably the *Zea mays* genome, which shows a cosegregation, preferably a perfect cosegregation, with the nucleic acid molecule of the invention.

In a further aspect, provided is the use of at least one oligonucleotide as a molecular marker or part thereof in the method of identifying or selecting a plant, preferably a plant of the species *Zea mays,* having increased resistance to a plant disease caused by fungal pathogen, preferably by *Helminthosporium turcicum,* more preferably by *Helminthosporium turcicum* races 0, 1 and/or N, or a part, a cell or a seed thereof, or in the method of identifying an allele of a resistance gene conferring or increasing resistance to a plant disease caused by a fungal pathogen, preferably by *Helminthosporium turcicum,* more preferably by *Helminthosporium turcicum* races 0, 1 and/or N, in *Zea mays,* wherein the molecular marker is able to detect at least one single nucleotide polymorphism, deletion or insertion diagnostic for the nucleic acid molecule of the present invention and/or comprises the oligonucleotide as described above and/or is the pair of oligonucleotides or the kit as described above.

In one aspect, provided is the use of at least one or at least two oligonucleotide(s) as a molecular marker or part thereof in method or process of identifying a plant as described above or in a method or process for the elimination of linkage drag close linked to the nucleic acid molecule of the invention for detecting the presence or absence of an A619HT2 or A619HT3 derived interval located between alleles of marker SYN14136 and marker MA0021 or an A619HT2 or A619HT3 derived interval located between alleles of marker MA0022 and marker SYN4196.

In another aspect, provided is a method for detecting the presence or absence of the nucleic acid molecule of the invention, including for example a nucleic acid molecule comprising or consisting of a nucleotide sequence of the identified allele, in a plant, preferably a plant of the species *Zea mays,* comprising the following steps: (a) isolating DNA from at least one cell of the plant, and (b) using a molecular marker to detect the presence or absence of the nucleic acid molecule of the invention, wherein the molecular marker is able to detect at least one single nucleotide polymorphism, deletion or insertion diagnostic for the nucleic acid molecule of the present invention and/or comprises the oligonucleotide as described above and/or is the pair of oligonucleotides or the kit as described above.

### Brief description of the drawings

FIG 1: shows vector p7U-nativeHT2_CDS_2 which can be used to transform *Zea mays* plants. The expression cassette containing the cDNA of HT2 gene (SEQ ID NO: 2) under control of the native promoter (SEQ ID NO: 4) and terminator (SEQ ID NO: 5) was transformed into a binary vector containing for instance a herbicide gene (e.g.: BASTA resistance, glyphosate resistance or ALS inhibitor resistance) for subsequent transformation into *Agrobacterium tumefaciens* for Agrobacterium mediated plant transformation into maize (*Zea mays*) genotype A188.
FIG 2 A - D: shows a CLUSTAL O (1.2.4) multiple sequence alignment of the RLK1 alleles HT2 (SEQ ID NO: 2), HTN1 (SEQ ID NO: 9), PH99N (SEQ ID NO: 11) and PH26N (SEQ ID NO: 13). Position with sequence differences are indicated by missing asterisk in the lowest line of each block.
FIG 3A and B: shows a ClustaIV (PAM250) multiple sequence alignment of RLK1 polypeptides: RLK1_A619HT2_CDS.seq (SEQ ID NO: 3), RLK1_A619HT3_CDS.seq (identical with SEQ ID NO: 3), RLK1_B37HTN_CDS.seq (SEQ ID NO: 10), RLK1_A619HT2_Exon1.seq (SEQ ID NO: 6), RLK1_A619HT2_Exon2.seq (SEQ ID NO: 7), and RLK1_A619HT2_Exon3.seq (SEQ ID NO: 8).
FIG 4 A - C: shows sequence alignment of the cDNA of HT2 (SEQ ID NO: 2) and the cDNA of the RLK1 allele derived from genotype A188. At position 1458 to 1459 of the A188 allele a 2 bp insertion "AC" (white letters on black background) which causes an early stop codon (bold and underlined) has been identified.
FIG 5: shows sequence alignment of amino acids of A619HT2 and modified A188 RLK1. Modified A188 RLK is 99.1% identical to A619HT2.
FIG 6: shows a map of marker positions with reference to the marker positions of AGPv02.

### Detailed description of the invention

The present invention is based on the identification of the *HT2* gene which encodes a polypeptide conferring (or increasing) resistance to a plant against a fungal pathogen, such as *Helminthosporium turcicum.*

Accordingly, in one aspect of the present invention, provided is a nucleic acid molecule comprising or consisting of a nucleotide sequence encoding said *HT2* gene. In one embodiment, the nucleic acid molecule of the invention is comprising or consisting of a nucleotide sequence having the nucleotide sequence set forth in SEQ ID NO: 1 (genomic DNA of *HT2*) or SEQ ID NO: 2 (cDNA of *HT2*). Also provided is a nucleic acid molecule comprising or consisting of a nucleotide sequence encoding the amino acid sequence set forth in SEQ ID NO: 3 (HT2 protein).

Further provided is a nucleic acid molecule comprising or consisting of a nucleotide sequence having at least 96% identity to the nucleotide sequence set forth in SEQ ID NO: 1 or SEQ ID NO: 2, preferably over the full length. Preferably, the nucleotide sequence having at least 97%, 98%, 99% or 99.5% identity to the nucleotide sequence set forth in SEQ ID NO: 1 or SEQ ID NO: 2, preferably over the full length. Also provided is a nucleic acid molecule comprising or consisting of a nucleotide sequence encoding an amino acid sequence having at least 92% identity to the amino acid sequence set forth in SEQ ID NO: 2, preferably over the full length. Preferably, the nucleic acid molecule comprises or consists of a nucleotide sequence encoding an amino acid sequence having at least 93%, 94%, 95%, 96%, 97%, 98%, 99% or 99.5% identity to the amino acid sequence set forth in SEQ ID NO: 3, preferably over the full length.

In another embodiment, provided is a nucleic acid molecule comprising or consisting of a nucleotide sequence hybridizing with the complementary strand of the nucleotide sequence set forth in SEQ ID NO: 1 or SEQ ID NO: 2 or the nucleotide sequence encoding the amino acid sequence set forth in SEQ ID NO: 3 under stringent hybridization conditions.

Moreover, provided is a nucleic acid molecule comprising or consisting of a nucleotide sequence encoding a protein derived from the protein encoded by the nucleotide sequence of SEQ ID NO: 1 or SEQ ID NO: 2, or derived from the amino acid sequence set forth in SEQ ID NO: 3 by way of substitution, deletion and/or addition of one or more amino acid(s).

Deletion, substitution, or addition of amino acid(s) can be carried out by a technique known in the art. Mutagenesis in a nucleotide sequence can be caused by a known method such as the Kunkel method, the gapped duplex method, or a method similar to such a known method. For instance, mutagenesis can be caused with the use of a mutagenesis kit (e.g., Mutant-K or Mutant-G (product name, TAKARA Bio)) based on a site-directed mutagenesis method, an LA PCR *in vitro* Mutagenesis series kit (product name, TAKARA Bio), or the like. Alternatively, a mutagenesis method may be a method using a chemical mutagen represented by EMS (ethyl methanesulfonate), 5-bromouracil, 2-aminopurine, hydroxylamine, N-methyl-N'-nitro-N-nitrosoguanidine, or a different carcinogenic compound, a method comprising radiation treatment using radioactive rays such as X-rays, alpha-rays, beta-rays, gamma-rays, or an ion beam, or a method comprising ultraviolet treatment.

When an amino acid residue is altered, the amino acid is preferably mutated for a different amino acid(s) that conserves the properties of the amino acid. Examples of amino acid properties are: hydrophobic amino acids (A, I, L, M, F, P, W, Y, and V), hydrophilic amino acids (R, D, N, C, E, Q, G, H, K, S, and T), amino acids containing aliphatic side chains (G, A, V, L, I, and P), amino acids containing hydroxyl group-containing side chains (S, T, and Y), amino acids containing sulfur-containing side chains (C and M), amino acids containing carboxylic acid- and amide-containing side chains (D, N, E, and Q), amino acids containing basic side chains (R, K, and H), and amino acids containing aromatic side chains (H, F, Y, and W) (amino acids are represented by one-letter codes in parentheses). Amino acid substitutions within each group are called conservative substitutions. Conservative substitutions are preferred.

Preferably, the nucleic acid molecule of the invention is encoding a polypeptide capable of conferring (or increasing) resistance to a plant disease caused by a fungal pathogen in a plant in which the polypeptide is expressed.

Additionally, the nucleic acid molecule of the invention is encoding a polypeptide which may not be capable of conferring resistance to a plant disease caused by *Helminthosporium turcicum* races 2 and/or 3 in a plant, preferably a plant of the species *Zea mays,* in which the polypeptide is expressed. The plant may show a susceptible response to infection with *Helminthosporium turcicum* races 2 and/or 3.

A further example of the nucleic acid molecule of the invention may be a nucleic acid molecule comprises or consists of the nucleotide sequence set forth in SEQ ID NO: 23, or a nucleic acid molecule comprising or consisting of a nucleotide sequence encoding the amino acid sequence set forth in SEQ ID NO: 24.

In one embodiment, the fungal pathogen belongs to the division of Ascomycota or Basidiomycota. The fungal pathogen may belong to family Pleosporaceae, Pucciniaceae or Botryosphaeriaceae. Preferably, the fungal pathogen belongs to the genus of Setosphaeria, Bipolaris, Puccinia or Diplodia, more preferably is the species of *Helminthosporium turcicum, Setosphaeria rostrata, Setosphaeria glycinea, Setosphaeria holmii, Setosphaeria khartoumensis, Setosphaeria minor, Setosphaeria monoceras, Setosphaeria pedicellata, Setosphaeria prolata, Bipolaris australis, Bipolaris brizae, Bipolaris buchloës, Bipolaris cactivora, Bipolaris clavata, Bipolaris coicis, Bipolaris colocasiae, Bipolaris crotonis, Bipolaris crustacean, Bipolaris cylindrical, Bipolaris euchlaenae, Bipolaris halepensis, Bipolaris heveae, Bipolaris incurvata, Bipolaris indica, Bipolaris iridis, Bipolaris leersiae, Bipolaris micropus, Bipolaris miyakei, Bipolaris multiformis, Bipolaris nicotiae, Bipolaris novae-zelandiae, Bipolaris ovariicola, Bipolaris panici-miliacei, Bipolaris papendorfii, Bipolaris sacchari, Bipolaris salkadehensis, Bipolaris sorghicola, Bipolaris subpapendorfii, Bipolaris tropicalis, Bipolaris urochloae, Bipolaris zeae, Puccinia asparagi, Puccinia graminis, Puccinia horiana, Puccinia mariae-wilsoniae, Puccinia poarum, Puccinia psidii, Puccinia recondite, Puccinia sessilis, Puccinia sorghi, Puccinia striiformis, Puccinia triticina, Diplodia maydis, Diplodia seriata* or *Stenocarpella (Diplodia) macrospora,* most preferably is *Helminthosporium turcicum, Puccinia sorghi, Diplodia macrospora* or *Bipolaris maydis.*

In one embodiment, the plant disease is a fungal disease. In a preferred embodiment, the plant disease is selected from the group consisting of Northern Corn Leaf Blight (caused by *Helminthosporium turcicum*), Southern Corn Leaf Blight (caused by *Bipolaris maydis*)*,* Common Rust (caused by *Puccinia sorghi*)*,* and Diplodia Leaf Streak (caused by *Diplodia macrospora,* also called *Stenocarpella macrospora*)*.* Most preferably, the plant disease is Northern Corn Leaf Blight (NCLB).

In a preferred embodiment, the nucleic acid molecule of the invention is encoding a polypeptide capable of conferring to a plant (or increasing in a plant) resistance against Northern Corn Leave Blight (NCLB), i.e. resistance against *Helminthosporium turcicum,* in particular resistance against *Helminthosporium turcicum* races 0, 1, 2, 3, N, 12, 23, 2N, 12N, 23N and/or 123N, or resistance against *Helminthosporium turcicum* races 0, 1 and/or N.

In one embodiment, the plant according to the invention is *Hordeum vulgare, Hordeum bulbusom, Sorghum bicolor, Saccharum officinarium, Zea mays, Setaria italica, Oryza minuta, Oriza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Morus notabilis, Arabidopsis arenosa, Arabidopsis lyrata, Arabidopsis thaliana, Crucihimalaya himalaica, Crucihimalaya wallichii, Cardamine flexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* or any variety or subspecies belonging to one of the aforementioned plants, more preferably *Hordeum vulgare, Hordeum bulbusom, Sorghum bicolor, Zea mays, Setaria italica, Oryza minuta, Oriza sativa, Oryza australiensis, Oryza alta, Triticum aestivum, Triticum durum, Secale cereale,* Triticale, *Hordeum marinum, Aegilops tauschii,* or any variety or subspecies belonging to one of the aforementioned plants, or most preferably *Hordeum vulgare, Sorghum bicolor, Zea mays, Triticum aestivum, Secale cereale,* or any variety or subspecies belonging to one of the aforementioned plants.

In a preferred embodiment, the plant according to the invention is *Zea mays.*

Preferably, the nucleic acid molecule according to the present invention encodes a polypeptide which is a wall associated receptor-like kinases 1 (WAK RLK1) or functionally belongs to the family of wall associated receptor-like kinases 1.

In another aspect, provided is a vector comprising the nucleic acid molecule of the present invention. The vector may be a plasmid, a cosmid, a phage or an expression vector, a transformation vector, shuttle vector or cloning vector, it may be double or single stranded, linear or circular, or it may be a prokaryotic or eukaryotic host, either by integration into its genome or transforming extrachromosomally.

Also provided is an expression cassette comprising the nucleic acid molecule of the invention.

In one embodiment, in the vector or expression cassette, the nucleotide sequence of the invention is operably linked to regulatory element allowing expression of the nucleotide sequence in a plant cell. The plant cell may be infectable by a fungal pathogen or infected by a fungal pathogen. In a preferred embodiment, the plant cell is located in a leaf or a leaf tissue. The regulatory element may be a promoter (native, synthetic, core promoter or chimeric promoter), a terminator, an enhancer or a cis-acting element. Furthermore, the regulatory element may be heterologous to the nucleotide sequence operably linked to regulatory element.

Preferably, the nucleotide sequence of the invention is operably linked in an expression vector to one or more regulatory sequences which allow transcription and optionally expression in a prokaryotic or eukaryotic host cell. As an example, the nucleotide sequence may be under the control of a suitable promoter or a terminator. Suitable promoters may be promoters which are constitutively induced (see, for example, the 35S promoter from the "cauliflower mosaic virus" (Odell JT, Nagy F, Chua N-H (1985) "Identification of DNA sequences required for activity of the cauliflower mosaic virus 35S promoter." Nature 313, 810 - 812 1985); particularly suitable promoters are those promoters which are pathogen-inducible (see, for example, the PR1 promoter from parsley (Rushton PJ, Torres JT, Parniske M, Wernert P, Hahlbrock K und Somssich IE (1996) Interaction of elicitor-induced DNA-binding proteins with elicitor response elements in the promoters of parsley PR1 genes. EMBO J. 15(20): 5690-5700). Particularly suitable pathogen-inducible promoters are synthetic or chimeric promoters which do not occur in nature, are composed of several elements and contain a minimum promoter as well as, upstream of the minimum promoter, at least one cis-regulatory element which act as the binding site for special transcription factors. Chimeric promoters are custom-designed and are induced by various factors or re-primed. Examples of such promoters can be found in WO2000/29592 and WO2007/147395. An example of a suitable terminator is the nos-terminator (Depicker A, Stachel S, Dhaese P, Zambryski P, Goodman HM (1982) Nopaline synthase: transcript mapping and DNA sequence. J Mol Appl Genet. 1(6): 561-73).

Further provided is a host cell comprising the nucleic acid molecule of the invention, or the vector of the invention, or the expression cassette of the invention.

The vector or the expression cassette may, for example, be introduced into the host cell by conjugation, mobilization, biolistic transformation, agrobacterium-conferred transformation, transfection, transduction, vacuum infiltration or electroporation. Methods of this type as well as methods for the preparation of the vectors described are familiar to the person skilled in the art (Sambrook et al., Molecular Cloning, Cold Spring Harbor Laboratory, 3rd Ed., 2001).

In one embodiment, the host cell may be a prokaryotic cell (for example, a bacterial cell). In another embodiment, the host cell may be a eukaryotic cell (for example, a plant cell or a yeast cell). Particularly preferred bacterial host cells are *Agrobacterium tumefaciens, A. rhizogenes, and E. coli.*

In another aspect, provided is a protein encoded by the nucleic acid molecule of the invention.

In yet another aspect, provided is a plant comprising the nucleic acid molecule of the invention, the vector of the invention, the expression cassette of the invention, or the protein of the invention. The plant may be a transgenic plant or a genetically edited plant. A part of the plant of the invention, plant cell of the plant of the invention and seed of the plant of the invention is also provided, wherein the seed comprising the nucleic acid molecule of the invention transgenically or endogenously, the vector of the invention, the expression cassette of the invention, or the polypeptide of the invention.

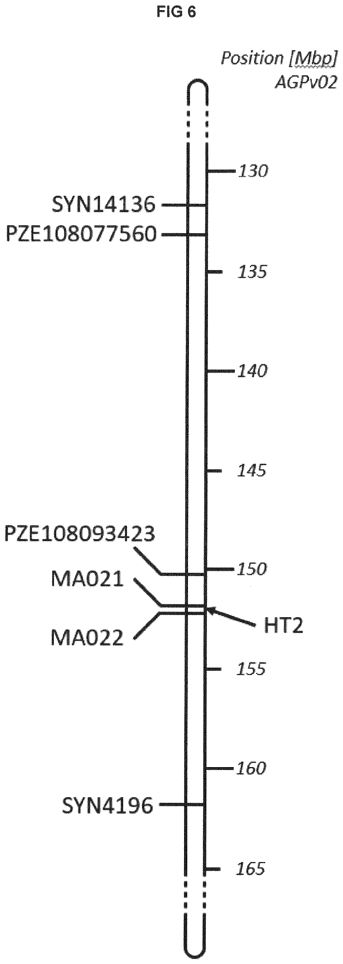

From WO2015/032494 which investigated and used introgression lines with *Htn1* from Pepitilla, it is known that this restance locus is closely linked to genomic regions carrying linkage drag resulting in negative effects on one or more agronomic features. First investigation of the flanking region of HT2 indicated that this or similar linkage drag is not only present in Pepitilla donor for HtN1 introgression, but also in other donors for this Helminthosporium resistance locus like A619. *Inter alia* the linkage drag as part of the HT2 introgression can effect a difference in the flowering time, which is an important agronomic characteristic. It can directly and substantially influence the yield potential of a Zea mays plant. A delayed flowering time usually results in a reduced yield. Further linkage drag affecting the yield potential, in particular the silage yield potential, may be found distal and/or proximal of the Helminthosporium resistance locus on bin 8.06 in *Zea mays.* Flanking regions, closely linked to this resistance locus, might be carrier of the known linkage drag, however, these regions can be limited to an interval located between alleles of marker SYN14136 and marker MA0021 and/or an interval located between alleles of marker MA0022 and marker SYN4196 (FIG. 6). Thus, the plant of the invention may be a plant of the species *Zea mays* comprising the nucleic acid molecule of the invention endogenously, wherein the flanking regions in the genome does not contain an A619HT2 or A619HT3 derived interval located between alleles of marker SYN14136 and marker MA0021 or an A619HT2 or A619HT3 derived interval located between alleles of marker MA0022 and marker SYN4196. In a preferred embodiment, the plant is a plant of the species *Zea mays* comprising the nucleic acid molecule of the invention endogenously, wherein the flanking regions in the genome does not contain an A619HT2 or A619HT3 derived interval located between alleles of marker SYN14136 and marker PZE108077560, an A619HT2 or A619HT3 derived interval located between alleles of marker PZE108093423 and marker MA0021, or an A619HT2 or A619HT3 derived interval located between alleles of marker MA0022 and marker SYN4196.

**Table 2: KASP marker primer sequences and assignment to donor alleles (allele X and allele Y: describe the biallelic values of the SNPs)**

| SNP marker | Marker position AGPv02 [bp] | Primer alleles X(5'-3') [SEQ ID NO] | Primer alleles Y(5'-3') [SEQ ID NO] | Common primer (5'-3') [SEQ ID NO] | A619HT2 donor alleles (SNP) |
|---|---|---|---|---|---|
| SYN14136 | 131681497 | 25 | 26 | 27 | A |
| PZE-108077560 | 133189880 | 28 | 29 | 30 | A |
| PZE-108093423 | 150279048 | 31 | 32 | 33 | A |
| MA0021 | 151907173 | 34 | 35 | 36 | G |
| MA0022 | 152046529 | 37 | 38 | 39 | A |
| SYN4196 | 161766769 | 40 | 41 | 42 | C |

As an example, removal of linkage drag may be carried out by genetic recombination during a crossing process between two maize plants, wherein one parent maize plant carries the HT2-resistance locus. In addition to the use of conventional breeding techniques to produce a genetic recombination which has the result of replacing at least one of the donor intervals with linkage drag identified above with genomic sequences of the recurrent parent which are preferably free from unwanted genes, modern biotechnology offers the person skilled in the art many tools which can enable precise genetic engineering to be carried out. Examples of known tools are meganucleases (Silva et al., 2011), homing endonucleases (Chevalier 2002), zinc finger nucleases, TALE nucleases (WO 2010/079430; WO 2011/072246) or CRISPR systems (Gaj et al., 2013). These are artificial nuclease fusion proteins which are capable of cleaving double stranded nucleic acid molecules such as plant DNA and thus of producing double strand breaks at desired positions in the genome. By exploiting the cells own mechanisms for repairing induced double strand breaks, a homologous recombination or a "non-homologous end joining" can be carried out, which could lead to the removal of the intervals of the donor carrying linkage drag. Suitable target sequences in the genome for the recognition domain nucleases may be taken, for example, from the sequence information of the SNP markers (Table 2). However, a person skilled in the art is also able to identify other sequences, preferably within the defined flanking regions described above, which are suitable as target sequences for the recognition domains of the nucleases.

In another aspect, provided is a genetically edited or transgenic plant comprising the nucleic acid molecule of the invention, the vector of the invention or the expression cassette of the invention. The nucleic acid molecule may be a transgene or a modified/edited endogenous gene. A promoter may be operably linked to the nucleic acid molecule or nucleotide sequence for expression.

Also provided is a part or seed of the plant of the invention comprising the nucleic acid molecule of the invention, the vector of the invention or the expression cassette of the invention, wherein the seed comprises the nucleic acid molecule of the invention, the vector of the invention, the expression cassette of the invention. The nucleic acid molecule may be a transgene or a modified/edited endogenous gene.

According to another aspect, provided a method of identifying or selecting a plant, preferably a plant of the species *Zea mays,* having increased resistance to a plant disease caused by fungal pathogen, preferably by *Helminthosporium turcicum,* more preferably by *Helminthosporium turcicum* races 0, 1 and/or N, or a part, a cell or a seed thereof, comprising the following steps: (a) detecting in the plant, or part, cell or seed thereof (or in a sample of the plant, or part, cell or seed thereof), the presence of the nucleic acid molecule of the present invention as described above or a nucleotide sequence selected from the group consisting of: (i) a nucleotide sequence having at least 60%, 65%, 70%, 75%, 80%, 85%, 90%, 92%, 94%, 96%, 98%, 99% or 99.5% identity to the nucleotide sequence set forth in SEQ ID NO: 1 or SEQ ID NO: 2, preferably over the full length, (ii) a nucleotide sequence encoding an amino acid sequence having at least 60%, 65%, 70%, 75%, 80%, 85%, 90%, 92%, 94%, 96%, 98%, 99% or 99.5% identity to the amino acid sequence set forth in SEQ ID NO: 3, preferably over the full length, (iii) a nucleotide sequence having at least 85%, 90%, 92%, 94%, 96%, 98%, 99% or 99.5% identity to nucleotide positions 1-920 of the nucleotide sequence set forth in SEQ ID NO: 1 or SEQ ID NO: 2 (Exon 1; SEQ ID NO: 6), preferably over the full length, and/or having at least 60%, 65%, 70%, 75%, 80%, 85%, 90%, 92%, 94%, 96%, 98%, 99% or 99.5% identity to nucleotide positions 23252-23288 of the nucleotide sequence set forth in SEQ ID NO: 1 (Exon 2; SEQ ID NO: 7) or to nucleotide positions 921-957 of the nucleotide sequence set forth in SEQ ID NO: 2 (Exon 2; SEQ ID NO: 7), preferably over the full length, and/or having at least 98%, 98,5%, 99% or 99.5% identity to nucleotide positions 23586-24632 of the nucleotide sequence set forth in SEQ ID NO: 1 (Exon 3; SEQ ID NO: 8) or to nucleotide positions 958-2004 of the nucleotide sequence set forth in SEQ ID NO: 2 (Exon 3; SEQ ID NO: 8), preferably over the full length, (iv) a nucleotide sequence encoding an amino acid sequence having at least 75%, 80%, 85%, 90%, 92%, 94%, 96%, 98%, 99% or 99.5% identity to the positions 1-306 of amino acid sequence set forth in SEQ ID NO: 3, preferably over the full length, and/or having at least 60%, 65%, 70%, 75%, 80%, 85%, 90%, 92%, 94%, 96%, 98%, 99% or 99.5% identity to positions 307-319 of amino acid sequence set forth in SEQ ID NO: 3, preferably over the full length, and/or having at least 98%, 98,5%, 99% or 99.5% identity to positions 320-668 of amino acid sequence set forth in SEQ ID NO: 3, preferably over the full length, wherein the nucleic acid molecule of any one of (i) to (iv) is encoding a polypeptide capable of conferring or increasing resistance to a plant disease caused by fungal pathogen, preferably by *Helminthosporium turcicum,* more preferably by *Helminthosporium turcicum* races 0, 1 and/or N, in a plant, preferably a plant of the species *Zea mays,* in which the polypeptide is expressed, and wherein preferably the nucleic acid molecule of any one of (i) to (iv) encodes a polypeptide which is or belongs functionally to the family of wall associated receptor-like kinases 1 (WAK RLK1); or detecting in the plant, or part, cell or seed thereof (or in a sample of the plant, or part, cell or seed thereof), the presence of polypeptide encoded by the nucleic acid molecule of the present invention as described above or a nucleotide sequence of any one of (i) to (iv), and (b) identifying or selecting the plant in which or in whose part, cell or seed the nucleic acid molecule as defined in (a) is present, as having a resistance or an increased resistance to a plant disease caused by a fungal pathogen, preferably by *Helminthosporium turcicum,* more preferably by *Helminthosporium turcicum* races 0, 1 and/or N.

The method may comprise the additional step of obtaining a nucleic acid sample from the plant or part, cell or seed and detecting the sequence in the sample. For example, the step of obtaining the nucleic acid sample from the plant, or part or seed thereof, may be carried out prior to the detection step (a).

The detection of a nucleotide sequence may be carried out by a hybridization method, using an oligonucleotide probe. The conditions of hybridization can appropriately be selected depending on factors such as the Tm value of the probe used and the CG content of the target DNA. Known hybridization methods are described, for example, in Sambrook et al., 2001.

Alternatively, the detection of a gene may be carried out by means of a DNA amplification method, such as PCR, using respective primers. When the detection is carried out by PCR method, PCR conditions can appropriately be selected depending on the factors such as the Tm value of the primer used and the length of the amplified region to be detected. The detection can be carried out by amplifying the target by PCR and confirming the presence or absence of a PCR-amplified product. The method for confirming the presence or absence of an amplification product is not particularly limited. For example, the amplification product can be confirmed by subjecting a reaction mixture of nucleic acid amplification to agarose gel electrophoresis; thereafter, staining the gel with an appropriate nucleic acid staining reagent such as ethidium bromide, SYBER Green I or the like; and detecting the presence or absence of the bands resulting from irradiation with ultraviolet rays. The bands may be detected by visual observation, or they may be detected by using, for example, a fluorescent image analyzer, or the like.

Further provided is, a method or process of identifying a plant, preferably a plant of the species *Zea mays,* having increased resistance to a plant disease caused by fungal pathogen, *Helminthosporium turcicum,* more preferably by *Helminthosporium turcicum* races 0, 1 and/or N, or a part, having increased resistance to a plant disease caused by *Helminthosporium turcicum,* preferably by *Helminthosporium turcicum* races 0, 1 and/or N and comprises the nucleic acid molecule of the invention endogenously, a cell or a seed thereof, the method or process comprising detecting in the plant alleles of at least two markers, wherein at least one of said markers is on or within the chromosomal interval between SYN14136 and the nucleic acid molecule of the invention, and at least one of said markers is on or within the chromosomal interval between the nucleic acid molecule of the invention and SYN4196. In a preferred embodiment, the method or process comprising detecting in the plant alleles of at least two markers, wherein at least one of said markers is on or within the chromosomal interval between PZE108093423 and the nucleic acid molecule of the invention, and at least one of said markers is on or within the chromosomal interval between the nucleic acid molecule of the invention and SYN4196.

In another aspect, provided is a method of identifying an allele of a resistance gene conferring or increasing resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, in *Zea mays* and preferably encoding a polypeptide which is or belongs functionally to the family of wall associated receptor-like kinases 1 (WAK RLK1), wherein the method comprises the following steps: (a) conducting sequence comparison using (i) at least one coding nucleotide sequence isolated from a *Zea mays* genotype, wherein the nucleotide sequence preferably maps to bin 8.05 resistance locus or to bin 8.06 resistance locus, and (ii) as reference sequence, a nucleotide sequence of the invention, preferably the nucleotide sequence set forth in SEQ ID NO: 1 or SEQ ID NO: 2 or the nucleotide sequence encoding the amino acid sequence set forth in SEQ ID NO: 3, or a part thereof, or a consensus sequence derived from a set of at least two nucleotide sequences wherein one nucleotide sequence is the nucleotide sequence of the invention, preferably the nucleotide sequence set forth in SEQ ID NO: 1 or SEQ ID NO: 2 or the nucleotide sequence encoding the amino acid sequence set forth in SEQ ID NO: 3, or a part thereof, and wherein each nucleotide sequence of the set of at least two nucleotide sequences encodes a polypeptide capable of conferring or increasing resistance to a plant disease caused by fungal pathogen, preferably by *Helminthosporium turcicum,* more preferably by *Helminthosporium turcicum* races 0, 1 and/or N, in Zea mays in which the polypeptide is expressed, and preferably maps to bin 8.05 resistance locus or to bin 8.06 resistance locus and preferably encoding a polypeptide which is or belongs functionally to the family of wall associated receptor-like kinases 1 (WAK RLK1); and (b) identifying the allele, if the sequence comparison reveals (i) a sequence identity on nucleotide level of at least 85%, 90%, 92%, 94%, 96%, 98%, 99% or 99.5% identity to nucleotide positions 1-920 of the nucleotide sequence set forth in SEQ ID NO: 1 or SEQ ID NO: 2, preferably over the full length, and/or of at least 60%, 65%, 70%, 75%, 80%, 85%, 90%, 92%, 94%, 96%, 98%, 99% or 99.5% identity to nucleotide positions 23252-23288 of the nucleotide sequence set forth in SEQ ID NO: 1 or to nucleotide positions 921-957 of the nucleotide sequence set forth in SEQ ID NO: 2, preferably over the full length, and/or of at least 98%, 98,5%, 99% or 99.5% identity to nucleotide positions 23586-24632 of the nucleotide sequence set forth in SEQ ID NO: 1 or to nucleotide positions 958-2004 of the nucleotide sequence set forth in SEQ ID NO: 2, preferably over the full length, and/or (ii) a sequence identity of encoded amino acid level of at least 75%, 80%, 85%, 90%, 92%, 94%, 96%, 98%, 99% or 99.5% identity to the positions 1-306 of amino acid sequence set forth in SEQ ID NO: 3, preferably over the full length, and/or of at least 60%, 65%, 70%, 75%, 80%, 85%, 90%, 92%, 94%, 96%, 98%, 99% or 99.5% identity to positions 307-319 of amino acid sequence set forth in SEQ ID NO: 3, preferably over the full length, and/or of at least 98%, 98,5%, 99% or 99.5% identity to positions 320-668 of amino acid sequence set forth in SEQ ID NO: 3, preferably over the full length.

Preferably, the consensus sequence is derived from a set of at least two nucleotide sequences wherein one nucleotide sequence is the nucleotide sequence of the invention, preferably the nucleotide sequence set forth in SEQ ID NO: 1 or SEQ ID NO: 2 or the nucleotide sequence encoding the amino acid sequence set forth in SEQ ID NO: 3, or a part thereof, and wherein a further nucleotide sequence is selected from the group consisting of the nucleotide sequence set forth in SEQ ID NO: 9 or a part thereof, the nucleotide sequence set forth in SEQ ID NO: 11 or a part thereof, the nucleotide sequence set forth in SEQ ID NO: 13 or a part thereof, the nucleotide sequence set forth in SEQ ID NO: 23 or a part thereof, a nucleotide sequence encoding the amino acid sequence set forth in SEQ ID NO: 10 or a part thereof, a nucleotide sequence encoding the amino acid sequence set forth in SEQ ID NO: 12 or a part thereof, a nucleotide sequence encoding the amino acid sequence set forth in SEQ ID NO: 14 or a part thereof, or a nucleotide sequence encoding the amino acid sequence set forth in SEQ ID NO: 24 or a part thereof.

The at least one coding nucleotide sequence isolated from a *Zea mays* genotype of step (a) (i) can be derived from a plant sequence database or from a gene bank where seed samples of various *Zea mays* genotype are deposited. Plant sequence databases or gene banks are known in the art.

The method of identifying a (new) allele of a resistance gene (or a potential new allele of a resistance gene) conferring resistance or increased resistance to a plant disease caused by a fungal pathogen may further comprise, e.g. as step (c), the step of determining the resistance level of the plant against the fungal pathogen caused by the identified allele.

A nucleic acid molecule comprising or consisting of a nucleotide sequence encoding an allele of a resistance gene (or potential new allele) identified by the method of identifying described above is also provided. Said allele may be used in a method of producing a plant, preferably a plant of the species *Zea mays,* or in a method for conferring/increasing resistance to a plant disease which is caused by a fungal pathogen such as *Helminthosporium turcicum,* preferably by *Helminthosporium turcicum* races 0, 1, 2, 3, N, 12, 23, 2N, 12N, 23N and/or 123N. In one embodiment, the method according to the invention of producing a plant or the method according to the invention for conferring/increasing resistance to a plant disease further comprises in step (a) the introduction of at least one nucleic acid molecule comprising or consisting of a nucleotide sequence encoding the new resistance gene into the at least one cell of the plant.

In another aspect, provided is a method for conferring or increasing resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, in a plant, preferably a plant of the species *Zea mays,* comprising the following steps: (a) introducing into at least one cell of the plant the nucleic acid molecule of the present invention, including for example a nucleic acid molecule comprising or consisting of a nucleotide sequence of the identified allele, the expression cassette of the invention or the vector of the invention, (b) regenerating the plant from the at least one cell, and (c) causing expression of the nucleic acid molecule in the plant.

In a preferred embodiment of the method for conferring or increasing resistance to a plant disease caused by a fungal pathogen, step (a) results in the modification of an endogenous nucleic acid molecule conferring susceptibility to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, preferably mapping to bin 8.05 resistance locus or to bin 8.06 resistance locus on chromosome 8 of *Zea mays,* and preferably encoding a polypeptide which is or belongs functionally to the family of wall associated receptor-like kinases 1 (WAK RLK1), wherein the modification converts the endogenous nucleic acid molecule into the nucleic acid molecule of the present invention conferring resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, if expressed.

In another preferred embodiment of the method for conferring or increasing resistance to a plant disease caused by a fungal pathogen, step (a) results in the modification of an endogenous nucleic acid molecule conferring resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, preferably mapping to bin 8.05 resistance locus or to bin 8.06 resistance locus on chromosome 8 of *Zea mays,* and preferably encoding a polypeptide which is or belongs functionally to the family of wall associated receptor-like kinases 1 (WAK RLK1), wherein the modification converts the endogenous nucleic acid molecule into the nucleic acid molecule of the present invention conferring increased resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, if expressed.

Further provided is a method for increasing resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, in a plant of the invention, comprising the steps of reducing the level of expression of the nucleic acid molecule of the present invention, including for example a nucleic acid molecule comprising or consisting of a nucleotide sequence of the identified allele, the expression cassette of the invention or the vector of the invention, in the plant or at least one cell of the plant, preferably compared to the expression level of the endogenous gene in a resistant wild type plant. Such resistant wild type plant is for instances selected from the lines A619HT2, B37HT2 or B73HT2. Reducing can be conducted transiently or durably, preferably as preventive measure if infestation by the fungal pathogen in expected e.g. due to particular environmental conditions which drives the distribution of the fungal pathogen. A person skilled in the art is knowing very well various methodologies to reduce the expression of gene in plant. Durable reduction of expression level can be achieved e.g. by modulating the promoter or other regulatory elements by means of random or targeted mutagenesis or by stable integration of an expression cassette allowing the expression of double-stranded RNA, single-stranded antisense RNA or a hairpin DNA, which are able to silence as interfering RNA the mRNA encoded by the nucleic acid molecule of the invention. Such inhibitory RNA molecule, preferably in form of siRNA molecules, can also be used for transient reduction of the level of expression if applied to the plants as spray and taken up actively or passively by the plant cells, preferably cells of leaves.

In another aspect, provided is a method for producing a plant (or part, cell or seed thereof), preferably a plant of the species *Zea mays,* having (increased) resistance to a plant disease caused by fungal pathogen, preferably by *Helminthosporium turcicum,* more preferably by *Helminthosporium turcicum* races 0, 1, 2, 3, N, 12, 23, 2N, 12N, 23N and/or 123N, or a part, a cell or a seed thereof, comprising the following steps: (a) introducing into the plant or at least one cell of the plant the nucleic acid molecule of the present invention, including for example a nucleic acid molecule comprising or consisting of a nucleotide sequence of the identified allele, the expression cassette of the invention or the vector of the invention, (b) optionally, regenerating the plant from the at least one cell, and (c) causing expression of the nucleic acid molecule in the plant or part thereof.

In a preferred embodiment of the method for producing a plant (or part, cell or seed thereof) of the species *Zea mays,* step (a) results in the modification of an endogenous nucleic acid molecule conferring susceptibility to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, preferably mapping to bin 8.05 resistance locus or to bin 8.06 resistance locus on chromosome 8 of *Zea mays,* and preferably encoding a polypeptide which is or belongs functionally to the family of wall associated receptor-like kinases 1 (WAK RLK1), wherein the modification converts the endogenous nucleic acid molecule into the nucleic acid molecule of the present invention conferring resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, if expressed.

In another preferred embodiment of the method for producing a plant (or part, cell or seed thereof) of the species *Zea mays,* step (a) results in the modification of an endogenous nucleic acid molecule conferring resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, preferably mapping to bin 8.05 resistance locus or to bin 8.06 resistance locus on chromosome 8 of *Zea mays,* and preferably encoding a polypeptide which is or belongs functionally to the family of wall associated receptor-like kinases 1 (WAK RLK1), wherein the modification converts the endogenous nucleic acid molecule into the nucleic acid molecule of the present invention conferring increased resistance to a plant disease caused by a fungal pathogen, preferably by *Helminthosporium turcicum,* more preferably by *Helminthosporium turcicum* races 0, 1, 2, 3, N, 12, 23, 2N, 12N, 23N and/or 123N, if expressed.

Preferably, the method is for producing a plant (or part, cell or seed thereof) having increased resistance to a plant disease caused by fungal pathogen, such as *Helminthosporium turcicum,* preferably by *Helminthosporium turcicum* races 0, 1, 2, 3, N, 12, 23, 2N, 12N, 23N and/or 123N. Accordingly, the method may comprise the additional step (d) of selecting a plant that has increased resistance compared to a reference plant. Preferably, the reference plant is a plant of the same species, more preferably the reference plant is a wild-type plant of the same species or a plant isogenic to the plant selected in step (d), expect for the nucleic acid molecule of the present invention that has introduced in step (a) or modified during step (a) as described above.

Preferably, in the methods of the invention, the nucleic acid molecule of the invention is expressed in the plant or part thereof in an amount and/or period sufficient to increase or confer resistance to a plant disease caused by a fungal pathogen in the plant.

In another aspect, provided is a plant produced by the methods of the invention, or progeny, fruit, or seed thereof. Preferably, the plant or progeny, fruit, or seed thereof comprises the nucleic acid molecule of the invention, or the vector of the invention, or the protein of the invention, and/or the cell of the invention.

In another aspect, provided is the use of at least one nucleic acid molecule of the invention, including for example a nucleic acid molecule comprising or consisting of a nucleotide sequence of the identified new allele of a resistance gene, in the production of a plant having increased resistance to a plant disease caused by a fungal pathogen, preferably *Helminthosporium turcicum,* more preferably *Helminthosporium turcicum* races 0, 1, 2, 3, N, 12, 23, 2N, 12N, 23N and/or 123N. The plant produced may be a genetically edited or a transgenic plant.

Further provided is a method for controlling infestation of a fungal pathogen, preferably *Helminthosporium turcicum,* more preferably *Helminthosporium turcicum* races 0, 1, 2, 3, N, 12, 23, 2N, 12N, 23N and/or 123N, comprising the following steps: (a) growing plants of the present invention on agricultural and horticultural fields, and (b) causing expression of the nucleic acid molecule of the present invention, including for example a nucleic acid molecule comprising or consisting of a nucleotide sequence of the identified allele, the expression cassette of the invention or the vector of the invention, in the plants.

Another aspect of the invention is an oligonucleotide having a length of at least 15, 16, 17, 18, 19 or 20, preferably at least 21, 22, 23, 24 or 25, more prefered at least 30, 35, 40, 45, 50, 100, 200, 300 or 500 nucleotides, wherein the oligonucleotide is able to hybridize or anneal to (i) the nucleic acid molecule of the invention, (ii) the nucleic acid molecule comprising the nucleotide sequence of SEQ ID NO: 4 or SEQ ID NO: 5, or (iii) nucleic acid molecule complementary to (i) or (ii). Preferably, the oligonucleotide comprises a nucleotide sequence having an identity of at least 90%, 92%, 94%, 95%, 96%, 97%, 98%, 98.5%, 99%, 99,5% or 100% to the nucleotide sequence of the nucleic acid molecule of (i), (ii) or (iii) to which the oligonucleotide is able to hybridize or anneal.

Further provided is a pair of oligonucleotides or a kit comprising said oligonucleotides, wherein the oligonucleotides are suitable to anneal as forward primer and reverse primer to a region in the plant genome, preferably the *Zea mays* genome, which shows a cosegregation, preferably a perfect cosegregation, with the nucleic acid molecule of the invention. Preferably, the pair comprises one or two oligonucleotides of the invention. Preferably, the kit comprises one, two or more oligonucleotides of the invention

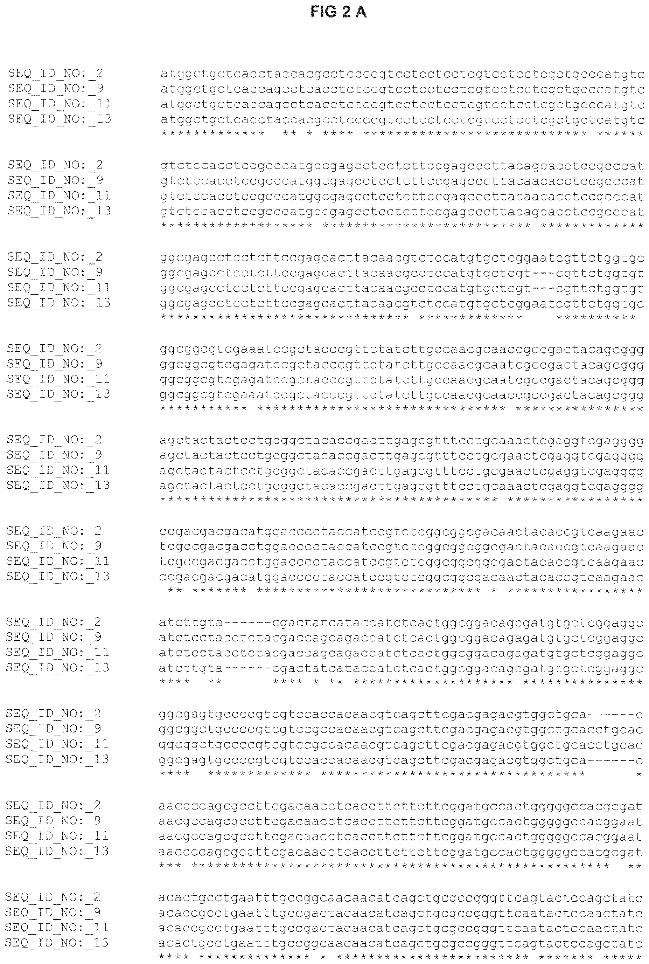

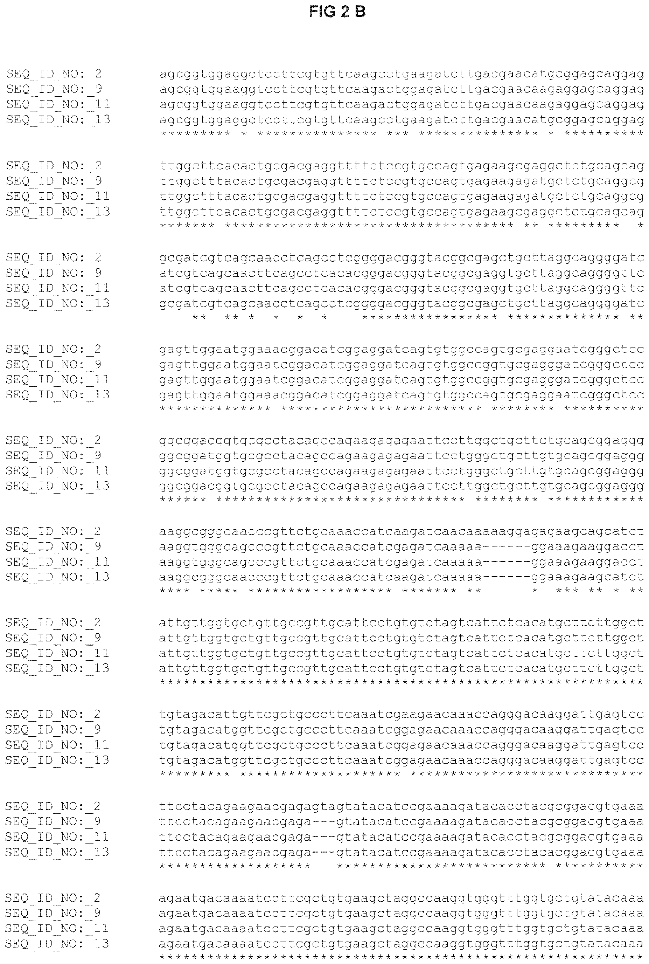

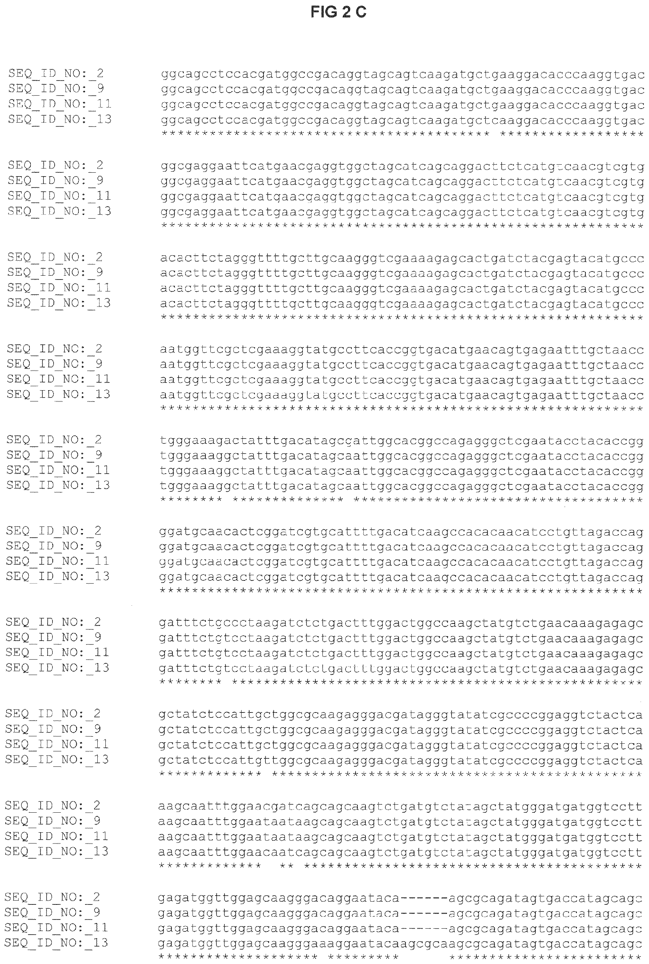

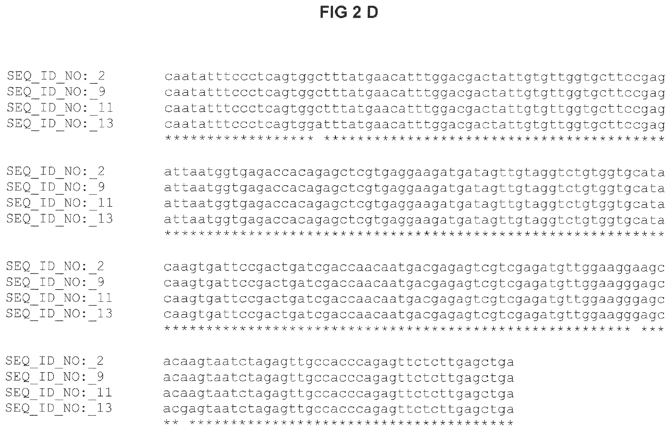

In a further aspect, provided is the use of at least one oligonucleotide as a molecular marker or part thereof in the method of identifying or selecting a plant, preferably a plant of the species *Zea mays,* having increased resistance to a plant disease caused by fungal pathogen, preferably by *Helminthosporium turcicum,* more preferably by *Helminthosporium turcicum* races 0, 1 and/or N, or a part, a cell or a seed thereof, or in the method of identifying an allele of a resistance gene conferring or increasing resistance to a plant disease caused by a fungal pathogen, preferably by *Helminthosporium turcicum,* more preferably by *Helminthosporium turcicum* races 0, 1 and/or N, in *Zea mays,* wherein the molecular marker is able to detect at least one single nucleotide polymorphism, deletion or insertion diagnostic for the nucleic acid molecule of the present invention and/or comprises the oligonucleotide as described above and/or is the pair of oligonucleotides or the kit as described above. Such single nucleotide polymorphism, deletion or insertion can be directly derived from the sequence alignment shown in FIG 2. Preferably, the at least one single nucleotide polymorphism, deletion or insertion results in the exchange, deletion or insertion of at least one amino acid. Characteristic amino acid exchanges, deletion or insertions from a comparison between the polypeptide having the amino acid sequence set forth in SEQ ID NO: 3 and RLK1 polypeptide (SEQ ID NO: 10) conferring the "Pepitilla" resistance phenotype as disclosed in WO2015/032494 are shown in Table 2.

In another aspect, provided is a method for detecting the presence or absence of the nucleic acid molecule of the invention, including for example a nucleic acid molecule comprising or consisting of a nucleotide sequence of the identified allele, in a plant, preferably a plant of the species *Zea mays,* comprising the following steps: (a) isolating DNA from at least one cell of the plant, and (b) using a molecular marker to detect the presence or absence of the nucleic acid molecule of the invention, wherein the molecular marker is able to detect at least one single nucleotide polymorphism, deletion or insertion diagnostic for the nucleic acid molecule of the present invention and/or comprises the oligonucleotide as described above and/or is the pair of oligonucleotides or the kit as described above.

In one aspect, provided is the use of at least one or at least two oligonucleotide(s) as a molecular marker or part thereof in method or process of identifying a plant as described above in a method or process for the elimination of linkage drag close linked to the nucleic acid molecule of the invention for detecting the presence or absence of an A619HT2 or A619HT3 derived interval located between alleles of marker SYN14136 and marker MA0021 or an A619HT2 or A619HT3 derived interval located between alleles of marker MA0022 and marker SYN4196. In a preferred embodiment, at least one oligonucleotide is used as molecular marker or part thereof in a method or process for the elimination of linkage drag close linked to the nucleic acid molecule of the invention for detecting the presence or absence of an A619HT2 or A619HT3 derived interval located between alleles of marker SYN14136 and marker PZE108077560, an A619HT2 or A619HT3 derived interval located between alleles of marker PZE108093423 and marker MA0021, and/or an A619HT2 or A619HT3 derived interval located between alleles of marker MA0022 and marker SYN4196.

In another aspect, provided is a method for detecting the presence of the nucleic acid molecule of the invention, including for example a nucleic acid molecule comprising or consisting of a nucleotide sequence of the identified allele, in a plant, preferably a plant of the species *Zea mays,* comprising the following steps: (a) isolating DNA from at least one cell of the plant, and (b) using a molecular marker to detect the presence or absence of the nucleic acid molecule of the invention, wherein the molecular marker is able to detect at least one single nucleotide polymorphism, deletion or insertion diagnostic for the nucleic acid molecule of the present invention and/or comprises the oligonucleotide as described above and/or is the pair of oligonucleotides or the kit as described above. Such single nucleotide polymorphism, deletion or insertion can be directly derived from the sequence alignment shown in FIG 2. Preferably, the at least one single nucleotide polymorphism, deletion or insertion results in the exchange, deletion or insertion of at least one amino acid. Characteristic amino acid exchanges, deletion or insertions from a comparison between the polypeptide having the amino acid sequence set forth in SEQ ID NO: 3 and RLK1 polypeptide conferring the "Pepitilla" resistance phenotype (SEQ ID NO: 10) as disclosed in WO2015/032494 are shown in Table 3.

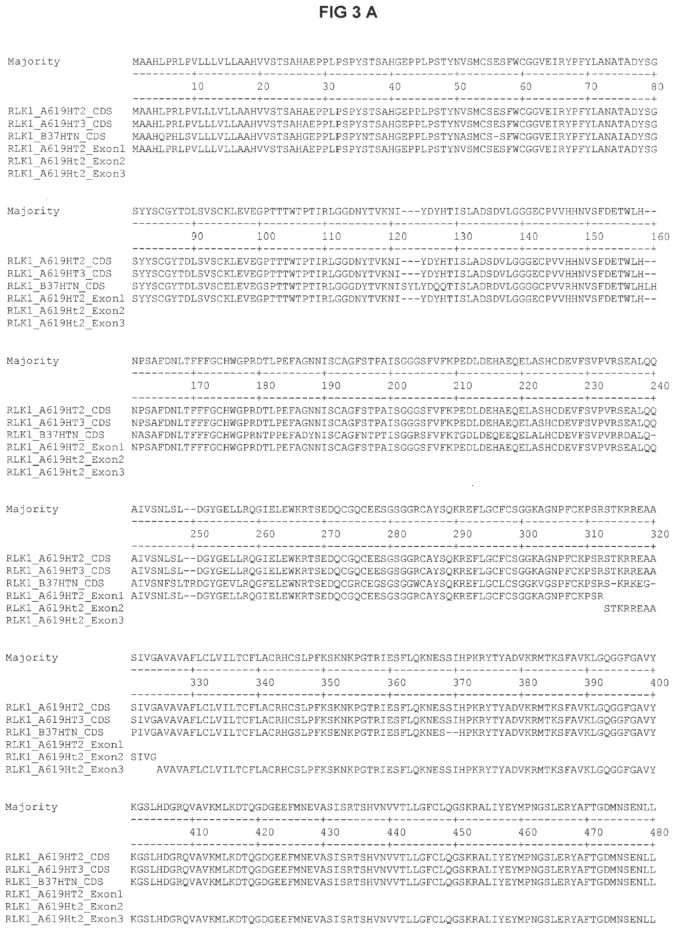

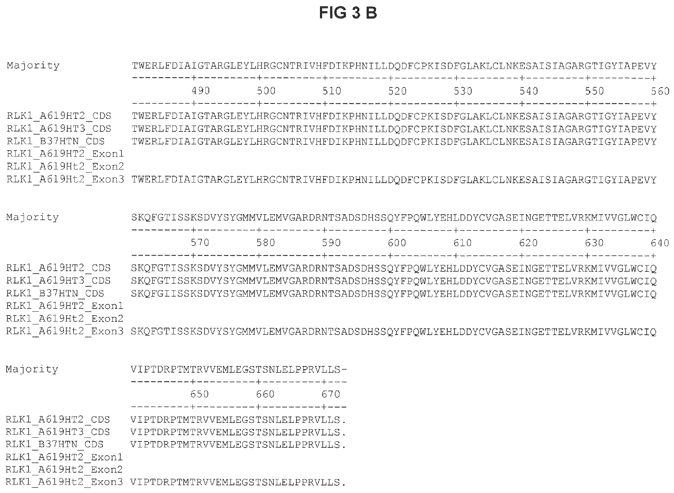

**Table 3: Characteristic amino acid exchanges, deletion or insertions from a comparison between the polypeptide having the amino acid sequence set forth in SEQ ID NO: 3 and RLK1 polypeptide (SEQ ID NO: 10) conferring the "Pepitilla" resistance phenotype as disclosed in WO2015/032494 (see also FIG 3).**

| **Position** | **HTN** | **HT2** | **effect** | **Exon** |
|---|---|---|---|---|
| 5 | Q | L | strong | Exon 1 |
| 7 | H | R | weak | Exon 1 |
| 9 | S | P | weak | Exon 1 |
| 27 | G | A | weak | Exon 1 |
| 36 | N | S | weak | Exon 1 |
| 51 | A | V | strong | Exon 1 |
| 56 | | E | | Exon 1 |
| 75 | I | T | strong | Exon 1 |
| 95 | E | K | weak | Exon 1 |
| 101 | S | P | weak | Exon 1 |
| 102 | P | T | weak | Exon 1 |
| 114 | G | D | strong | Exon 1 |
| 115 | D | N | weak | Exon 1 |
| 122 | S | | | Exon 1 |
| 123 | Y | | | Exon 1 |
| 127 | Q | Y | weak | Exon 1 |
| 128 | Q | H | moderate | Exon 1 |
| 135 | R | S | moderate | Exon 1 |
| 142 | G | E | strong | Exon 1 |
| 147 | R | H | moderate | Exon 1 |
| 157 | L | | | Exon 1 |
| 158 | H | | | Exon 1 |
| 162 | A | P | moderate | Exon 1 |
| 180 | N | D | weak | Exon 1 |
| 182 | P | L | strong | Exon 1 |
| 187 | D | G | strong | Exon 1 |
| 188 | Y | N | weak | Exon 1 |
| 196 | N | S | weak | Exon 1 |
| 199 | T | A | moderate | Exon 1 |
| 204 | R | G | strong | Exon 1 |
| 210 | T | P | weak | Exon 1 |
| 211 | G | E | moderate | Exon 1 |
| 216 | Q | H | weak | Exon 1 |
| 217 | E | A | strong | Exon 1 |
| 223 | L | S | strong | Exon 1 |
| 235 | R | S | weak | Exon 1 |
| 236 | D | E | weak | Exon 1 |
| 239 | | Q | | Exon 1 |
| 246 | F | L | weak | Exon 1 |
| 249 | T | G | moderate | Exon 1 |
| 250 | R | | | Exon 1 |
| 256 | V | L | moderate | Exon 1 |
| 261 | F | I | strong | Exon 1 |
| 266 | N | K | weak | Exon 1 |
| 275 | R | Q | weak | Exon 1 |
| 278 | G | E | moderate | Exon 1 |
| 284 | W | R | strong | Exon 1 |
| 297 | L | F | weak | Exon 1 |
| 303 | V | A | strong | Exon 1 |
| 305 | S | N | weak | Exon 1 |
| 314 | | T | | Exon 2 |
| 315 | | K | | Exon 2 |
| 316 | K | R | weak | Exon 2 |
| 318 | K | E | weak | Exon 2 |
| 319 | E | A | strong | Exon 2 |
| 320 | G | A | weak | Exon 2 |
| 321 | P | S | weak | Exon 2 |
| 345 | G | C | strong | Exon 3 |
| 352 | E | K | weak | Exon 3 |
| 368 | | S | | Exon 3 |
| 566 | I | T | strong | Exon 3 |

### Definitions

The term "resistance" or "resistant" as regards a pathogen should be understood to mean the ability of a plant, plant tissue or plant cell to resist the damaging effects of the pathogen and extends from a delay in the development of disease to complete suppression of the development of the disease. The resistance may be complete or partial and may be specific or non-specific to the pathogen race. A conferred resistance may be a newly inherited resistance or an increase in a partial resistance which is already extant.

Resistance may be quantified by methods known in the art. For example, resistance to *Helminthosporium turcicum* may be quantified by determining classification scores using phenotyping experiments in accordance with the scheme shown in the Table 4 below. For example, a *Helminthosporium turcicum*-resistant maize plant in the meaning of the invention exhibits an "increased resistance" to *H. turcicum* by at least 1 classification score, preferably by at least 2 classification scores or at least 3 classification scores, and most preferably by at least 4 classification scores. Preferably, a maize plant in accordance with the invention exhibits resistance to at least one race of *Helminthosporium turcicum* which does not correspond to the known race specificity known in the prior art. In a particularly preferred embodiment, a maize plant in accordance with the invention is resistant to all known races of *Helminthosporium turcicum,* i.e. the conferred resistance is not race-specific and may be particularly advantageous in the formation of a broad resistance to *Helminthosporium turcicum.*

**Table 4: Classification score scheme for phenotyping experiments in field trials at various locations with natural and artificial H. turcicum inoculation (from the Deutsche Maiskomitee (DMK, German maize committee); AG variety 27.02.02; (DMK J. Rath; RP Freiburg H.J. Imgraben)**

| Classification score | Phenotype |
|---|---|
| 1 | Plants exhibit no symptoms of disease, 0% |
| 2 | Beginning of infestation, first small spots (less than 2 cm) visible. Less than 5% of leaf surface affected. |
| 3 | Some spots have developed on a leaf stage. Between 5-10% of leaf surface affected. |
| 4 | 10-20% of leaf surface affected. Clearly visible spots on several leaf stages. |
| 5 | 20-40% of leaf surface affected. Spots start to coalesce. |
| 6 | 40-60% of leaf surface affected. Systematic infestation visible on leaves. |
| 7 | 60-80% of leaf surface affected. Approximately half of leaves destroyed or dried out because of fungal infestation. |
| 8 | 80-90% of leaf surface affected. More than half of leaves destroyed or dried out because of fungal infestation. |
| 9 | 90-100% of leaf surface affected. The plants are almost completely dried out. |

The term "hybridize" or "hybridization" should be understood to mean a procedure in which a single stranded nucleic acid molecule agglomerates with a nucleic acid strand which is as complementary as possible, i.e. base-pairs with it. Examples of standard methods for hybridization have been described in 2001 by Sambrook et al. Preferably, this should be understood to mean that at least 60%, more preferably at least 65%, 70%, 75%, 80% or 85%, particularly preferably 90%, 91%, 92%, 93%, 94%, 95%, 96%, 97%, 98% or 99% of the bases of the nucleic acid molecule undergo base pairing with the nucleic acid strand which is as complementary as possible. The possibility of such agglomeration depends on the stringency of the hybridization conditions. The term "stringency" refers to the hybridization conditions. High stringency is when base pairing is more difficult, low stringency is when base pairing is easier. The stringency of the hybridization conditions depends, for example, on the salt concentration or ionic strength and the temperature. In general, the stringency can be increased by raising the temperature and/or by reducing the salt content. The term "stringent hybridization conditions" should be understood to mean those conditions under which a hybridization takes place primarily only between homologous nucleic acid molecules. The term "hybridization conditions" in this respect refers not only to the actual conditions prevailing during actual agglomeration of the nucleic acids, but also to the conditions prevailing during the subsequent washing steps. Examples of high stringent hybridization conditions are conditions under which primarily only those nucleic acid molecules that have at least 90% or at least 95% sequence identity undergo hybridization. Such high stringent hybridization conditions are, for example: 4 x SSC at 65°C and subsequent multiple washes in 0.1 x SSC at 65°C for approximately 1 hour. The term "high stringent hybridization conditions" as used herein may also mean: hybridization at 68°C in 0.25 M sodium phosphate, pH 7.2, 7 % SDS, 1 mM EDTA and 1 % BSA for 16 hours and subsequently washing twice with 2 x SSC and 0.1 % SDS at 68°C. Preferably, hybridization takes place under stringent conditions. Less stringent hybridization conditions are, for example: hybridizing in 4 x SSC at 37 °C and subsequent multiple washing in 1 x SSC at room temperature.

The present invention encompasses nucleic acid molecules comprising or consisting of a nucleotide sequence encoding a protein, said protein being derived from the amino acid sequence encoded by the nucleotide sequence of SEQ ID NO: 1 or SEQ ID NO: 2 or from the amino acid sequence set forth in SEQ ID NO: 3 by way of substitution, deletion and/or addition of one or more amino acid(s). Herein, the term "one or more amino acid(s)" refers to, for example, 1 to 50, 1 to 40, 1 to 30, or 1 to 20, preferably 1 to 10, more preferably 1 to 7, further preferably 1 to 5, and particularly preferably 1, 2, or 3 amino acids.

"Operably linked" means linked in a common nucleic acid molecule in a manner such that the linked elements are positioned and orientated with respect to each other such that transcription of the nucleic acid molecule can take place. A DNA which is operably linked with a promoter is under the transcriptional control of this promoter.

"Introducing" in the meaning of the present invention includes stable integration by means of transformation including Agrobacterium-mediated transformation, transfection, microinjection, biolistic bombardment, insertion using gene editing technology like CRISPR systems (e.g. CRISPR/Cas, in particular CRISPR/Cas9 or CRISPR/Cpf1), CRISPR/CasX, or CRISPR/CasY), TALENs, zinc finger nucleases or meganucleases, homologous recombination optionally by means of one of the above mentioned gene editing technology including preferably a repair template, modification of endogenous gene using random or targeted mutagenesis like TILLING or above mentioned gene editing technology, etc. The term "introducing" may or may not encompass the introgression using conventional breeding.

The term "transgenic", "transgenical" or "transgene" is understood to mean that the respective gene is an exogenous gene that was introduced into the plant. The exogenous gene may be derived from a species other than the plant species into which it is introduced. Alternatively, the respective gene may be a gene already present in the plant species into which it is introduced, so that one or more additional copies of said gene are present as a result introducing the transgene. A "transgenic plant" is a plant into the genome of which at least one polynucleotide, preferably a heterologous polynucleotide, has been integrated. Preferably, the polynucleotide has been integrated in a stable manner, which means that the integrated polynucleotide remains stable in the plant, is expressed and can also be stably inherited to descendants. The term "heterologous" means that the introduced polynucleotide originates, for example, from a cell or an organism with another genetic background of the same species or from another species, or is homologous with the prokaryotic or eukaryotic host cell, but then is localized in a different genetic environment and thus is different from any possible corresponding naturally occurring polynucleotide. A heterologous polynucleotide can be present in addition to a corresponding endogenous gene.

Plant "organs" are leaves, plant stems, stems, roots, vegetative buds, meristems, embryos, anthers, ovulae or fruit. Plant "parts" can mean a fusion of several organs, for example a flower or a seed or a part of an organ, for example a cross segment from the stem. Examples of plant "tissues" are callus tissue, storage tissue, meristematic tissue, embryogenic tissue, leaf tissue, bud tissue, root tissue, plant tumour tissue or reproductive tissue. The term "cells" should be understood to mean isolated plant cells with a cell wall or aggregates thereof or protoplasts, for example.

The term "genetically edited" means that an endogenous gene is modified e.g. by means of random mutagenesis, TILLING or gene editing technology. For example, according to the invention, the endogenous gene of a plant may be modified to confer resistance (or increased resistance) to a plant disease caused by a fungal pathogen. Genetic modification may, for example, be achieved using methods of random or targeted mutagenesis (such as gene editing), or homologous recombination (optionally, supported by gene editing tools) or combinations thereof.

As used herein, "a modification", means that the genetic sequence has changed by at least one nucleotide. This can occur by replacement of at least one nucleotide and/or a deletion of at least one nucleotide and/or an insertion of at least one nucleotide, as long as it results in a total change of at least one nucleotide compared to the nucleotide sequence before modification, thereby allowing the identification of the modification, e.g. by techniques such as sequencing or PCR analysis and the like, of which the skilled person will be well aware.

The term "allele" refers to one or two or more nucleotide sequences at a specific locus in the genome. A first allele is on one chromosome, a second allele on the sister chromosome at the same position. If the two alleles are different, they are heterozygous, and if they are the same, they are homozygous. Various alleles of a gene (gene alleles) differ in at least one SNP. Various alleles of a resistance gene may either confer resistance, possibly different level of resistance to a plant against a fungal pathogen, i.e. causes differ types of phenotypes of the plant in response to infestation with fungal pathogen, or constitute a variant of the gene which is not able to confer resistance, i.e. the resulting plant phenotype is susceptible to a fungal pathogen.

According to the present invention, the term "regulatory sequence" means a nucleotide sequence which influences the specificity and/or strength of expression, for example insofar as the regulatory sequence confers a specific tissue specificity. A regulatory sequence of this type may be localized upstream of the transcription initiation point of a minimum promoter, but also downstream thereof, for example in a transcribed but not translated leader sequence or within an intron.

A "molecular marker" or "marker" is a nucleotide sequence which is used as a reference or orientation point. A marker for recognizing a recombination event should be suitable for monitoring differences or polymorphisms in a plant population. For markers, these differences are on a DNA level and, for example, are polynucleotide sequence differences such as, for example, SSRs (simple sequence repeats), RFLPs (restriction fragment length polymorphisms), FLPs (fragment length polymorphisms) or SNPs (single nucleotide polymorphisms). The markers may be derived from genomic or expressed nucleic acids such as spliced RNA, cDNA or ESTs and may be based on nucleic acids which are used as probes or primer pairs and as such are suitable for amplifying a sequence fragment using PCR-based methods. Markers which concern genetic polymorphisms between parts of a population can be detected using established methods from the prior art (An Introduction to Genetic Analysis. 7th Edition, Griffiths, Miller, Suzuki et al., 2000). These include, for example: DNA sequencing, PCR-based, sequence-specific amplification, assaying of RFLPs, assaying of KASP, assaying of polynucleotide polymorphisms using allele-specific hybridization (ASH), detection of SSRs, SNPs or AFLPs. Methods for detecting ESTs (expressed sequence tags) and RAPD (randomly amplified polymorphic DNA) are also known. Depending on the context, the term "marker" in the description may also mean a specific chromosome position in the genome of a species where a specific marker (for example SNP) can be found.

The terms "distal" and "proximal" describe the position of a chromosomal interval or a genetic segment in relation to a specific reference point (for example a specific polynucleotide, another chromosomal interval or a gene) on a whole chromosome; "distal" means that the interval or the segment is localized on the side of the reference point distant from the chromosome centromere, and "proximal" means that the interval or the segment is localized on the side of the reference point close to the chromosome centromere.

"closely linked" means two loci, two intervals, two genetic segments (e.g. resistance gene and flanking regions) or two markers (marker loci) which are less than 15 cM, less than 12 cM, less than 10 cM, less than 8 cM, less than 7 cM, less than 6 cM, less than 5 cM, less than 4 cM, less than 3 cM, less than 2 cM, less than 1 cM, less than 0.5 cM, less than 0.2 cM, less than 0.1 cM distant from each other, established using the IBM2 neighbors 4 genetic map which is publicly available on the Maize GDB website, or which are less than 50 Mbp (mega base pairs), less than 40 Mbp, less than 30 Mbp, less than 25 Mbp, less than 20 Mbp, less than 15 Mbp, or less than 10 Mbp distant from each other.

The term "interval" or "chromosomal interval" means a continuous linear segment on a genomic DNA which is present in an individual chromosome in a plant or on a chromosome fragment and which is usually defined through two markers which represent the end points of the interval on the distal and proximal side. In this regard, the markers which define the ends of the interval may themselves also be a part of the interval. Furthermore, two different intervals might overlap. In the description, an interval is specified by the statement "between marker A and marker B". An end marker of an interval may also be localized in a defined marker region to one side of the interval. A marker region is then defined by providing two flanking markers and constitutes a chromosomal segment on which more markers might be located, in addition to the flanking markers. Flanking markers determine the end points of a marker region and are themselves still a part of the marker region. If both end markers of an interval are markers in different marker regions on both sides of an interval, the description specifies an interval by stating "between a marker in a marker region X which is flanked by the markers C and D and a marker in a marker region Y which is flanked by markers E and F". A marker region may extend over up to 500 000 base pairs (bp), and can preferably be between 100 000 and 400 000 bp in size, or can particularly preferably be between 140 000 and 315 000 bp in size.

The term "introgression" as used in connection with the present invention means the transfer of at least one desired gene allele on a genetic locus of a genetic background into another. As an example, an introgression of a desired gene allele at a specific locus may be transferred to a descendant by sexual crossing between two parents of the same species. Alternatively, for example, the transfer of a gene allele may also occur by recombination between two donor genomes in a fused protoplast, wherein at least one donor protoplast carries the desired gene allele in its genome. In each case the descendants, which then comprise the desired gene allele, can then be backcrossed again with a line which comprises a preferred genetic background and can be selected for the desired gene allele. The result is fixing of the desired gene allele in a selected genetic background.

A "Locus" is a position on a chromosome where one or more genes are found which cause an agronomic feature or influence one. In particular, "locus" as used here means the HT2-resistance locus which confers resistance against the pathogen *Helminthosporium turcicum* or at least against a race of *Helminthosporium turcicum.*

The term "allele" refers to one or two or more nucleotide sequences at a specific locus in the genome. A first allele is on a chromosome, a second on a second chromosome at the same position. If the two alleles are different, they are heterozygous, and if they are the same, they are homozygous. Various alleles of a gene (gene alleles) differ in at least one SNP. Depending on the context of the description, an allele also means a single SNP which, for example, allows for a distinction between the resistance donor and recurrent parent.

### Examples

The following examples, including the experiments conducted and the results achieved, are provided for illustrative purposes only and are not construed as limiting the present invention.

### 1. Cloning and functional validation of the resistance gene HT2 and HT3

### a. QTL mapping and development of recombinants

The donor line A619HT2 was crossed and backcrossed with the line RP1 to create a near isogenic line (NIL, RP1 HT2A) with the main fragment of the original donor A619HT2 on chromosome 8 and very few other small donor regions. This NIL RP1 HT2A was crossed with its recurrent parent RP1 to build up a F2 population. The same was done for RP2 x RP2HT3A (original donor was here A619HT3). The recurrent parents RP1 and RP2 were susceptible to NCLB (Scores 7-9; cf. Table 4), while the donor lines A619HT2 and A619HT3 are resistant (scores 1-3). The scores of NILs RP1 HT2A and RP2HT3A are 2-3 and 1-2, respectively.

The F2 populations were planted in the field with 720 individuals at the location Pocking, Germany. QTL mapping resulted in a peak (LOD value for population with RP2HT3A = 168.77; LOD value for population with RP1 HT2A = 44.02) for both populations on chromosome 8 (1.1 cM and 3.5 Mbp frame). No other significant peak was detected. Recombinant plants (No. total ∼ 2000) were developed in several generations until F11 status. QTL mapping in F2 and further fine mapping with recombinants narrowed down the chromosomal region down to a physical interval of 490 kbp (genetic interval = 0.2 cM). This interval includes the RLK1 gene.

### b. Molecular analysis of target region

From the lines RP1 HT2A and RP2HT3A non-gridded BAC libraries were developed and screened with 7 probes from the target locus. For the RLK1 candidate gene region a contiguous BAC contig could be developed for both donor lines. Sequence analysis revealed that the donor line A619HT2 and A619HT3 are identical for the target region (in 1 Mbp). The candidate gene RLK1 cDNA sequence shows 97 polymorphisms (DNA level; incl. SNPs and Indels/Deletions) and 61 amino acid changes (protein level) between the lines harboring the *HTN1* of WO 2015/032494 A2 and the *HT2* allele. Out of the 97 polymorphisms, only 14 single nucleotide polymorphisms (SNPs) result in silent amino acid exchanges which probably do not influence the activity of the resistance gene. All other polymorphisms change the structure of the protein significantly by substitution, addition or deletion. Table 3 shows 15 amino acid exchanges for which a strong effect on the protein structure is predicted. Among these, multiple genotype specific additional amino acids have been identified. Compared to RLK1 cDNA sequences set forth in SEQ ID NO: 11 and 13 from the donor line disclosed in WO 2011/163590 A1, A619HT2 and A619HT3 differ in 31 nucleotides and 12 amino acids.

### c. Functional validation of the HT2 allele of RLK1

### (i) Functional validation using EMS mutagenesis:

An EMS-mutagenized population from RP2HT3A was developed. The exonic regions 1 and 3 from RLK1 were screened and 3 positive mutants harbouring an amino acid change were detected (SEQ ID NOs: 16, 18 and 20). In RLK cDNA of Mutant WVE16-92125-001 G at position 1625 replaced by A (see also SEQ ID NO: 15), leading to an amino acid exchange from Gly to Asp at position 542 (see also SEQ ID NO: 16); in RLK cDNA of Mutant WVE16-92149-012 C at position 95 replaced by T (see also SEQ ID NO: 17), leading to an amino acid exchange from Pro to Leu at position 32 (see also SEQ ID NO: 18); in RLK cDNA of Mutant WVE16-92168-005 G at position 115 replaced by A (see also SEQ ID NO: 19), leading to an amino acid exchange from Ala to Thr at position 39 (see also SEQ ID NO: 20). After selfing of these mutants, they have been evaluated in the field and greenhouse.

### d. Expression analysis:

The expression analysis of RLK1 in RP1, RP1 HT2A, RP2 and RP2HT3A in non-infected and infected leaf material showed a similar expression in the NILs RP1 HT2A and RP2HT3A. The expression is down-regulated in the infected leaf material. This response to infection could also be shown for the *HTN1* allele of RLK1.

### 2. RLK1 allelic series and identification of the relevant region for the resistance reaction

In order to identify a relevant region for the resistance reaction to the pathogen *Helminthosporium turcicum,* the following analysis are carried out: Bioinformatic analysis of re-sequencing of the RLK1 gene in different donor lines and recurrent parents reveals the amino acid region relevant for the resistance reaction. Therefore, genomic sequence primer pairs for RLK1 have been developed to cover the exonic regions. Exon 1 could only be covered partially, and Exon 2 and 3 completely. The developed amplicons were amplified and sequenced in 96 genotypes with the PACBio-sequencing technique. The sequences were assembled to consensus sequences per genotype. For some genotypes, multiple consensus-sequences were obtained. The consensus sequence with the highest amount of sequencing reads was chosen for an assembly of all 96 genotypes. The haplotypes were determined according to the exonic region/parts. For Exon 1, 12 different haplotypes; for Exon 2, 7 different haplotypes; and for Exon 3, 9 different haplotypes were detected. The genetic distance was calculated within the software Lasergene MegAlign (DNASTAR, Inc.). The different haplotypes were assembled on DNA and Protein sequence level (see Table 5). As a result, the Exon 1 and 2 regions seems to be highly variable for the gene and harbor the WAK-associated domains. This protein part is located in the intercellular space and could interact with fungal proteins. Variances in these two exons are interesting candidate base pairs for this interaction. On this basis, it is possible to identify haplotypes of Exon 1 and 2 in new alleles of RLK1 which are able to confer or increase resistance at least to the pathogen *Helminthosporium turcicum.*

Furthermore, phenotyping of different donor lines, near isogenic lines and recurrent parents and haplotype analysis of the RLK1 locus as well as expression analysis of RLK1 in different donor lines, near isogenic lines and recurrent parents combined with the phenotypic analysis are carried out for identification of relevant region for the resistance reaction and finally for identification of new allelic variants of the resistance gene. Evaluating all described datasets should narrow down the relevant region for the resistance reaction.

**Table 5: Genetic distances [%] between different exon-based haplotypes**

| Homology on DNA level | Homology on protein level |
|---|---|
| Total sequence: > 60% | Total sequence: > 60% |
| Exon 1: > 85% | Exon 1: > 75% |
| Exon 2: > 60% | Exon 2: > 60% |
| Exon 3: > 98% | Exon 3: > 98% |

### 3. Resistance reaction to other pathogens

A set of genotypes harboring the different RLK1 alleles have been inoculated with other plant pathogens like Southern corn leaf blight (*Bipolaris maydis*)*,* Common rust (*Puccinia sorghi*) and *Diplodia macrospora* (*Stenocarpella macrospora*)*.* A common feature of these pathogens is that the infection relies on a very similar biology of the fungi and on the fact that they penetrate the host via leaf tissue. A first experiment with Southern corn leaf blight (*Bipolaris maydis*) indicates also a resistance reaction of the HT2- and HTN-allele of RLK1.

### 4. Introducing resistance to NCLB caused by Helminthosporium turcicum into a susceptible genotype via Agrobacterium-mediated transformation

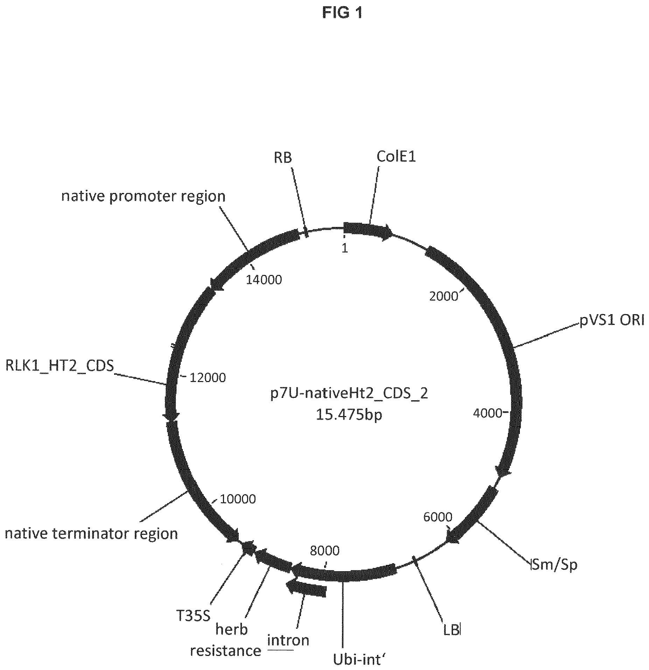

A construct with the HT2 cDNA sequence (SEQ ID NO: 2), the native promoter region of ∼1980 bp (SEQ ID NO: 4) and terminator region (SEQ ID NO: 5) (vector p7U, see Fig. 1) was transformed into the susceptible maize genotype A188. To produce the vector p7U-nativeHT2_CDS_2, the expression cassette containing the HT2 gene under control of the native promoter and terminator was transformed into a binary vector containing a herbicide gene (e.g.: BASTA resistance, glyphosate resistance or ALS inhibitor resistance) for subsequent transformation into *Agrobacterium tumefaciens* for Agrobacterium mediated plant transformation into maize (*Zea mays*) genotype A188. T0 plants have been multiplied and progenies grown in greenhouse are tested for resistance to NCLB.

### 5. Introducing resistance to NCLB caused by Helminthosporium turcicum into a susceptible genotype via gene editing

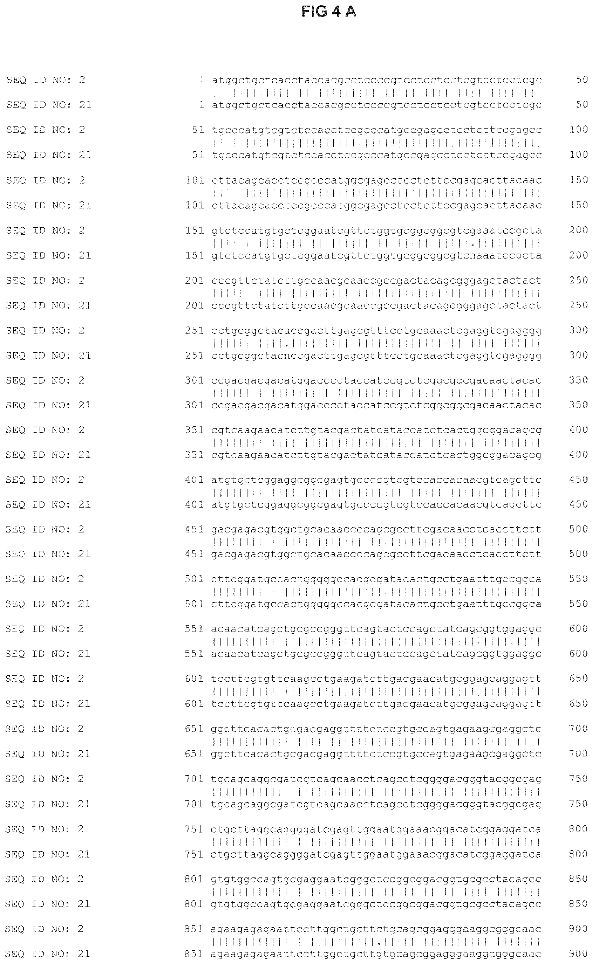

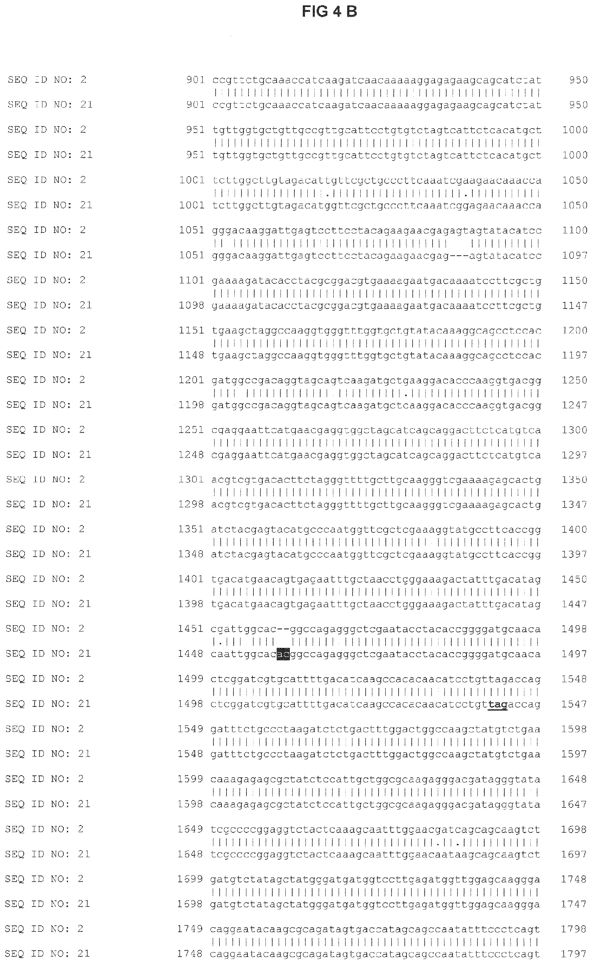

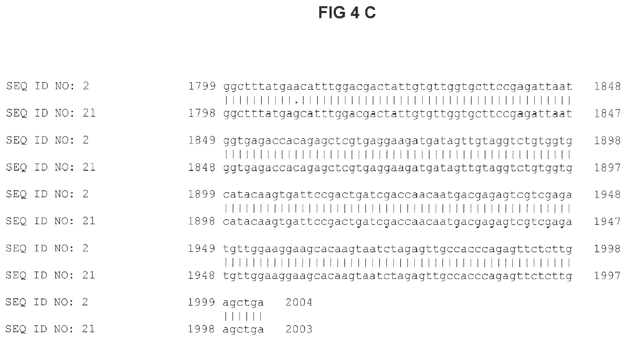

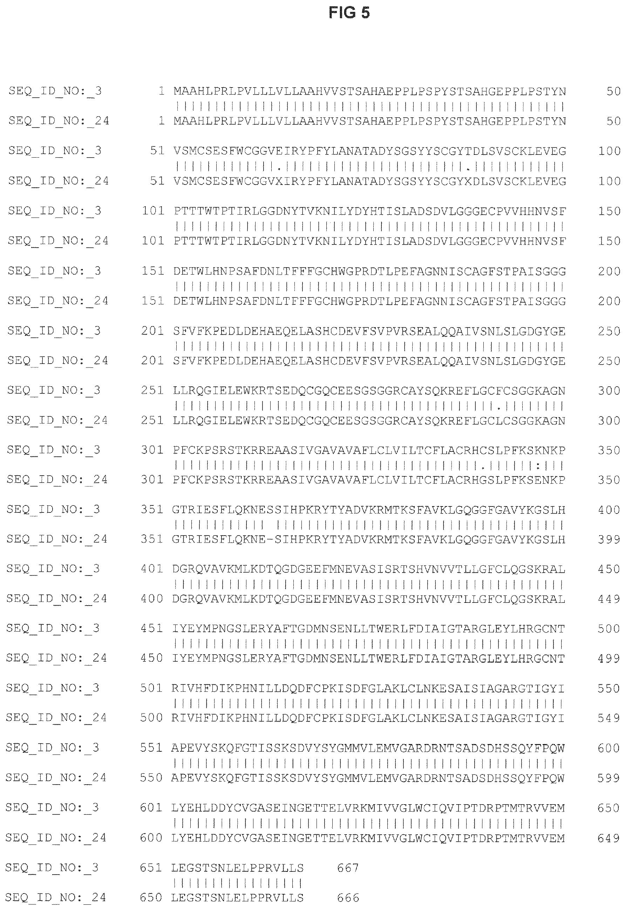

The allele of RLK1 in susceptible maize genotype A188 has been identified, sequenced and a cDNA predicted (SEQ ID NO: 21). A comparison between the cDNA of this A188 allele and cDNA of HT2 shows that the sequences show a sequence identity of 99% (FIG 4). However, there is at a position of 1458 to 1459 of the A188 allele a 2 bp insertion "AC" which causes an early stop codon after a Cysteine at position 513 (SEQ ID NO: 22). By elimination of this 2 bp insertion using gene editing based in TALENS or CRISPR systems, the HT2 resistance could be restored in the genotype A188. Figure 5 shows in the alignment between the RLK1 protein derived from the modified RLK1 allele of A188 (SEQ ID NO: 24) and the HT2 allele the high level of identity on amino acid sequence. The cDNA of the modified RLK1 allele from A188 is shown in SEQ ID NO: 23.

## Claims

1. Nucleic acid molecule comprising a nucleotide sequence selected from the group consisting of:
(a) a nucleotide sequence set forth in SEQ ID NO: 1 or SEQ ID NO: 2;
(b) a nucleotide sequence encoding an amino acid sequence set forth in SEQ ID NO: 3;
(c) a nucleotide sequence having at least 96% identity to the nucleotide sequence set forth in SEQ ID NO: 1 or SEQ ID NO: 2;
(d) a nucleotide sequence encoding an amino acid sequence having at least 92% identity to the amino acid sequence set forth in SEQ ID NO: 3;
(e) a nucleotide sequence hybridizing with the complementary strand of a nucleotide sequence as defined in (a) or (b) under stringent hybridization conditions; and
(f) a nucleotide sequence encoding a protein derived from the amino acid sequence encoded by the nucleotide sequence of (a) or (b) by way of substitution, deletion and/or addition of one or more amino acid(s) of the amino acid sequence encoded by the nucleotide sequence of (a) or (b);
wherein the nucleic acid molecule is encoding a polypeptide capable of conferring or increasing resistance to a plant disease caused by fungal pathogen in a plant in which the polypeptide is expressed.

2. A method of identifying an allele of a resistance gene, wherein the allele confers increased resistance to a plant disease caused by a fungal pathogen in *Zea mays,* comprising the following steps:
(a) conducting sequence comparisons using (i) at least one coding nucleotide sequence originating from a *Zea mays* genotype, wherein the nucleotide sequence preferably maps to bin 8.05 resistance locus or to bin 8.06 resistance locus, and (ii) as reference sequence, a nucleotide sequence of the nucleic acid molecule of claim 1 or a part thereof, or a consensus sequence derived from a set of at least two nucleotide sequences wherein one nucleotide sequence is the nucleotide sequence of the nucleic acid molecule of claim 1 or a part thereof, and wherein each nucleotide sequence of the set of at least two nucleotide sequences encodes a polypeptide capable of conferring or increasing resistance to a plant disease caused by fungal pathogen in *Zea mays* in which the polypeptide is expressed, and preferably maps to bin 8.05 resistance locus or to bin 8.06 resistance locus; and
(b) identifying the allele, if the sequence comparison reveals
i. a sequence identity on nucleotide level of at least 85% identity to nucleotide positions 1-920 of the nucleotide sequence set forth in SEQ ID NO: 1 or SEQ ID NO: 2 and/or of at least 60% identity to nucleotide positions 23252-23288 of the nucleotide sequence set forth in SEQ ID NO: 1 or to nucleotide positions 921-957 of the nucleotide sequence set forth in SEQ ID NO: 2 and/or of at least 98% identity to nucleotide positions 23586-24632 of the nucleotide sequence set forth in SEQ ID NO: 1 or to nucleotide positions 958-2004 of the nucleotide sequence set forth in SEQ ID NO: 2, and/or
ii. a sequence identity on amino acid level of at least 75% identity to the positions 1-306 of amino acid sequence set forth in SEQ ID NO: 3 and/or of at least 60% identity to positions 307-319 of amino acid sequence set forth in SEQ ID NO: 3 and/or of at least 98% identity to positions 320-668 of amino acid sequence set forth in SEQ ID NO: 3.

3. A nucleic acid molecule comprising or consisting of a nucleotide sequence of the allele identified by the method of claim 2.

4. A vector or expression cassette comprising the nucleic acid molecule of claim 1 or 3, preferably operably linked to a promoter allowing expression of the nucleotide sequence in a plant cell.

5. A polypeptide encoded by the nucleic acid molecule of claim 1 or 3.

6. A plant or part thereof comprising the nucleic acid molecule of claim 1 or 3, the vector or expression cassette of claim 4, or the polypeptide of claim 5.

7. The plant of claim 6, wherein the plant is a genetically modified plant or transgenic plant.

8. The plant or part thereof of claim 6, wherein the plant or part thereof comprising the nucleic acid molecule of claim 1 endogenously and the genomic flanking regions closely linked to the nucleic acid molecule does not contain an A619HT2 or A619HT3 derived interval located between alleles of marker SYN14136 and marker MA0021 or an A619HT2 or A619HT3 derived interval located between alleles of marker MA0022 and marker SYN4196.

9. A seed of the plant of claim 6 comprising the nucleic acid molecule of claim 1 or 3, the vector or expression cassette of claim 4, or the protein of claim 6.

10. A method of identifying or selecting a plant having increased resistance to a plant disease caused by a fungal pathogen, or a part, a cell or seed thereof, comprising the following steps:
(a) detecting in the plant, or part, cell or seed thereof, the presence of the nucleic acid molecule of claim 1, or a nucleic acid molecule comprising or consisting of a nucleotide sequence selected from the group consisting of:
i. a nucleotide sequence having at least 60% identity to the nucleotide sequence set forth in SEQ ID NO: 1 or SEQ ID NO: 2;
ii. a nucleotide sequence encoding an amino acid sequence having at least 60% identity to the amino acid sequence set forth in SEQ ID NO: 3, wherein the nucleic acid molecule is encoding a polypeptide capable of increasing resistance to a plant disease caused by fungal pathogen in a plant in which the polypeptide is expressed;
iii. to nucleotide positions 1-920 of the nucleotide sequence set forth in SEQ ID NO: 1 or SEQ ID NO: 2 (Exon 1; SEQ ID NO: 6) and/or having at least 60% identity to nucleotide positions 23252-23288 of the nucleotide sequence set forth in SEQ ID NO: 1 (Exon 2; SEQ ID NO: 7) or to nucleotide positions 921-957 of the nucleotide sequence set forth in SEQ ID NO: 2 (Exon 2; SEQ ID NO: 7) and/or having at least 98% identity to nucleotide positions 23586-24632 of the nucleotide sequence set forth in SEQ ID NO: 1 (Exon 3; SEQ ID NO: 8) or to nucleotide positions 958-2004 of the nucleotide sequence set forth in SEQ ID NO: 2;
iv. a nucleotide sequence encoding an amino acid sequence having at least 75% identity to the positions 1-306 of amino acid sequence set forth in SEQ ID NO: 3 and/or having at least 60% identity to positions 307-319 of amino acid sequence set forth in SEQ ID NO: 3 and/or having at least 98% identity to positions 320-668 of amino acid sequence set forth in SEQ ID NO: 3;
wherein the nucleic acid molecule of any one of (i) to (iv) is encoding a polypeptide capable of conferring or increasing resistance to a plant disease caused by fungal pathogen in a plant in which the polypeptide is expressed;
(b) identifying or selecting the plant in which or in whose part, cell or seed the nucleic acid molecule as defined in (a) is present, as having a resistance or an increased resistance to a plant disease caused by a fungal pathogen.

11. A method for increasing resistance to a plant disease caused by a fungal pathogen in a plant, comprising the following steps:
(a) introducing into at least one cell of the plant the nucleic acid molecule of claim 1 or 3, or the vector or expression cassette of claim 4,
(b) regenerating the plant from the at least one cell, and
(c) causing expression of the nucleic acid molecule in the plant.

12. An oligonucleotide having a length of at least 15 nucleotides, wherein the oligonucleotide is able to hybridize or anneal to (i) the nucleic acid molecule of claim 1 or 3, (ii) the nucleic acid molecule comprising the nucleotide sequence of SEQ ID NO: 4 or SEQ ID NO: 5, or (iii) nucleic acid molecule complementary to (i) or (ii).

13. A pair of oligonucleotides or a kit comprising said oligonucleotides, wherein the oligonucleotides are suitable to anneal as forward primer and reverse primer to a region in the plant genome, preferably the Zea mays genome, which shows a cosegregation with the nucleic acid molecule of claim1 or 3.

14. A method for detecting the presence or absence of the nucleic acid molecule of claim 1 or 3 in a plant comprising the following steps:
(a) isolating DNA from at least one cell of the plant, and
(b) using a molecular marker to detect the presence or absence of the nucleic acid molecule of claim 1 or 3, wherein the molecular marker is able to detect at least one single nucleotide polymorphism, deletion or insertion diagnostic for the nucleic acid molecule and/or comprises the oligonucleotide of claim 13 and/or is the pair of oligonucleotides or the kit of claim 14.

15. A method for identifying a plant having an increased resistance to a plant disease caused by *Helminthosporium turcicum,* and comprises the nucleic acid molecule of the claim 1 endogenously, the method or process comprising detecting in the plant alleles of at least two markers, wherein at least one of said markers is on or within the chromosomal interval between SYN14136 and the nucleic acid molecule of the claim 1, and at least one of said markers is on or within the chromosomal interval between the nucleic acid molecule of the claim 1 and SYN4196.


SEQUENCE LISTING

<110>  KWS SAAT SE
       University of Zurich

<120>  Gene conferring resistance to fungal pathogen

<130>  KWS0267EP

<160>  42

<170>  PatentIn version 3.5

<210>  1
<211>  24632
<212>  DNA
<213>  Zea mays

<400>  1
   atggctgctc acctaccacg cctccccgtc ctcctcctcg tcctcctcgc tgcccatgtc      60
   gtctccacct ccgcccatgc cgagcctcct cttccgagcc cttacagcac ctccgcccat     120
   ggcgagcctc ctcttccgag cacttacaac gtctccatgt gctcggaatc gttctggtgc     180
   ggcggcgtcg aaatccgcta cccgttctat cttgccaacg caaccgccga ctacagcggg     240
   agctactact cctgcggcta caccgacttg agcgtttcct gcaaactcga ggtcgagggg     300
   ccgacgacga catggacccc taccatccgt ctcggcggcg acaactacac cgtcaagaac     360
   atcttgtacg actatcatac catctcactg gcggacagcg atgtgctcgg aggcggcgag     420
   tgccccgtcg tccaccacaa cgtcagcttc gacgagacgt ggctgcacaa ccccagcgcc     480
   ttcgacaacc tcaccttctt cttcggatgc cactgggggc cacgcgatac actgcctgaa     540
   tttgccggca acaacatcag ctgcgccggg ttcagtactc cagctatcag cggtggaggc     600
   tccttcgtgt tcaagcctga agatcttgac gaacatgcgg agcaggagtt ggcttcacac     660
   tgcgacgagg ttttctccgt gccagtgaga agcgaggctc tgcagcaggc gatcgtcagc     720
   aacctcagcc tcggggacgg gtacggcgag ctgcttaggc aggggatcga gttggaatgg     780
   aaacggacat cggaggatca gtgtggccag tgcgaggaat cgggctccgg cggacggtgc     840
   gcctacagcc agaagagaga attccttggc tgcttctgca gcggagggaa ggcgggcaac     900
   ccgttctgca aaccatcaag taaagtcctg aaccgaccct cccttatttt tttttcattt     960
   tttgcaatca ccagagcacg catcggttgc gtcagtatct tgcaacctcg tagctagccc    1020
   cgcagtgtcc cctgtgtgcg agtaccgcgc tgctccagct tgcctcctgc taacgcctaa    1080
   cggtgaatgc ttcatgcttg acatgatcta gctagtctac actttgcttg gggtttgcct    1140
   gggagctgga aattctggct cctgtttgca tcactcgaca aggacgcttt cagacttgcg    1200
   actctcgttc tgcttttgca ccaaatccgt gttttttcat ttcgtgatcg agattaatct    1260
   agcgtagaga tgtcaatggg tatccgaaac tcgaaacccg atggattttt actctattag    1320
   gtacaagttt gaatcaattt tcatatcata gatttgttaa taggcacaaa tctatatcca 1380
   acaggttcat agatacgggt ttgttcctac agtaatcaaa tccgtaaacc catgagtttt 1440
   ttagacccga ccaaacctag cgtatattgt cattttattt tataaacgaa caacaaaatt 1500
   attatctctc tatttacttc ctattttttt atcggttggt gaatgtataa gtagttggtg 1560
   agagtgttgc ttgcttgcta ttataatatt tagttattta ctagtgttat atatgtggtg 1620
   gatggataac ttattgaaaa ggtcacttga ttatacaact tattatttgt attcattctt 1680
   tctactaata atttttatat caaatcatga actcggtgtt tatcatataa attttggacc 1740
   atgatcttat taatcatcac gatagttatt aattatgaga aaaacaaata tattggagat 1800
   aaaaccctcg gctaacccgt taacccgatg ggcacgggtt tgaacaaaat ttcaaattta 1860
   ttatgaatac aagtttttta acaaatatag atatgtttca tggatagagt ttgagatgac 1920
   aaaatccaac ggatttgtat ccgttgccat ctctatccgg cggcccttac cgtgctccac 1980
   gagcagaggt cgtatcgtcc ctcttcccgt gtcgcctgct tcgcgttgcc gaacggagac 2040
   gtttggtagc gttggccggc tctagcagtc gggtcaattt ttttgttgtt gttttcgatg 2100
   ttgttggatt tttgttccgt ataagccatg ttttggtaat ttatttagtc cagccgaatc 2160
   cgaagacgtg tttgctgggt tggagacttt ggagttgcta gtcatgatat gctttctact 2220
   cggtttgatt tcaacccagt taggctatat ttgatactct agtatttatt ccaatataaa 2280
   tggtttgaaa gggattaaga tataaattag tttaatttat atctttaatt tctcttaacc 2340
   catatgtatt gggctgaata ctaagtatca aaacaagccc ttatttaaga tgcattttct 2400
   ttacagttac acgtggccac tattgtgtgg ggtaggccga tcctacttgt cagtgctcat 2460
   tcgagagcaa agataccgaa ggagattaga gagactaaaa gctttttact atttaaaatt 2520
   agataataag acgatttaat cccgttccat atctttgctc taaacaaacc ctcaatgatc 2580
   atatatctcg gaagatccgg ccggctgttc tttatttatc aagtgatgac tgctgaccgc 2640
   ttatagaata tatattttaa agcaaaattt cttctacatc agtaaaagga ctagacgaaa 2700
   caatgatgca tttctctaac aaaagaaagt agaattatca agcggagagc caagaccaaa 2760
   agccttacat ctatgggcgt caaccaatga taccgcgaac caccccagct ggtctatact 2820
   gtctgtcacg acccagcgag taaccgtgtg gctacgctgt tagcttagga ttgaatgaaa 2880
   tgctcggttc attaataaat cagctcacga gttaaatgat ttattatata acgaaactat 2940
   atgcatatta tttaccaggg tgacgacagg gggcataggt gggcaatatc caccctaatg 3000
   ctcctctaat tctataggga ttgttaattt atttagctaa ttatcatgta aacgatataa 3060
   aaaatgtttc taacagcccc acggtccacc ctaatcttag accctagctt cgcctgttac 3120
   gataaatatg ttatacatgt gtagtgtcta tgtcctatga attaaactaa tgattgatga 3180
   actgtgctta tatattaaat tggtctatgc gaatataact atgtgttaaa ctgatgaaca 3240
   tgtgtgtgaa ttgtgaattg ataagtgatg agttgtgtct aatttagtgt tatattgatg 3300
   tgttttgtga aactatgagt ataattaata ttttctataa ttaaatttgt ttaaaattaa 3360
   ctagaaattg attattatat atatatattg tttttctgct ctaattcgca agctaaacaa 3420
   gcaagctcaa gctcgtaaac gagccgaacc gagctgactc tgtggctcat taacttaaca 3480
   acccgagccg gaccaacttg ttagcttaac gagccagctc gaatacggac gagttgagcc 3540
   gagctggcat gatatccagc cctaattagg ctcgtaccag tgcaacatat ccctctcgcc 3600
   tttgtcacgt ccagacatgt caatgggccc cgattcccgc aaggaattcc tctattaagg 3660
   gatgatgatg agaaagtttc tcccccacgg aaaaattctc tcctgacgca taaacgggga 3720
   caacactcca catccccatt cctcgtgggg acctattaaa cttacatatg gtgatgtttt 3780
   catgtaaaag ttaatgataa aaataaataa ttacgttgtc aagatatcac tttttgtaca 3840
   aatgtgctca ttctgatgta gacatatttt ttcttacatc taacaatctg tatgagtgag 3900
   aatgttttgt attaataaca aagtaagtat gttctaatta taatttaagc gggtccgggg 3960
   atccccgatg gggatttata cccttgggaa acgaagatgg ggaagaaatg tcctacacaa 4020
   gttttcatgg cgatcctcgc ggggaacttt ttcgtcgtgg ggatagggat ggggagctaa 4080
   aacccgatga ggaattcctc gttgtcatcc ccagtcacgt caactctcga ctctactatt 4140
   gtctctgctg catagtctgt ggtaaactat agcctcagcc tatagcacat cagcttttct 4200
   atttcagaat taagtaattc atgcagtttt atttcaacgc tcgaaaatta cacaacataa 4260
   tattttttat tgaattaaaa attataaagt gcatacaaat caataaaaat atattataat 4320
   taaaacatat ataaatctaa tcggagacct cgatcttatc ctcgtcttcc accacctctt 4380
   tttgttttgc ttctggttcc tcttcttcgt catcaaccag accacgtcct ttttcgaaca 4440
   tctttatggc cttggcatgg gtttatcttt cagctaggtc catatccctg gttgaatggc 4500
   tctttagagt attccattgc tttaaaagct tggtcttcac taatccaccc cacaagttct 4560
   tcattttaaa tgcttcactt gttgactgtt gcttgtggct tccttccctt tccccttcac 4620
   cttcatcttt cgcccagccg cctttgccct atcacgccct actgatcggg gcacttcctt 4680
   cttggtatct ttagtgcctc cagaactata ttcactagag acactgagtc gggttatctt 4740
   attcgtagtc ccattttatc ctagaacctt gtcattgctg cctccatgac cattgcctcg 4800
   tctgcttcgc tctgatgcaa cttttcttct cgaaggtagt atccattaaa cttgctcacc 4860
   tcccggttgt aggtgcccta atgattttta agttgcttgg cggtttgagg atatcaaggg 4920
   tcggaggtgg agttatatgt tgccgctatt tgactccaaa aacatgatcc agacttattg 4980
   ttgacagggg ttgaattatt agaatgcata ttccaagaat taacctataa tataagtaaa 5040
   ctacaaatat aaatattaaa taaatgagaa atgattcact caccaacttt tcctcatagt 5100
   cattcatcca ttctagcctc tttggacact tttctttcgg cccaggattt tcctcggctt 5160
   ggttttcaaa tccaacatac tagacatgta tgtggaattg gaggaggtgc tcatacagag 5220
   gtggagcata tgcaggaggg gcatacagag gtactgagaa agaagtttga cttccatccg 5280
   cagcaggtgg aggggcatac agacgtaccg aggaaaaagc ttgaattctg gcttgggaag 5340
   ttggaggttg accatatgaa ggcggcaagt atgaatacag atgataggaa aaggttatgg 5400
   tggtggtgga cgcgcaactg aaaatagtgc atgggggact gaagctgaaa catcagagcg 5460
   acaatgtagt ggagagactc atcaaacgga ggggtttgtg acccatcgga ctacaagaga 5520
   tccatgaagg tgttcaaatc tggggaccaa cccgatattg tgtgaacaga ggggagtttg 5580
   gaagagagaa acgaaatgaa atggtgtgtg gaatgagctc aacccaagta gaggtactta 5640
   caaagggatt ggggatgaaa tttgtgattt tttgcaattt tttatttttt ggattccaaa 5700
   cgatcaaaaa caactagttg caatagctag ctgtggggga tagatatccc ccgggtccac 5760
   taagggagta aaagacctca cgaaaggccc aagggcccaa taaatcgtaa ggtcattctt 5820
   ccgtgggcct ggggagaaac aaccaacaaa acagagaaga cgtaagaccg gattggtgca 5880
   aacccggacg gcccacaacg tcgaacgagt aaatcgcaac agagacccga ctttcccgcg 5940
   ctggagcccc catgcaacgg agccatgcga ggataagtcg gcgagggtta cgtagggata 6000
   aactcaagag gttcactacc ttttagctac ttgttgttat catatccacg tgtactgccc 6060
   cacggtcgag tatataaggc ctagggggca ccccttcaga acgatcgacc ctatcttact 6120
   tagccaccca cagcaactct ctgtgtcttc aatccagaga gccctcttgt aaccacgctc 6180
   acatactcac caggacgtag ggtgttacgc atctctaagc ggcccgaacc tgtaaatctt 6240
   gtccactgtc cctcgtgtga tcggcacgaa ctgttttgct acagtcgtcg acaccgtcct 6300
   actcctagaa acaccttgag ggcaaccccc gggtgtgcgg ttggacccaa aacaccgaca 6360
   gctggcgcgc caggtagggg gtgtgtcgac gatccaagct agctcaatgg ccatcacctt 6420
   ctacagcaag atcaccatgc gtcctggatc cgtattctgc ttcgggacaa tctcatctgt 6480
   agcggatgaa gagggaattc tacaccgtat cgcagatccg ccggaaaaga agactcctcc 6540
   aacgaactcc gaaaatgccg gagaggcaca acctccagct cttcggaaaa agatcgtctc 6600
   cggaaaggcg gggccgaggg gctcgctgac ccggaagact ccgttgtcta cttcctcgac 6660
   gaaagaatgg acacggatca cgaggaagaa ggagaccggg gaaaagcaag tcgttctttc 6720
   cgttcctccg gtctcaaagg agaacggaaa gaaagtcgcc gcgacagcag caccattcta 6780
   ccccgacgtc ctcttcgtcg ggagagtgga gtcgcccacc gtctctgacg acgaaccgac 6840
   cgcgcccgga gaagagccgc ctcaacgaga atcccgccga cggagaaacc ggcgcaggaa 6900
   cgttcgacga catcacgcgg ccggagaacg ggatccggag cagcctgtct cgcgagacga 6960
   ggtctcggag ataggagaaa ctccggaaga acgcgtcttc agagaacgaa ggaactcccg 7020
   acgacgtgat cgccggcggg ctcaggaaca ggccgagcaa gatgcaaggc aacgccggga 7080
   gaatccgctc ttcgggcgca acctaaatcc cgacttcgcc cgagctatga acacgccaag 7140
   cgaagtcgga ggggtactag cttggatagc tgatggactt cctcgaactc ccgacgccga 7200
   gggataccga cgcctgttca cccaggcagc caaccatctt ctacctctcg ctcacccgcc 7260
   gaacgatcta cgacacgcca tcaacagtcg ccgagacgcg cgaagctcca tcaatgcttc 7320
   gcgcgaacgg cggcatgaga acgagattcg ccgtcgggag gaatacgacc gggatcatgg 7380
   catcccgact cagagccagg ccaccaggac tgagtcggca acagcctcaa ctggcggaac 7440
   cacccgggga cggccgagga accacaatca caactcccct ccccgggaca gacatcgtcc 7500
   ccgacaacag gaggacacgt gcggagtgtc cgctcttact ccacgcctta gggccatcca 7560
   atggcctccc aacttcaagg tatccaatgt cgacaaatat gagcctaagc aggatccagg 7620
   gggttggcta gccgtctaca ccaccgctgc tcgggctgtc ggagcatctg aggacgttat 7680
   gaccgcgtat ctgcccatcg tccttgggca agatgcgcta cagtggctac gacatttacc 7740
   ccgacactgc atcgacgact ggggagactt cagtcgacgt ttcaccgcca acttccagtc 7800
   cctctccgac aagccagcgc aaccgtggga cctcaaatcc atcaagcgtc ggggggatga 7860
   gactctccgg tcgtacctca aaaggttcca gaccatgaga aatcgcatcc ccgaggtcac 7920
   ggaggcggcc gtgattgagg acttctacag aggatccaac gactcggctt ttgtccgagc 7980
   catactacag aaagcgccga ctacttccga ggagctgttc cgggaagccg acctctacat 8040
   caccgccgac gagcgagctc aggacctcat cggaggagcg aagcctgcac cggcagcacc 8100
   acgacgcgac gcgaaccaac cacccgacaa acgctgggag aagaggcctc gcgaagaagt 8160
   acacgccgcc ggaccacctg cctctcgcgc ccgaggagga cctcgtggag gcgagcgcac 8220
   gctggacgac atcctcgacg cccagtgccc ataccacaag gacatgcgcc acactcttcg 8280
   caactgccgg gacttcaagc actccgttgg gcatggccga cccttccaac ctctacctcc 8340
   tcctccgccg aggggaggac cggatgaacc gcgacaacct catcagccgg aggagggagg 8400
   aggaggagct ttcccacgcg tcgacaggga ggtcaacgtc atcttcggcg gacatgggtc 8460
   gcaggagaac aaaagacaac aaaagctcaa cgaccgccag atactggtgg cgaccaccgg 8520
   tcctcccgcc ccataccggt ggtcagaaca cccgatcacc ttcactcggg aagatcaatg 8580
   gctcaacttc gaccatccag gcaaataccc gctcctcgtc gatccggtga tccgagagag 8640
   ccgggtgaag aaggtgctag tggacggggg gagcagcatc aacatcacct tcccccggac 8700
   gctccaaggc ttgggggctc acctcaaaga gctccacaag tcggacactc ctttctttgg 8760
   catcgtgccg acggagggag aatacccgct ggggcacatc tacatgccgg tcaccttcgg 8820
   aactccggag aactacagaa tcgagttcct gaggtttgaa gtggcgaact tcgactgcgg 8880
   atacaacgcc atcattggaa gaccgggatt ggccaaattc atggccattc cgcactatac 8940
   gtacatgata ctgaagatgc caggaccgca agggatcata actgtgcgcg ccgacttcca 9000
   aggcgccgca gaatgtttcc gagtggccat ccaggcagcc ctcaccacca agccgccaac 9060
   ggtcccctct acactggcga attctaagcc tgaggaggac ctcgccgtac cggcaaacga 9120
   agctcaggcc gcgacctcta tgcggccgac tgaagaaacc aagcgaatca acctggggtt 9180
   cgctgatgag cgcaagacgg ccatcatcag ctccagcttg aacgacaaat aggaaggcgc 9240
   gctcgtccag ttcctgcaag ataaccgaga tgtattcgca tggcaacctg cggatatgcc 9300
   gggagtccca agagaactgg ccgagcacaa gttgaaggtt tatccccagg cgaggccgat 9360
   ccggcaaaag ctacgtcgtt tcacgcccga caagagagag gccatttgcg ccgagttagc 9420
   tcgcttggtt gcggctggat ttattagaga agtattacac cccgagtggt tagctaaccc 9480
   tgttcttgta ctcaaaaaga ataaagtgga ttggcgcatg tgcgtcgact atactgatct 9540
   caacaaacac tgtcggaagg atcccttcgg gctcccgagg atagatcagg tggtggactc 9600
   taccgccgga tgttctgtgc tgtcattctg agattgctat tccggatatc atcagattag 9660
   tttggcaaaa gaagacgagg agaaaacggc gttcatcact ccgtttggtg ctttctgtta 9720
   cacctccatg tcgttcggcc tcaaaaacgc tggagcgact tatcagagag ccattcaaac 9780
   atgcttagcc aatcactggg gcaagcgtgt ggaggcttac gtagatgatg tggtgatcaa 9840
   aacagagaat tcggaaaact tcatcgaaga tttacagctg gttttcaaca gcctgagaag 9900
   atatagatgg aagctcaatc ctgaaaaatg tgttttcggg gtaccagcag gaaagttgct 9960
   cgggtttatc gtcagccact ggggaattga ggctaatcca gataagattg aagctatcat 10020
   gaagatggaa gctcctcggt cacaaaagaa ggttcagcga cttactggat gtatggcagc 10080
   tctgagcaga tttatatcca ggctgggaga aaaaggttta ccattttaca agctactcaa 10140
   gaaggtggat aagtttcaat ggacttcaga agcacaagaa gctctagacg cactgaagaa 10200
   attcttgaca acagcaccag tactgaaacc accccggcga gctacgccga ctcaaccggc 10260
   tgaagatttg ctgctgtata tctcttgcac gactcatgtg gtaagcaccg cgttggtagt 10320
   cgagcgagta gaagaaggac atgcctatcc agtacaacat cctgtttatt tcatcagtga 10380
   agttctaggc ccctcaaaga aaaagtatcc tcaggttcag aagctattat atgcagtact 10440
   tctaactgcc cgcaagctgc gtcactactt tgacgaccac aaagtcatag tagtcactgg 10500
   ttttccaata ggggacattc ttcacaacaa ggaagccatt ggccgaatag ccaagtgggc 10560
   ttgcgagctg ggatcccacg acatcgagtt ccgacctcgc actgccatca aaactcaagc 10620
   actggttgat ttcgtatcag agtggacaga acagcaagta ccagataatc cagaaactgc 10680
   agaagtatgg cgaatgtatt ttgacggctc gctgaagctg cagggagcag gggcagggat 10740
   tctcttcact gcacctggag gcgaacacct caagtacgcc ctctagttgc tattcccggc 10800
   ctctaacaat gcagccgagt atgaagcttt gatacacgga ttgaacatcg ccatatcact 10860
   gggcgtcaag agattgatgg tatacggaga ttctctggta gtcatcagcc agataaacaa 10920
   ggaatgagat tgttcaagtg attcaatggg aaaatactgc gctgccgtcc gaaagctgga 10980
   agataaattc gagggtctgg aatttcatca tatagaaaga gatcgaaaca cagcagccga 11040
   cgtactgtcc aagctcggat ccggtcgaac tcaggtccca accggagtct tcgtgcaaga 11100
   aattctgcag ccgagtatct caatggatca aacagaagag tgcaacgtca taaatcaacc 11160
   tgagtcagac tctgatgact ggagaaggct gattattaag tacataaaga atgacgaaga 11220
   accagacgac aagaactcgg cagagcgcat tgccaggcag tcggctcgct atacactcat 11280
   tggggaggag ttgtacagaa ggggcgcatc aggcatcctc atgaagtgcg ttctcccgtc 11340
   cactgggaaa caacttctgg aggaggtcca cgcagggcaa tgtgggatac atgcagcatc 11400
   cagaacacta gtcgggaagg ttttcaggtc aggattctat tggccgacgg cgaagaacga 11460
   tgcagccgag ttagttcaga gatgcgaagc ttgccaatac ttgtcaaagc aacagcacct 11520
   gccagcacag caactacata ccataccagt aacctggcct ttcgcatgct ggggactgga 11580
   tatgattgga cctttcaaga aagctcaagg gggatatact catgtattgg tggcgattga 11640
   caaattcact aaatggatag aattcaagcc cattgcttct ttaacctctg ctaaagccgt 11700
   ggaattcata caagacataa tattcagatt cgggataccc aacagtatca tcactgactt 11760
   aggatccaac tttaccagtt cagaattctt cgacttctgc gagcaaaaga gcattcagat 11820
   caagtatgca tctgtagcac atccaagagc caacgggcag gttgagcgag ccaacggaat 11880
   gatattggag gcactcagga aaagggtctt cgacaagaat gaaaaattcg caggaaagtg 11940
   gataagagaa ttaccttatg ttgtttggag cctaagaacc caacctagcc gagccctgca 12000
   tggaaacact cctttcttca tggtctatgg gtcggaggca gtgctacctg ctgatctcaa 12060
   gtttggggcg ccaaggttga tcttcgaaag catagcggaa gctgaggcca ccaggctgga 12120
   ggacgttgat gtacttgagg aagaacggct gaacgcggta atccaatcag cacggtacca 12180
   gcagactcta aggcgctatc acgacaaggc tatgcggcaa cggttctttt cagtaggaga 12240
   cctcgtcctc cgccgaattc taacgggaga gggacggcac aagttatcac ccttatggga 12300
   aggacccttc ataatagcag aagtcactcg gcccggatcg tatcgcctca ctcagatgga 12360
   tggcacagaa gttgggaact cctggaacat agagcacctc agaaaatttt atccctagct 12420
   gtatttcaaa actgctgggg cgacaatgta ttctgtaaag tggaaatatg tcgtcaataa 12480
   agagagattt caaagatact cggctcgttt acaattgact tgcattctca cttaactcgg 12540
   ggtgaccact atgccctaca aacggagcaa tcggcttaag tcggcaacga cttaaggcga 12600
   tgcaacatgc tcacgcttac ttaacccggg atgaccacta tgccctacaa acggagctat 12660
   cggcttaagt cggcaacgac ttaaggcggt gcaacatgct cacgcttact taacttaggg 12720
   tgaccactat gccctacaaa cggagcaatt ggcttaagtc ggcaacgact taaggcggtg 12780
   caacatgctc acgcttactt aacttagggt gaccactatg ccctacaaac agagcaatcg 12840
   gcttaagtcg gcaacgactt aaggcggtgc aacatgctca cgcttactta actcggggtg 12900
   accactatgc cctacaaacg gagcaatcgg cttaagtcgg caacgactta aggcggtgca 12960
   acatgctcac gcttacttaa cccgggatga ccactatgcc ctactaacgg agctatcggc 13020
   taaagtcagt ggtggcttaa gccggcgcaa catactcgcg cccatataat tcaggggacg 13080
   acttccatat cccgccaaca aatttcatga tcatcatgcg tcattaccac aaatttacga 13140
   accaataaat tctgatacga taagagagta attatgtcat tcgaatgata aactgatgta 13200
   ttttaacaaa tactcgatca tgctaaccgc gcgctcagag cacgcaaaat gtttctctat 13260
   ttcgcaatgt ctttttcagg aacgaccgac gaagtaggac gattcgagga atcgggggca 13320
   gcattcacgt cagcctttat gtcttcagga acaaccacgc cgggtctcct cattaagatt 13380
   cgtccagcac gaatagcctt gtccatggct ttatcgcgct gggctttgag agtagtcgca 13440
   gtttcttcgg caactttagc agccagccga gcagcctcta actcctgggc aatggatttc 13500
   ttggaagctc ggagttcttt gatggtggca tccttcgctg atatcacttg cctaaggcgg 13560
   gtcacttcgt ccgcagattc ttgcagccta caacgctgat tgtccagttc ctccattgta 13620
   aagcggtgac tcctctccaa gatggtcagc gagttgttag aatcacgata caatctgtct 13680
   agattgtcac gagaagcagc aaggatacgt ttttcttcct acaaacaaaa atcaactcct 13740
   tcagaaatca agcaagtaat aacagcacac ggttttgtaa gttacctttt cagagtcaag 13800
   ccgagcatga agagaagaac tcagggcatt ggcatcatcc aaagcagcgg agacctgatt 13860
   taattcagtt tgactctgag agtacttctc ctcgaagtca gcgcaccgtt gaaccatttc 13920
   agcctgctct cgggagtgtt tttcctcaag aactgcaatc tgctgagtca ttcctatcaa 13980
   gaggtaatca aacaaaatga ggtcagaagg taaaacagtc cacgaacaaa ggcgaagaag 14040
   aataagaaaa aagggcacta accagcgtta gttgcctcca actcggatac acgacgatga 14100
   agcgacactg gattccaaca cgcaagagac gaagccactt ctggggcgga agcaccctgc 14160
   gatctcagct ggctggccat cccatctacc aaagcctgat aagaagaaca gatgaacata 14220
   atgacaaaag ttcacgcaac ataaagaaca agctaaaata cagcacagta cctggaggtt 14280
   ggaaaagaac gaagggatcc ccaaatcagg agaaatcagc tgataaccag gcgcaaggcc 14340
   accacccgaa gcagcttgca acgaaccagt aggaatcagt tcagtgcctt caacagagcc 14400
   aaacgcccga tcagcgggga cgctgccctc taaaatgcct ctctggtcca aagtttgggc 14460
   taccaccatg cagccagagt gtggaggtga acccacgtga acgtccatag aagtgcagga 14520
   aggagaccct gctcgggcac cctcgggggc tgggtcatag ttggcactgc ccacttgagc 14580
   cggatcatcc ccggcagaac cctcgggggc tgggcagtgg cttacattct tcacccgagc 14640
   taagtcatcc ccagcgacac cctcgggggc tgggcatgtg tcagcaccat tcttgagatc 14700
   caggctctcc gcagtaacca cctctaaggt tgacgggcct tcagccactt ccatggaacc 14760
   aggacaatcc aagtcagcat cccgaccctc gagatcgcgc tctaaggtcg acgaggcacg 14820
   ggatgacctc aacccagcat caggcacggc agcgcaagcc tccgttgcat catcatccac 14880
   tggctctgat aacaagtcct ctgggaccat atcctcaaga gtttgatcaa agttggccag 14940
   ggacagctct tgcagaccaa tgagggcaga caaagcagga gaaggaactc cactacttcc 15000
   accgctggcg atgaactgcc gactgttttt ccgagttaaa ggagcttcat tttcttcctc 15060
   ctcatcttcc acagtgacaa cacaagcagc acccccattg ggatcagcaa cagatggagc 15120
   acccacattg ggatcagcag cagtttgggt acacccattg gggtcagcgt cagtaaggcc 15180
   agttgcaggc acttcttcaa cagcaggaac taaggtaccg gcgtcctcat caaaactgga 15240
   tactcgccgg aggcgtcttc tcttcttctt ttgctcatca gcagaaggct caggctgact 15300
   ggttcggcta gggcgcctcg ggcgagtatt gactggtttc tggacatcca aagttgtatc 15360
   aacctctgga aggggcacca ctgccaacgt accagcagga gcagcgtcgg aggaatcctc 15420
   agctagcaga tcaagcatgg catttatgtc cgcatcacta gggttcaggg gcacctcaaa 15480
   atggacctgc cgttcgtcat caggaatctc cccaagcgaa gcaaccaagg taccgacttc 15540
   ttcagcagag gatcgcactc taaggcccaa attgccatca gtcacaggcg gatttgacac 15600
   aaaaagggtg aaagctttgg gaggaggcag gttccaggcc gagtatgcca ctggagcacc 15660
   aacattcgaa accttacccc tgaggatcat ttcaagtcgg cctaccaagt ccacaggagg 15720
   aattctccta ttggtaaccc gagttgagtc ggctaaccct cgatacagat atgctggata 15780
   ggccctgtct ttcagtggct gaatgttctt aaaaacaaag tcagtgacca cagcttcagc 15840
   agttagaccc ctctctttca gcagtccaac ttcagttagc aacacacctg cctcagccac 15900
   ttcctgatct gtgggagact cagtccagct cggagtgcga acgtctggct gtcttcccga 15960
   ccgggggggg ggggaatttc cataattatc aactatgaac cactctagac gccatccctt 16020
   aatactgtcc ttgaggggaa tctcaagata ctcagttttc cgcccgcggc gcatctccaa 16080
   actggcacct ccgaccaact gatgctgtcc cccggccatc ccagggcgac aatgatacaa 16140
   atacttccat aagccaaaat gcggcagcac gccaagaaag gcttcgcata agtggacgaa 16200
   aatggagatt tgcagaatgg aattaggatt caaatgggtc aagttaatgt ggtagaaatc 16260
   aaggaggtcg cggaaaaagg gagagatggg aaggccgagg ccgcggagaa gaaaaggagc 16320
   gtaaactaca gactcgtggg tatcctctgt cgggacagta gtcccgtggc aaatccgcca 16380
   agaacagagt tctcgcggag gaagaacctc gatggatacg aggtggagaa gttcagcttc 16440
   ggaaatgatg gacatatggt tacctgcgaa gggcaactgg ctgttggggt cgattggagg 16500
   gatcaccgca gcagacgagc tcgcagtttt cctcttgggc gccatcttga ttttcaccag 16560
   cgaacagaat agggggcgaa tggcagagag gattcagaag cgggaatgca agaactaggg 16620
   cacggaaagc aaaggcggct aaaagcgcag ctcttatggg atattctcga gccagatacc 16680
   gtttcaaaag tgcccagtca tcacccgacg gttatttccg aaaccccagc atgccacgtg 16740
   gtcatctggc agttatttcc aaaaagccga cgtgtcactt catcactcgg ttatcatcct 16800
   caaaataact catgcggccg gctatcactc ggcgttgact ctaaaacgct gatttcatat 16860
   tcagtcgctc ggcattacct ctaaaatgcc gacgtcacca tccagacact cggtgttgac 16920
   tctaaaacgc tgatttttta ttcagttgct cggcattacc actaaaaagc cgacgccgac 16980
   ctccagtcac tcggcgttgc ctctaaaacg ctgatctcgt attcaatcgc tcggcattac 17040
   ttctaaaatg ccgacgttaa ccttcaatca ctcggcgttg actctaaaac gctgatttta 17100
   tattcagttg ctcggcatta ccactaaaaa gccgacaccg acctccagtc actcggcgtt 17160
   gcctctaaaa cgctgatctc gtattcaatc gctcggcatt acttctaaaa tgccgacgtt 17220
   aaccttcaat cactcggcat tgcctctaaa acgctgatct cgcattcgat cgctcggcat 17280
   tacttctaaa atgccgacgt cgaccttcaa tcactcggcg ttgcctctaa agcgatgatc 17340
   tcgtattcga ttgctcggta ttacttctaa aatgccgacg tcgaccttca atcgctcggc 17400
   gttgcttcta aaacgccgat accgactaca agattactcg gcagctactt ctggaagcgt 17460
   cagcgtgata cccaagttat tcatctactg tctcaaaaga atcagtttta taatcaagca 17520
   aagcgagaca tcaagaagga gccaacgtca ttctttcatt aagggaagac aataagttta 17580
   caaatcaact ctttgcgagt tgatgcttcc ctactattac tacattacat tactactact 17640
   actatactat actatactac tctacattac tactacatta ttctaactac actatactaa 17700
   tatctaaaga ccagcgacgc ttagccactg gccttgctgc tgctgccacc gccgccgccc 17760
   ctggtgctgc ccttgctgct gccgcctctg gtgctgcccg tgctgctgcc gcctctggtg 17820
   ctgcccttgc tcccgtcacc gctgccgctg cccccgccgc tgccgctgcc gtgaccatcg 17880
   ccgtcgccac cgtcgtcgtc gtcgtcttcg tcgtcgtcgt catcctcgcc ctcgccgtcg 17940
   tccgagtcct cctcctccga ggagaactcc gactcatccg agccctcgag cccggagcgc 18000
   tcgacggccc ggatgtacgt ccagacttcc gcggcaatcc cctgggagtc gtctgaatca 18060
   tcagacgacg ccgggggaga gacgggagcc agagacccct cggactcgga ggagaactcc 18120
   tcctctgagt attccgagtc gccgaagtcc tccgacggag gagtcggctc gcgcttgcgt 18180
   ttgttactct tgcccatggc ggaagcagac aggagagaag agaaggatgc agtaaagaac 18240
   agcgaagaga tgtgaaaaaa ccagaggagc aacggcgtta tttatagaag ggaaaggcaa 18300
   ccgctcacct ccaaccgtgg tcactaaaca gtcgcaaagc attcaataag cacttccacc 18360
   cgtctgaaga cgcgtcagac ggctgacgcc gtttcatgca acactacacc cattgggact 18420
   ccagtcaaca gcgcaaagga tatgattaca ctcgccgtcc catcacatta ctactcagaa 18480
   gcagccggct aaaacactca gcgtaccaag ccgtcccttg cccacaccca ttggggggac 18540
   gcccaggaat tatcagtata tttttcaaaa cggtcacatc cgtgcagggc acgcagtgaa 18600
   tacctcaaga caaaaatgag atatcagtaa ggatcagagg aaagccaaat atagccagca 18660
   atgtacaaga tatggccgac agaagcgacg tgaagactag acagagaacg tccgtcaaac 18720
   gtccaatcag tcgcagaagc aaataaattg cactacctag caagatatgg atggattgca 18780
   aactcggctg ctaaagacaa aatatacgct gacctctgca gcaaaatatt gatgacataa 18840
   tgctgacctc caaagcataa tgcaaatagc ttcatgccaa tttttacaaa cgaaagaaag 18900
   accttcgaat ggattatcct caaaaatcca tttgaaggtc gggggctaca cccattgggt 18960
   gcacctccgg tgcaccccct ggattatcat tccaaaccga gatagtgccg acttctaagg 19020
   cacgggacaa gattaaaata ccaatcctcg accaagatgc aggagcacaa atggagataa 19080
   tttctgggga agaccttcga gtagattatt ttctaaaaat ctactcaaag gtcgggggct 19140
   acacccattg ggtgcacctc cggtgcaccc aatgaagttc agaatcctgc aaatcgaaat 19200
   gtcgacctct aaggcacaag cacgaaacta agcttcggtc aaatgagcga tcggagcaag 19260
   agaagacagc aggaagtcat tttttggacc tgggatcttt gggttgatta cctctaaatt 19320
   aacccaaaga tcgggggctt gtggtggata gatatccccc gggtccacta agggagtaaa 19380
   agacctcacg aaaggcccaa gggcccaata aatggtaagg tcattcttcc gtgggcctgg 19440
   ggagaaacaa ccaacaaaac agagaagacg taagaccgga ttggtgcaaa cccggacgac 19500
   ccacaacgtc gaacgagtaa atcgcaacag agacccgact ttcccgcgct ggagccccca 19560
   tgcaacggag ccatgcaagg ataagtcggc gagggttacg tagggataaa ctcaagaggt 19620
   tcactacctt ttagctactt gttgttatca tatccacgtg tactgcccca cagtcgagta 19680
   tataaggcct agggggcacc ccttcagaac gatcgaccct atcttactta gccccccaca 19740
   gcaactctct gtgtcttcaa tccagagagc cctcttgtaa ccacgctcac atactcaccg 19800
   ggacgtaggg tgttacgcat ctctaagcgg cccgaacctg taaatcttgt ccactgtccc 19860
   tcgtgtgatc ggcacgaacc gttttgctac agtcgtcgac actgtcctac tcctagaaac 19920
   accttgaggg gcaaccccgg gtgtgcggtc ggacccaaaa caccgacact agcacacgtg 19980
   gaccaattga aagcccccac cttgctctcg ttcgtcgctc gttcacgcca gcgcgtcacc 20040
   cacccactgc gccctctcat cctccttgcc cgagaacgtg cttcagagca ggcgagagtg 20100
   gggcgatatc gcccgaggga tgcatctctc cctcctctca ccccgggcca ggctacaaca 20160
   gccccctact gcccaaccac tcgccctcgt tggcaccaac catactacca caacactcca 20220
   atgcacaatt tttaatgtta gcctatattc accttagcat cacatcatat ttcttctaca 20280
   aaattatatt tagaacgaaa cacgacgttt tgcttggaat tttttcgatg gagtagaaaa 20340
   aatataatcc aaacaaatta cgtaaaatac gtaaaataaa aaatatagct cctaaaagtt 20400
   aaaattactc atcattatca ccctcctcgt tttcttcctc gtctagtttt aattgcgtga 20460
   tcaatttttt tagaaccaac ctagaccctc ctcataccag tggtggattg tttcagtaac 20520
   ttggtgaact ggtcgtacaa ttttttgatt ggtttctcgt gtcactaacc ttgaataatt 20580
   gcttcgttat ggttttcttc tggacaaggg actcattggt actcattcct ggtgcctaac 20640
   ccttgttttg tgctcttgcc caatcacgac caatagggta tggtggtttg gcactatcgg 20700
   acgatgtaga gttataatca ccctccacgt tgaggcaaga tcattttctc tcattgctag 20760
   agtttgaatc accatgtcac gtgcaccact tgaattgatg atgaagagca cgccattagt 20820
   ttcatcgagc ctgttgtgga agagtagcat aacattattt gatctgctcg gtaggttata 20880
   cgtggccgat atttggcacc aaaaggtcaa acatttttgt tgtttccctc aaatcttttt 20940
   tggtctcaaa aaggtcaaac ctttttttgg ctttccatcc ctgtccccac gaacagtcac 21000
   gagtaacttt ttctcccatc cccgttcgtg tgggggaatt tatccccatg ggaaatctat 21060
   ccctgttaga gaatgtaata tttttggggc aaggtcaaat tgataaacca atataaattg 21120
   aacaaataag atttaaaatt acaagagaaa tctactttag agttgagctt gctattttat 21180
   aaaaggctat gtgttgaatt tttattataa ctttaacata tcaatggatt gattttagat 21240
   taagagaagt tcgtgggggc ggatatgcga ggaacaggga tggggaatgg ttcaccattc 21300
   tcgtcccacg aacacgacaa ggactaaatt tttctcgttt caatctccgt ggggactaaa 21360
   ttacacctat ccccgtccac taataaaggg ataccccgcg gggaattagg gatcacgtcc 21420
   ccatttcaca tttttagact tctcacattg agcaacaaca aaacatttta tgaattagta 21480
   gccattttac ttatctaaaa caattactat tgaatcttaa tcattttgtg caaaataaag 21540
   aatgaactaa attttgggtc gggtgtgggt cacccgtagg ttgaaataaa aactcgcacc 21600
   cacactcgtg aaactttggg tcagatgagg gttacacccg cggattaaaa tctctaccca 21660
   tacccacacc cgtcagatcg agtacccaaa gattttgggt ttgcgggtta aattgtcatc 21720
   cctagacacg agtgtgctgg aggatttgga agtttcgcaa tgcgtactta ccaagtgttt 21780
   ggttctatgg attaaagttt agcatgtgtc gcattagatg attgaatgcc tattatgagt 21840
   attaaatatt gtctgattat taatcaaatt acacaagtga aggctaaaca actagacaaa 21900
   tttattaagc ctaattaatc tatgattagc aaatgtttat agtagcagca tcttagtgaa 21960
   tcatgaacta attaggttta ataggttcgt cttaacgttt agttcttatc tatgtaatta 22020
   attttataag taaactatat ttaatattcc taattagtct ccggatataa catagactaa 22080
   aatttagtca gtggtgctgc tgctggctcc ttttcatttc atccgacata atgttttcct 22140
   cccaatttat tgtgttgttt tgtttgtgct ttaaaaatga cttgtcctct gcttgctgta 22200
   tcaaggtcgt gatgtgtgct taacttattc ccttccttcc tagctgccct tagcatttat 22260
   ataggggtat ttggttacat ggactaaagt ttagtttagt ccatgtccaa ttataaaact 22320
   aattacataa ctgaatacta aaataccaaa atacgagaaa aattattaaa cctaattaat 22380
   ttattattag caatgtttat tgtaacatca cataagcaaa gcatagactg atttagttta 22440
   ataggttcgt cttatcattt agtcttgatc tgtataatta gggtatgttt ggttcactgc 22500
   ctaacttacc acactttgcc taacttttct gcctaaggtt agttcttcaa tttgaacgac 22560
   taaccttagg caaagtatgg cgtagttagc cacgaaccaa acagtccctt agttttgtaa 22620
   ttaaactata tttactattt ttaactagta tccaaacatt aaatgtgaca tggattaaaa 22680
   tttagttagt gactttaggc ttttagagtt gagttagttg tagcttggtt tagttatttg 22740
   tttgttctct tgcgtttatt taggacctct ctataatgtt ttatcacctt aatacaaatt 22800
   aaaatacgca gctcttgtgt attgcaggcg tgtgtgttcc tatattttta ggctcaatta 22860
   tgaaggaaat tattctcagc gagcatataa ccgttttgga ggtaaaatga actaaaagca 22920
   tataggcctg cctgtttgca taagtacacc ctccgtctaa aaaagaataa aaatctcatt 22980
   tcttgatgag tcaaaaaagt tcaaatttaa gaaaatatat gttacgacac gaatatttat 23040
   aatgtgtaat aagtaccgct agattaattt taaaataaaa ttttcataat aaacctattt 23100
   gaagatacaa gtattggtac tatttctaat aaatctaatc aaactggtgt tatatctttt 23160
   ggaacaaatt tgtgctttat gtttctggtt gacgtgaatc agcttaatct tgacgaaatc 23220
   taacattgtc ttttgttcgt tggcatacag gatcaacaaa aaggagagaa gcagcatcta 23280
   ttgttggtaa gagcctatag tcaataccca tgttcatttc gtctaaaaga gcagaagaaa 23340
   agcatatgat gaattattgc catgtcatgt ttaaaataca gaattctcaa aaacaaaaac 23400
   aaaaaaaaaa acttggaatc cactaaccac tgatagcatt gtagaaaatt tcatcctccc 23460
   tttgggcaat acactgatga gtttacatgc tgactagtgg tgcatttgtt ctttgccaat 23520
   tgagttttta gaatgctttg cagctgaatt cacttgtgat gttttttttt gtgtgtgatg 23580
   caggtgctgt tgccgttgca ttcctgtgtc tagtcattct cacatgcttc ttggcttgta 23640
   gacattgttc gctgcccttc aaatcgaaga acaaaccagg gacaaggatt gagtccttcc 23700
   tacagaagaa cgagagtagt atacatccga aaagatacac ctacgcggac gtgaaaagaa 23760
   tgacaaaatc cttcgctgtg aagctaggcc aaggtgggtt tggtgctgta tacaaaggca 23820
   gcctccacga tggccgacag gtagcagtca agatgctgaa ggacacccaa ggtgacggcg 23880
   aggaattcat gaacgaggtg gctagcatca gcaggacttc tcatgtcaac gtcgtgacac 23940
   ttctagggtt ttgcttgcaa gggtcgaaaa gagcactgat ctacgagtac atgcccaatg 24000
   gttcgctcga aaggtatgcc ttcaccggtg acatgaacag tgagaatttg ctaacctggg 24060
   aaagactatt tgacatagcg attggcacgg ccagagggct cgaataccta caccggggat 24120
   gcaacactcg gatcgtgcat tttgacatca agccacacaa catcctgtta gaccaggatt 24180
   tctgccctaa gatctctgac tttggactgg ccaagctatg tctgaacaaa gagagcgcta 24240
   tctccattgc tggcgcaaga gggacgatag ggtatatcgc cccggaggtc tactcaaagc 24300
   aatttggaac gatcagcagc aagtctgatg tctatagcta tgggatgatg gtccttgaga 24360
   tggttggagc aagggacagg aatacaagcg cagatagtga ccatagcagc caatatttcc 24420
   ctcagtggct ttatgaacat ttggacgact attgtgttgg tgcttccgag attaatggtg 24480
   agaccacaga gctcgtgagg aagatgatag ttgtaggtct gtggtgcata caagtgattc 24540
   cgactgatcg accaacaatg acgagagtcg tcgagatgtt ggaaggaagc acaagtaatc 24600
   tagagttgcc acccagagtt ctcttgagct ga 24632

<210> 2
<211> 2004
<212> DNA
<213> Artificial Sequence

<220>
<223> cDNA derived from Ht2 gene (SEQ ID NO: 1)

<400> 2
atggctgctc acctaccacg cctccccgtc ctcctcctcg tcctcctcgc tgcccatgtc 60

gtctccacct ccgcccatgc cgagcctcct cttccgagcc cttacagcac ctccgcccat 120

ggcgagcctc ctcttccgag cacttacaac gtctccatgt gctcggaatc gttctggtgc 180

ggcggcgtcg aaatccgcta cccgttctat cttgccaacg caaccgccga ctacagcggg 240

agctactact cctgcggcta caccgacttg agcgtttcct gcaaactcga ggtcgagggg 300

ccgacgacga catggacccc taccatccgt ctcggcggcg acaactacac cgtcaagaac 360

atcttgtacg actatcatac catctcactg gcggacagcg atgtgctcgg aggcggcgag 420

tgccccgtcg tccaccacaa cgtcagcttc gacgagacgt ggctgcacaa ccccagcgcc 480

ttcgacaacc tcaccttctt cttcggatgc cactgggggc cacgcgatac actgcctgaa 540

tttgccggca acaacatcag ctgcgccggg ttcagtactc cagctatcag cggtggaggc 600

tccttcgtgt tcaagcctga agatcttgac gaacatgcgg agcaggagtt ggcttcacac 660

tgcgacgagg ttttctccgt gccagtgaga agcgaggctc tgcagcaggc gatcgtcagc 720

aacctcagcc tcggggacgg gtacggcgag ctgcttaggc aggggatcga gttggaatgg 780

aaacggacat cggaggatca gtgtggccag tgcgaggaat cgggctccgg cggacggtgc 840

gcctacagcc agaagagaga attccttggc tgcttctgca gcggagggaa ggcgggcaac 900

ccgttctgca aaccatcaag atcaacaaaa aggagagaag cagcatctat tgttggtgct 960

gttgccgttg cattcctgtg tctagtcatt ctcacatgct tcttggcttg tagacattgt 1020

tcgctgccct tcaaatcgaa gaacaaacca gggacaagga ttgagtcctt cctacagaag 1080

aacgagagta gtatacatcc gaaaagatac acctacgcgg acgtgaaaag aatgacaaaa 1140

tccttcgctg tgaagctagg ccaaggtggg tttggtgctg tatacaaagg cagcctccac 1200

gatggccgac aggtagcagt caagatgctg aaggacaccc aaggtgacgg cgaggaattc 1260

atgaacgagg tggctagcat cagcaggact tctcatgtca acgtcgtgac acttctaggg 1320

ttttgcttgc aagggtcgaa aagagcactg atctacgagt acatgcccaa tggttcgctc 1380

gaaaggtatg ccttcaccgg tgacatgaac agtgagaatt tgctaacctg ggaaagacta 1440

tttgacatag cgattggcac ggccagaggg ctcgaatacc tacaccgggg atgcaacact 1500

cggatcgtgc attttgacat caagccacac aacatcctgt tagaccagga tttctgccct 1560

aagatctctg actttggact ggccaagcta tgtctgaaca aagagagcgc tatctccatt 1620

gctggcgcaa gagggacgat agggtatatc gccccggagg tctactcaaa gcaatttgga 1680

acgatcagca gcaagtctga tgtctatagc tatgggatga tggtccttga gatggttgga 1740

gcaagggaca ggaatacaag cgcagatagt gaccatagca gccaatattt ccctcagtgg 1800

ctttatgaac atttggacga ctattgtgtt ggtgcttccg agattaatgg tgagaccaca 1860

gagctcgtga ggaagatgat agttgtaggt ctgtggtgca tacaagtgat tccgactgat 1920

cgaccaacaa tgacgagagt cgtcgagatg ttggaaggaa gcacaagtaa tctagagttg 1980

ccacccagag ttctcttgag ctga 2004

<210> 3
<211> 667
<212> PRT
<213> Zea mays

<400> 3

Met Ala Ala His Leu Pro Arg Leu Pro Val Leu Leu Leu Val Leu Leu
1               5                   10                  15

Ala Ala His Val Val Ser Thr Ser Ala His Ala Glu Pro Pro Leu Pro
            20                  25                  30

Ser Pro Tyr Ser Thr Ser Ala His Gly Glu Pro Pro Leu Pro Ser Thr
        35                  40                  45

Tyr Asn Val Ser Met Cys Ser Glu Ser Phe Trp Cys Gly Gly Val Glu
    50                  55                  60

Ile Arg Tyr Pro Phe Tyr Leu Ala Asn Ala Thr Ala Asp Tyr Ser Gly
65                  70                  75                  80

Ser Tyr Tyr Ser Cys Gly Tyr Thr Asp Leu Ser Val Ser Cys Lys Leu
                85                  90                  95

Glu Val Glu Gly Pro Thr Thr Thr Trp Thr Pro Thr Ile Arg Leu Gly
            100                 105                 110

Gly Asp Asn Tyr Thr Val Lys Asn Ile Leu Tyr Asp Tyr His Thr Ile
        115                 120                 125

Ser Leu Ala Asp Ser Asp Val Leu Gly Gly Gly Glu Cys Pro Val Val
    130                 135                 140

His His Asn Val Ser Phe Asp Glu Thr Trp Leu His Asn Pro Ser Ala
145                 150                 155                 160

Phe Asp Asn Leu Thr Phe Phe Phe Gly Cys His Trp Gly Pro Arg Asp
                165                 170                 175

Thr Leu Pro Glu Phe Ala Gly Asn Asn Ile Ser Cys Ala Gly Phe Ser
            180                 185                 190

Thr Pro Ala Ile Ser Gly Gly Gly Ser Phe Val Phe Lys Pro Glu Asp
        195                 200                 205

Leu Asp Glu His Ala Glu Gln Glu Leu Ala Ser His Cys Asp Glu Val
    210                 215                 220

Phe Ser Val Pro Val Arg Ser Glu Ala Leu Gln Gln Ala Ile Val Ser
225                 230                 235                 240

Asn Leu Ser Leu Gly Asp Gly Tyr Gly Glu Leu Leu Arg Gln Gly Ile
                245                 250                 255

Glu Leu Glu Trp Lys Arg Thr Ser Glu Asp Gln Cys Gly Gln Cys Glu
            260                 265                 270

Glu Ser Gly Ser Gly Gly Arg Cys Ala Tyr Ser Gln Lys Arg Glu Phe
        275                 280                 285

Leu Gly Cys Phe Cys Ser Gly Gly Lys Ala Gly Asn Pro Phe Cys Lys
    290                 295                 300

Pro Ser Arg Ser Thr Lys Arg Arg Glu Ala Ala Ser Ile Val Gly Ala
305                 310                 315                 320

Val Ala Val Ala Phe Leu Cys Leu Val Ile Leu Thr Cys Phe Leu Ala

                325                 330                 335

Cys Arg His Cys Ser Leu Pro Phe Lys Ser Lys Asn Lys Pro Gly Thr
            340                 345                 350

Arg Ile Glu Ser Phe Leu Gln Lys Asn Glu Ser Ser Ile His Pro Lys
        355                 360                 365

Arg Tyr Thr Tyr Ala Asp Val Lys Arg Met Thr Lys Ser Phe Ala Val
    370                 375                 380

Lys Leu Gly Gln Gly Gly Phe Gly Ala Val Tyr Lys Gly Ser Leu His
385                 390                 395                 400

Asp Gly Arg Gln Val Ala Val Lys Met Leu Lys Asp Thr Gln Gly Asp
                405                 410                 415

Gly Glu Glu Phe Met Asn Glu Val Ala Ser Ile Ser Arg Thr Ser His
            420                 425                 430

Val Asn Val Val Thr Leu Leu Gly Phe Cys Leu Gln Gly Ser Lys Arg
        435                 440                 445

Ala Leu Ile Tyr Glu Tyr Met Pro Asn Gly Ser Leu Glu Arg Tyr Ala
    450                 455                 460

Phe Thr Gly Asp Met Asn Ser Glu Asn Leu Leu Thr Trp Glu Arg Leu
465                 470                 475                 480

Phe Asp Ile Ala Ile Gly Thr Ala Arg Gly Leu Glu Tyr Leu His Arg
                485                 490                 495

Gly Cys Asn Thr Arg Ile Val His Phe Asp Ile Lys Pro His Asn Ile
            500                 505                 510

Leu Leu Asp Gln Asp Phe Cys Pro Lys Ile Ser Asp Phe Gly Leu Ala
        515                 520                 525

Lys Leu Cys Leu Asn Lys Glu Ser Ala Ile Ser Ile Ala Gly Ala Arg
    530                 535                 540

Gly Thr Ile Gly Tyr Ile Ala Pro Glu Val Tyr Ser Lys Gln Phe Gly
545                 550                 555                 560

Thr Ile Ser Ser Lys Ser Asp Val Tyr Ser Tyr Gly Met Met Val Leu
                565                 570                 575

Glu Met Val Gly Ala Arg Asp Arg Asn Thr Ser Ala Asp Ser Asp His
            580                 585                 590

Ser Ser Gln Tyr Phe Pro Gln Trp Leu Tyr Glu His Leu Asp Asp Tyr
        595                 600                 605

Cys Val Gly Ala Ser Glu Ile Asn Gly Glu Thr Thr Glu Leu Val Arg
    610                 615                 620

Lys Met Ile Val Val Gly Leu Trp Cys Ile Gln Val Ile Pro Thr Asp
625                 630                 635                 640

Arg Pro Thr Met Thr Arg Val Val Glu Met Leu Glu Gly Ser Thr Ser
                645                 650                 655

Asn Leu Glu Leu Pro Pro Arg Val Leu Leu Ser
            660                 665

<210>  4
<211>  1487
<212>  DNA
<213>  Zea mays

<400>  4
aagtgaacct caccatcgac atcgtctggc tacgactcgc accctccggc tccaacatca      60

cattcctcta caattgcaag aagaacatta gagcatctcc agcaggtcgt gtaaatagtc     120

gtgcaaaata caatttgtac tatagattac actatttgca gtgtgaagtt taagatagag     180

agtaggataa agagtctgct ggacatatta ccctgtcctc tgtcgtggaa ctgagtgggt     240

gcccacagca acaagaagat agaagtaggt cgtacgtgtt gtcgggtggc gggatcatgt     300

gtgctgagac gtacgtatac atgtccaaat aggcaggtcg ggccgactcg gcccgagccc     360

gactaagctc ggcacggatt atgatcgtgc tggcctgacc cagattagca agcaggtcgt     420

gtcgtgcctg cccacgggta tcgagcgagg cccagacacg acacgctaag gtattaaccg     480

tgccgaaccg acccacggcc cgacgggcca aacaggtcta taatagtata tgacgtttaa     540

atgtaaaaaa tatattaaaa tatacataac gaaggtttaa accatattta ttagctctaa     600

acacctattt acacccacat caccaacaaa acaaatatgt ttactgtatt ttatactata     660

tattaagtat aatgtatgta tttgtattaa aaaaatagga acaaaattaa aaatgggtcg     720

tggcgtgttt agcctctcgt ggcgcgcgta gacccagaca caacctagtc accagaccgg     780

gccaaccagt acctgagttt cggaccgccc gaccggccgg tacaagcacg ggtgcgaggg     840

cgtggtggtg gcgccgccgg tgctggatgt acacaagaag gggggaagag aagcccgggc     900

accaccggcg gtccgcctcc ggaacggatc gctccgtcgg ttgctgcagg gcgggttcga     960

gctgaactac gacactcact ccgagcagtg cgtccgatgc tagggctccg gcggatggtg    1020

tggctaccag cgcgaggaga cgcccgccgg cgggatgaca ttcgcctgtt tctgcgacgg 1080

cggccagacc acgggccgat gcggtgccgg tatgtctttt ttttttcttc gaacaaggtg 1140

tggcatgtgt tctaccgttt caactagtaa atgattacat tgagctaggc agctagccac 1200

acattttctt gaatgatttt ctttgatgaa cctgctgttt gcttttatga cgtgaacaac 1260

ggggcctgca agctgccaca tacctaggga gactagttcg tgaccttctt tacacgtctt 1320

ctctactcgc cactgggagt tgacgccgct cggtcgtccc actttgtgac gttcaaccag 1380

agtctagaga tgtaattctc tgcgaataca ggactagttg gagctaacaa cgcagcttga 1440

cgagggtgaa cccagctcca cctcgtctac cacgtcttct cctcgcc 1487

<210> 5
<211> 1979
<212> DNA
<213> Zea mays

<400> 5
caaagcgtag atatttttcc tatcaaatgt tgcttccagg tcacataaat gcaaatattt 60

gtggagacgt acgagtgccc attaacctca tacactgtat ctgtatgaca aaagtcccac 120

gactcactgg acgcggaaat gtcgcttgac tacgccaatt ttctaaaaag attggcagca 180

attaattggg cttatagcgg taactttggt tcgcattagt tgtaggacta gagttgaata 240

tcgatccaac tcgccgtggc acggtcgagc tcaagctagc tccgctcagc tcattaaaga 300

attcagccag agaatcagct catgtcgttt acgagtttga gctggctcgt ttagctcgtg 360

aatcataaca aaaacaacat gcacatatat acaataatat aatcaatact agttaattct 420

agactagttt aacactagaa aagagtaata atactcataa tttcacatac aatatcaatc 480

taacaccaaa ttaacatatt tcgtcacttg ttagttcatc caatcaagtg taggctttgc 540

tttactgaca aatggttgct cgttcgagct agcgatcgag cttgcttgtt aataaaccga 600

gttgagatgc tagctcaatt tgtgacaaaa ttaaaataag ccgagtcgaa acaagttaag 660

cttatgacga ttcgagcaag ctcacgagcc acaagtatta ttttttttcc agtactacct 720

aagagcatga cgtcggtatg ttttctttac cgacgatggt ggtgattttt atttcgcaat 780

gcattggaaa actggcgaca tgagttcaaa cgcaatgcaa cacactacct cccacgagct 840

ggtttttgtg cgagacagta aagaagcaaa caaatctaac cagccaaatc cttggccggg 900

caccacagac cacggcgaac aactccccct ctctcttccg tccatcttat ttcttcgccg 960

tgcgtggctc tgcttgcgcc gacggccgac ggatgagcta ctaggattac ggctagcaac 1020

tcgggacggg caccttgcac gcggtgaaca ccctgcgctt gtgcctggcg tcgacgaggc 1080

gcttcaggct ggcgtcgtcc ttgtaccggc acaggcagcc gggcccgtgg gacatgagct 1140

tggcgcagca ggcggccgtg ggcgacgcct tccccgcgaa cgcgctcgcg caggggctca 1200

gctgcgtcac gtcgcacggc gccagggccg agaagtcgcc cgcggcgcgg ccggcgagga 1260

ggaggaggac gctggccagc accgcggcga ggaagagcgc ggccggcctc atcgtcccga 1320

ccgacgatag atggtgtcac tggatatata ggtcggaccg gactagctgc gagtggacta 1380

gctgggtggg aagccacgta cgagcgtcgt cgggagcttt tttgtaggcg ccgattagtt 1440

gtgtgctatg gcggattatt agcccggcgg aaggtgaggt gagtcacggc gtaagcatgc 1500

ggcacagtcg agcgagccca gcagtgtccg ttaggttagg tgaagcacgc tgcacgcccg 1560

gtggaagggc tcaccatttg atgctttctc atcgctcatc agtcatcact atgattctag 1620

agaatatatc tagtacatat ggtgtacata ttaaatgatc atcacttatt ttagatctaa 1680

tgtctctagt tgtttgatat attttgctaa tgacaccctc caacgttggt gtgtagtggt 1740

ggaaggtgtt atttgtaaat taaatgatca actagagacg ttagatctaa aataagtggt 1800

gatgatttaa tatatgcacc atgtacacca atcttctatg tgcaccagat atgtcctcat 1860

gattctaaca gagaggtacg tgcgcatcct gcgaacgtat tgttgagttt tttttaagtt 1920

caacacatgg ttacgtcatg atcctatact attctcgaat atagatgaac aaacgggcc 1979

<210> 6
<211> 920
<212> DNA
<213> Zea mays

<400> 6
atggctgctc acctaccacg cctccccgtc ctcctcctcg tcctcctcgc tgcccatgtc 60

gtctccacct ccgcccatgc cgagcctcct cttccgagcc cttacagcac ctccgcccat 120

ggcgagcctc ctcttccgag cacttacaac gtctccatgt gctcggaatc gttctggtgc 180

ggcggcgtcg aaatccgcta cccgttctat cttgccaacg caaccgccga ctacagcggg 240

agctactact cctgcggcta caccgacttg agcgtttcct gcaaactcga ggtcgagggg 300

ccgacgacga catggacccc taccatccgt ctcggcggcg acaactacac cgtcaagaac 360

atcttgtacg actatcatac catctcactg gcggacagcg atgtgctcgg aggcggcgag 420

tgccccgtcg tccaccacaa cgtcagcttc gacgagacgt ggctgcacaa ccccagcgcc 480

ttcgacaacc tcaccttctt cttcggatgc cactgggggc cacgcgatac actgcctgaa 540

tttgccggca acaacatcag ctgcgccggg ttcagtactc cagctatcag cggtggaggc 600

tccttcgtgt tcaagcctga agatcttgac gaacatgcgg agcaggagtt ggcttcacac 660

tgcgacgagg ttttctccgt gccagtgaga agcgaggctc tgcagcaggc gatcgtcagc 720

aacctcagcc tcggggacgg gtacggcgag ctgcttaggc aggggatcga gttggaatgg 780

aaacggacat cggaggatca gtgtggccag tgcgaggaat cgggctccgg cggacggtgc 840

gcctacagcc agaagagaga attccttggc tgcttctgca gcggagggaa ggcgggcaac 900

ccgttctgca aaccatcaag 920

<210> 7
<211> 37
<212> DNA
<213> Zea mays

<400> 7
atcaacaaaa aggagagaag cagcatctat tgttggt 37

<210> 8
<211> 1047
<212> DNA
<213> Zea mays

<400> 8
gctgttgccg ttgcattcct gtgtctagtc attctcacat gcttcttggc ttgtagacat 60

tgttcgctgc ccttcaaatc gaagaacaaa ccagggacaa ggattgagtc cttcctacag 120

aagaacgaga gtagtataca tccgaaaaga tacacctacg cggacgtgaa aagaatgaca 180

aaatccttcg ctgtgaagct aggccaaggt gggtttggtg ctgtatacaa aggcagcctc 240

cacgatggcc gacaggtagc agtcaagatg ctgaaggaca cccaaggtga cggcgaggaa 300

ttcatgaacg aggtggctag catcagcagg acttctcatg tcaacgtcgt gacacttcta 360

gggttttgct tgcaagggtc gaaaagagca ctgatctacg agtacatgcc caatggttcg 420

ctcgaaaggt atgccttcac cggtgacatg aacagtgaga atttgctaac ctgggaaaga 480

ctatttgaca tagcgattgg cacggccaga gggctcgaat acctacaccg gggatgcaac 540

actcggatcg tgcattttga catcaagcca cacaacatcc tgttagacca ggatttctgc 600

cctaagatct ctgactttgg actggccaag ctatgtctga acaaagagag cgctatctcc 660

attgctggcg caagagggac gatagggtat atcgccccgg aggtctactc aaagcaattt 720

ggaacgatca gcagcaagtc tgatgtctat agctatggga tgatggtcct tgagatggtt 780

ggagcaaggg acaggaatac aagcgcagat agtgaccata gcagccaata tttccctcag 840

tggctttatg aacatttgga cgactattgt gttggtgctt ccgagattaa tggtgagacc 900

acagagctcg tgaggaagat gatagttgta ggtctgtggt gcatacaagt gattccgact 960

gatcgaccaa caatgacgag agtcgtcgag atgttggaag gaagcacaag taatctagag 1020

ttgccaccca gagttctctt gagctga 1047

<210> 9
<211> 2004
<212> DNA
<213> Artificial Sequence

<220>
<223> cDNA of HtN1

<400>  9
   atggctgctc accagcctca cctctccgtc ctcctcctcg tcctcctcgc tgcccatgtc      60
   gtctccacct ccgcccatgg cgagcctcct cttccgagcc cttacaacac ctccgcccat     120
   ggcgagcctc ctcttccgag cacttacaac gcctccatgt gctcgtcgtt ctggtgtggc     180
   ggcgtcgaga tccgctaccc gttctatctt gccaacgcaa tcgccgacta cagcgggagc     240
   tactactcct gcggctacac cgacttgagc gtttcctgcg aactcgaggt cgaggggtcg     300
   ccgacgacct ggacccctac catccgtctc ggcggcggcg actacaccgt caagaacatc     360
   tcctacctct acgaccagca gaccatctca ctggcggaca gagatgtgct cggaggcggc     420
   ggctgccccg tcgtccgcca caacgtcagc ttcgacgaga cgtggctgca cctgcacaac     480
   gccagcgcct tcgacaacct caccttcttc ttcggatgcc actgggggcc acggaataca     540
   ccgcctgaat ttgccgacta caacatcagc tgcgccgggt tcaatactcc aactatcagc     600
   ggtggaaggt ccttcgtgtt caagactgga gatcttgacg aacaagagga gcaggagttg     660
   gctttacact gcgacgaggt tttctccgtg ccagtgagaa gagatgctct gcaggcgatc     720
   gtcagcaact tcagcctcac acgggacggg tacggcgagg tgcttaggca ggggttcgag     780
   ttggaatgga atcggacatc ggaggatcag tgtggccggt gcgagggatc gggctccggc     840
   ggatggtgcg cctacagcca gaagagagaa ttcctgggct gcttgtgcag cggagggaag     900
   gtgggcagcc cgttctgcaa accatcgaga tcaaaaagga aagaaggacc tattgttggt     960
   gctgttgccg ttgcattcct gtgtctagtc attctcacat gcttcttggc ttgtagacat    1020
   ggttcgctgc ccttcaaatc ggagaacaaa ccaggacaaa ggattgagtc cttcctacag    1080
   aagaacgaga gtatacatcc gaaaagatac acctacgcgg acgtgaaaag aatgacaaaa    1140
   tccttcgctg tgaagctagg ccaaggtggg tttggtgctg tatacaaagg cagcctccac    1200
   gatggccgac aggtagcagt caagatgctg aaggacaccc aaggtgacgg cgaggaattc    1260
   atgaacgagg tggctagcat cagcaggact tctcatgtca acgtcgtgac acttctaggg    1320
   ttttgcttgc aagggtcgaa aagagcactg atctacgagt acatgcccaa tggttcgctc    1380
   gaaaggtatg ccttcaccgg tgacatgaac agtgagaatt tgctaacctg ggaaaggcta    1440
   tttgacatag caattggcac ggccagaggg ctcgaatacc tacaccgggg atgcaacact    1500
   cggatcgtgc attttgacat caagccacac aacatcctgt tagaccagga tttctgtcct    1560
   aagatctctg actttggact ggccaagcta tgtctgaaca aagagagcgc tatctccatt    1620
   gctggcgcaa gagggacgat agggtatatc gccccggagg tctactcaaa gcaatttgga    1680
   ataataagca gcaagtctga tgtctatagc tatggatgat tggtccttga gatggttgga    1740
   gcaagggaca ggaatacaag cgcagatagt gaccatagca gccaatattt ccctcagtgg    1800
   ctttatgaac atttggacga ctattgtgtt ggtgcttccg agattaatgg tgagaccaca    1860
   gagctcgtga ggaagatgat agttgtaggt ctgtggtgca tacaagtgat tccgactgat 1920
   cgaccaacaa tgacgagagt cgtcgagatg ttggaaggga gcacaagtaa tctagagttg 1980
   ccacccagag ttctcttgag ctga 2004

<210> 10
<211> 667
<212> PRT
<213> Zea mays

<400> 10

Met Ala Ala His Gln Pro His Leu Ser Val Leu Leu Leu Val Leu Leu
1               5                   10                  15

Ala Ala His Val Val Ser Thr Ser Ala His Gly Glu Pro Pro Leu Pro
            20                  25                  30

Ser Pro Tyr Asn Thr Ser Ala His Gly Glu Pro Pro Leu Pro Ser Thr
        35                  40                  45

Tyr Asn Ala Ser Met Cys Ser Ser Phe Trp Cys Gly Gly Val Glu Ile
    50                  55                  60

Arg Tyr Pro Phe Tyr Leu Ala Asn Ala Ile Ala Asp Tyr Ser Gly Ser
65                  70                  75                  80

Tyr Tyr Ser Cys Gly Tyr Thr Asp Leu Ser Val Ser Cys Glu Leu Glu
                85                  90                  95

Val Glu Gly Ser Pro Thr Thr Trp Thr Pro Thr Ile Arg Leu Gly Gly
            100                 105                 110

Gly Asp Tyr Thr Val Lys Asn Ile Ser Tyr Leu Tyr Asp Gln Gln Thr
        115                 120                 125

Ile Ser Leu Ala Asp Arg Asp Val Leu Gly Gly Gly Gly Cys Pro Val
    130                 135                 140

Val Arg His Asn Val Ser Phe Asp Glu Thr Trp Leu His Leu His Asn
145                 150                 155                 160

Ala Ser Ala Phe Asp Asn Leu Thr Phe Phe Phe Gly Cys His Trp Gly
                165                 170                 175

Pro Arg Asn Thr Pro Pro Glu Phe Ala Asp Tyr Asn Ile Ser Cys Ala
            180                 185                 190

Gly Phe Asn Thr Pro Thr Ile Ser Gly Gly Arg Ser Phe Val Phe Lys
        195                 200                 205

Thr Gly Asp Leu Asp Glu Gln Glu Glu Gln Glu Leu Ala Leu His Cys
    210                 215                 220

Asp Glu Val Phe Ser Val Pro Val Arg Arg Asp Ala Leu Gln Ala Ile
225                 230                 235                 240

Val Ser Asn Phe Ser Leu Thr Arg Asp Gly Tyr Gly Glu Val Leu Arg
                245                 250                 255

Gln Gly Phe Glu Leu Glu Trp Asn Arg Thr Ser Glu Asp Gln Cys Gly
            260                 265                 270

Arg Cys Glu Gly Ser Gly Ser Gly Gly Trp Cys Ala Tyr Ser Gln Lys
        275                 280                 285

Arg Glu Phe Leu Gly Cys Leu Cys Ser Gly Gly Lys Val Gly Ser Pro
    290                 295                 300

Phe Cys Lys Pro Ser Arg Ser Lys Arg Lys Glu Gly Pro Ile Val Gly
305                 310                 315                 320

Ala Val Ala Val Ala Phe Leu Cys Leu Val Ile Leu Thr Cys Phe Leu
                325                 330                 335

Ala Cys Arg His Gly Ser Leu Pro Phe Lys Ser Glu Asn Lys Pro Gly
            340                 345                 350

Thr Arg Ile Glu Ser Phe Leu Gln Lys Asn Glu Ser Ile His Pro Lys
        355                 360                 365

Arg Tyr Thr Tyr Ala Asp Val Lys Arg Met Thr Lys Ser Phe Ala Val
    370                 375                 380

Lys Leu Gly Gln Gly Gly Phe Gly Ala Val Tyr Lys Gly Ser Leu His
385                 390                 395                 400

Asp Gly Arg Gln Val Ala Val Lys Met Leu Lys Asp Thr Gln Gly Asp
                405                 410                 415

Gly Glu Glu Phe Met Asn Glu Val Ala Ser Ile Ser Arg Thr Ser His
            420                 425                 430

Val Asn Val Val Thr Leu Leu Gly Phe Cys Leu Gln Gly Ser Lys Arg
        435                 440                 445

Ala Leu Ile Tyr Glu Tyr Met Pro Asn Gly Ser Leu Glu Arg Tyr Ala
    450                 455                 460

Phe Thr Gly Asp Met Asn Ser Glu Asn Leu Leu Thr Trp Glu Arg Leu
465                 470                 475                 480

Phe Asp Ile Ala Ile Gly Thr Ala Arg Gly Leu Glu Tyr Leu His Arg
                485                 490                 495

Gly Cys Asn Thr Arg Ile Val His Phe Asp Ile Lys Pro His Asn Ile
            500                 505                 510

Leu Leu Asp Gln Asp Phe Cys Pro Lys Ile Ser Asp Phe Gly Leu Ala
        515                 520                 525

Lys Leu Cys Leu Asn Lys Glu Ser Ala Ile Ser Ile Ala Gly Ala Arg
    530                 535                 540

Gly Thr Ile Gly Tyr Ile Ala Pro Glu Val Tyr Ser Lys Gln Phe Gly
545                 550                 555                 560

Ile Ile Ser Ser Lys Ser Asp Val Tyr Ser Tyr Gly Met Met Val Leu
                565                 570                 575

Glu Met Val Gly Ala Arg Asp Arg Asn Thr Ser Ala Asp Ser Asp His
            580                 585                 590

Ser Ser Gln Tyr Phe Pro Gln Trp Leu Tyr Glu His Leu Asp Asp Tyr
        595                 600                 605

Cys Val Gly Ala Ser Glu Ile Asn Gly Glu Thr Thr Glu Leu Val Arg
    610                 615                 620

Lys Met Ile Val Val Gly Leu Trp Cys Ile Gln Val Ile Pro Thr Asp
625                 630                 635                 640

Arg Pro Thr Met Thr Arg Val Val Glu Met Leu Glu Gly Ser Thr Ser
                645                 650                 655

Asn Leu Glu Leu Pro Pro Arg Val Leu Leu Ser
            660                 665

<210>  11
<211>  2004
<212>  DNA
<213>  Artificial Sequence

<220>

<223>  cDNA of RLK1 allele from PH99N

<400>  11
atggctgctc accagcctca cctctccgtc ctcctcctcg tcctcctcgc tgcccatgtc      60

gtctccacct ccgcccatgg cgagcctcct cttccgagcc cttacaacac ctccgcccat     120

ggcgagcctc ctcttccgag cacttacaac gcctccatgt gctcgtcgtt ctggtgtggc     180

ggcgtcgaga tccgctaccc gttctatctt gccaacgcaa tcgccgacta cagcgggagc     240

tactactcct gcggctacac cgacttgagc gtttcctgcg aactcgaggt cgaggggtcg     300

ccgacgacct ggacccctac catccgtctc ggcggcggcg actacaccgt caagaacatc     360

tcctacctct acgaccagca gaccatctca ctggcggaca gagatgtgct cggaggcggc     420

ggctgccccg tcgtccgcca caacgtcagc ttcgacgaga cgtggctgca cctgcacaac     480

gccagcgcct tcgacaacct caccttcttc ttcggatgcc actgggggcc acggaataca     540

ccgcctgaat ttgccgacta caacatcagc tgcgccgggt tcaatactcc aactatcagc     600

ggtggaaggt ccttcgtgtt caagactgga gatcttgacg aacaagagga gcaggagttg     660

gctttacact gcgacgaggt tttctccgtg ccagtgagaa gagatgctct gcaggcgatc     720

gtcagcaact tcagcctcac acgggacggg tacggcgagg tgcttaggca ggggttcgag     780

ttggaatgga atcggacatc ggaggatcag tgtggccggt gcgagggatc gggctccggc     840

ggatggtgcg cctacagcca gaagagagaa ttcctgggct gcttgtgcag cggagggaag     900

gtgggcagcc cgttctgcaa accatcgaga tcaaaaagga aagaaggacc tattgttggt     960

gctgttgccg ttgcattcct gtgtctagtc attctcacat gcttcttggc ttgtagacat    1020

ggttcgctgc ccttcaaatc ggagaacaaa ccagggacaa ggattgagtc cttcctacag    1080

aagaacgaga gtatacatcc gaaaagatac acctacgcgg acgtgaaaag aatgacaaaa    1140

tccttcgctg tgaagctagg ccaaggtggg tttggtgctg tatacaaagg cagcctccac    1200

gatggccgac aggtagcagt caagatgctg aaggacaccc aaggtgacgg cgaggaattc    1260

atgaacgagg tggctagcat cagcaggact tctcatgtca acgtcgtgac acttctaggg    1320

ttttgcttgc aagggtcgaa aagagcactg atctacgagt acatgcccaa tggttcgctc    1380

gaaaggtatg ccttcaccgg tgacatgaac agtgagaatt tgctaacctg ggaaaggcta    1440

tttgacatag caattggcac ggccagaggg ctcgaatacc tacaccgggg atgcaacact    1500

cggatcgtgc attttgacat caagccacac aacatcctgt tagaccagga tttctgtcct    1560

aagatctctg actttggact ggccaagcta tgtctgaaca aagagagcgc tatctccatt    1620

gctggcgcaa gagggacgat agggtatatc gccccggagg tctactcaaa gcaatttgga    1680

ataataagca gcaagtctga tgtctatagc tatgggatga tggtccttga gatggttgga    1740

gcaagggaca ggaatacaag cgcagatagt gaccatagca gccaatattt ccctcagtgg    1800

ctttatgaac atttggacga ctattgtgtt ggtgcttccg agattaatgg tgagaccaca 1860

gagctcgtga ggaagatgat agttgtaggt ctgtggtgca tacaagtgat tccgactgat 1920

cgaccaacaa tgacgagagt cgtcgagatg ttggaaggga gcacaagtaa tctagagttg 1980

ccacccagag ttctcttgag ctga 2004

<210> 12
<211> 667
<212> PRT
<213> Zea mays

<400> 12

Met Ala Ala His Gln Pro His Leu Ser Val Leu Leu Leu Val Leu Leu
1               5                   10                  15

Ala Ala His Val Val Ser Thr Ser Ala His Gly Glu Pro Pro Leu Pro
            20                  25                  30

Ser Pro Tyr Asn Thr Ser Ala His Gly Glu Pro Pro Leu Pro Ser Thr
        35                  40                  45

Tyr Asn Ala Ser Met Cys Ser Ser Phe Trp Cys Gly Gly Val Glu Ile
    50                  55                  60

Arg Tyr Pro Phe Tyr Leu Ala Asn Ala Ile Ala Asp Tyr Ser Gly Ser
65                  70                  75                  80

Tyr Tyr Ser Cys Gly Tyr Thr Asp Leu Ser Val Ser Cys Glu Leu Glu
                85                  90                  95

Val Glu Gly Ser Pro Thr Thr Trp Thr Pro Thr Ile Arg Leu Gly Gly
            100                 105                 110

Gly Asp Tyr Thr Val Lys Asn Ile Ser Tyr Leu Tyr Asp Gln Gln Thr
        115                 120                 125

Ile Ser Leu Ala Asp Arg Asp Val Leu Gly Gly Gly Gly Cys Pro Val
    130                 135                 140

Val Arg His Asn Val Ser Phe Asp Glu Thr Trp Leu His Leu His Asn
145                 150                 155                 160

Ala Ser Ala Phe Asp Asn Leu Thr Phe Phe Phe Gly Cys His Trp Gly
                165                 170                 175

Pro Arg Asn Thr Pro Pro Glu Phe Ala Asp Tyr Asn Ile Ser Cys Ala
            180                 185                 190

Gly Phe Asn Thr Pro Thr Ile Ser Gly Gly Arg Ser Phe Val Phe Lys
        195                 200                 205

Thr Gly Asp Leu Asp Glu Gln Glu Glu Gln Glu Leu Ala Leu His Cys
    210                 215                 220

Asp Glu Val Phe Ser Val Pro Val Arg Arg Asp Ala Leu Gln Ala Ile
225                 230                 235                 240

Val Ser Asn Phe Ser Leu Thr Arg Asp Gly Tyr Gly Glu Val Leu Arg
                245                 250                 255

Gln Gly Phe Glu Leu Glu Trp Asn Arg Thr Ser Glu Asp Gln Cys Gly
            260                 265                 270

Arg Cys Glu Gly Ser Gly Ser Gly Gly Trp Cys Ala Tyr Ser Gln Lys
        275                 280                 285

Arg Glu Phe Leu Gly Cys Leu Cys Ser Gly Gly Lys Val Gly Ser Pro
    290                 295                 300

Phe Cys Lys Pro Ser Arg Ser Lys Arg Lys Glu Gly Pro Ile Val Gly
305                 310                 315                 320

Ala Val Ala Val Ala Phe Leu Cys Leu Val Ile Leu Thr Cys Phe Leu
                325                 330                 335

Ala Cys Arg His Gly Ser Leu Pro Phe Lys Ser Glu Asn Lys Pro Gly
            340                 345                 350

Thr Arg Ile Glu Ser Phe Leu Gln Lys Asn Glu Ser Ile His Pro Lys
        355                 360                 365

Arg Tyr Thr Tyr Ala Asp Val Lys Arg Met Thr Lys Ser Phe Ala Val
    370                 375                 380

Lys Leu Gly Gln Gly Gly Phe Gly Ala Val Tyr Lys Gly Ser Leu His
385                 390                 395                 400

Asp Gly Arg Gln Val Ala Val Lys Met Leu Lys Asp Thr Gln Gly Asp
                405                 410                 415

Gly Glu Glu Phe Met Asn Glu Val Ala Ser Ile Ser Arg Thr Ser His
            420                 425                 430

Val Asn Val Val Thr Leu Leu Gly Phe Cys Leu Gln Gly Ser Lys Arg
        435                 440                 445

Ala Leu Ile Tyr Glu Tyr Met Pro Asn Gly Ser Leu Glu Arg Tyr Ala
    450                 455                 460

Phe Thr Gly Asp Met Asn Ser Glu Asn Leu Leu Thr Trp Glu Arg Leu
465                 470                 475                 480

Phe Asp Ile Ala Ile Gly Thr Ala Arg Gly Leu Glu Tyr Leu His Arg
                485                 490                 495

Gly Cys Asn Thr Arg Ile Val His Phe Asp Ile Lys Pro His Asn Ile
            500                 505                 510

Leu Leu Asp Gln Asp Phe Cys Pro Lys Ile Ser Asp Phe Gly Leu Ala
        515                 520                 525

Lys Leu Cys Leu Asn Lys Glu Ser Ala Ile Ser Ile Ala Gly Ala Arg
    530                 535                 540

Gly Thr Ile Gly Tyr Ile Ala Pro Glu Val Tyr Ser Lys Gln Phe Gly
545                 550                 555                 560

Ile Ile Ser Ser Lys Ser Asp Val Tyr Ser Tyr Gly Met Met Val Leu
                565                 570                 575

Glu Met Val Gly Ala Arg Asp Arg Asn Thr Ser Ala Asp Ser Asp His
            580                 585                 590

Ser Ser Gln Tyr Phe Pro Gln Trp Leu Tyr Glu His Leu Asp Asp Tyr
        595                 600                 605

Cys Val Gly Ala Ser Glu Ile Asn Gly Glu Thr Thr Glu Leu Val Arg
    610                 615                 620

Lys Met Ile Val Val Gly Leu Trp Cys Ile Gln Val Ile Pro Thr Asp
625                 630                 635                 640

Arg Pro Thr Met Thr Arg Val Val Glu Met Leu Glu Gly Ser Thr Ser
                645                 650                 655

Asn Leu Glu Leu Pro Pro Arg Val Leu Leu Ser
            660                 665

<210>  13
<211>  2001
<212>  DNA
<213>  Artificial Sequence

<220>
<223>  cDNA of RLK1 allele from PH26N

<400>  13
atggctgctc acctaccacg cctccccgtc ctcctcctcg tcctcctcgc tgctcatgtc      60

gtctccacct ccgcccatgc cgagcctcct cttccgagcc cttacagcac ctccgcccat     120

ggcgagcctc ctcttccgag cacttacaac gtctccatgt gctcggaatc gttctggtgc     180

ggcggcgtcg aaatccgcta cccgttctat cttgccaacg caaccgccga ctacagcggg     240

agctactact cctgcggcta caccgacttg agcgtttcct gcaaactcga ggtcgagggg     300

ccgacgacga catggacccc taccatccgt ctcggcggcg acaactacac cgtcaagaac     360

atcttgtacg actatcatac catctcactg gcggacagcg atgtgctcgg aggcggcgag     420

tgccccgtcg tccaccacaa cgtcagcttc gacgagacgt ggctgcacaa ccccagcgcc     480

ttcgacaacc tcaccttctt cttcggatgc cactgggggc cacgcgatac actgcctgaa     540

tttgccggca acaacatcag ctgcgccggg ttcagtactc cagctatcag cggtggaggc     600

tccttcgtgt tcaagcctga agatcttgac gaacatgcgg agcaggagtt ggcttcacac     660

tgcgacgagg ttttctccgt gccagtgaga agcgaggctc tgcagcaggc gatcgtcagc     720

aacctcagcc tcggggacgg gtacggcgag ctgcttaggc aggggatcga gttggaatgg     780

aaacggacat cggaggatca gtgtggccag tgcgaggaat cgggctccgg cggacggtgc     840

gcctacagcc agaagagaga attccttggc tgcttgtgca gcggagggaa ggcgggcaac     900

ccgttctgca aaccatcaag atcaaaaagg aaagaagcat ctattgttgg tgctgttgcc     960

gttgcattcc tgtgtctagt cattctcaca tgcttcttgg cttgtagaca tggttcgctg    1020

cccttcaaat cggagaacaa accagggaca aggattgagt ccttcctaca gaagaacgag    1080

agtatacatc cgaaaagata cacctacacg gacgtgaaaa gaatgacaaa atccttcgct    1140

gtgaagctag gccaaggtgg gtttggtgct gtatacaaag gcagcctcca cgatggccga    1200

caggtagcag tcaagatgct caaggacacc caaggtgacg gcgaggaatt catgaacgag    1260

gtggctagca tcagcaggac ttctcatgtc aacgtcgtga cacttctagg gttttgcttg    1320

caagggtcga aaagagcact gatctacgag tacatgccca atggttcgct cgaaaggtat    1380

gccttcaccg gtgacatgaa cagtgagaat ttgctaacct gggaaaggct atttgacata    1440

gcaattggca cggccagagg gctcgaatac ctacaccggg gatgcaacac tcggatcgtg    1500

cattttgaca tcaagccaca caacatcctg ttagaccagg atttctgtcc taagatctct    1560

gactttggac tggccaagct atgtctgaac aaagagagcg ctatctccat tgttggcgca    1620

agagggacga tagggtatat cgccccggag gtctactcaa agcaatttgg aacaatcagc    1680

agcaagtctg atgtctatag ctatgggatg atggtccttg agatggttgg agcaagggaa    1740

aggaatacaa gcgcaagcgc agatagtgac catagcagcc aatatttccc tcagtggatt    1800

tatgaacatt tggacgacta ttgtgttggt gcttccgaga ttaatggtga gaccacagag 1860

ctcgtgagga agatgatagt tgtaggtctg tggtgcatac aagtgattcc gactgatcga 1920

ccaacaatga cgagagtcgt cgagatgttg gaagggagca cgagtaatct agagttgcca 1980

cccagagttc tcttgagctg a 2001

<210> 14
<211> 666
<212> PRT
<213> Zea mays

<400> 14

Met Ala Ala His Leu Pro Arg Leu Pro Val Leu Leu Leu Val Leu Leu
1               5                   10                  15

Ala Ala His Val Val Ser Thr Ser Ala His Ala Glu Pro Pro Leu Pro
            20                  25                  30

Ser Pro Tyr Ser Thr Ser Ala His Gly Glu Pro Pro Leu Pro Ser Thr
        35                  40                  45

Tyr Asn Val Ser Met Cys Ser Glu Ser Phe Trp Cys Gly Gly Val Glu
    50                  55                  60

Ile Arg Tyr Pro Phe Tyr Leu Ala Asn Ala Thr Ala Asp Tyr Ser Gly
65                  70                  75                  80

Ser Tyr Tyr Ser Cys Gly Tyr Thr Asp Leu Ser Val Ser Cys Lys Leu
                85                  90                  95

Glu Val Glu Gly Pro Thr Thr Thr Trp Thr Pro Thr Ile Arg Leu Gly
            100                 105                 110

Gly Asp Asn Tyr Thr Val Lys Asn Ile Leu Tyr Asp Tyr His Thr Ile
        115                 120                 125

Ser Leu Ala Asp Ser Asp Val Leu Gly Gly Gly Glu Cys Pro Val Val
    130                 135                 140

His His Asn Val Ser Phe Asp Glu Thr Trp Leu His Asn Pro Ser Ala
145                 150                 155                 160

Phe Asp Asn Leu Thr Phe Phe Phe Gly Cys His Trp Gly Pro Arg Asp
                165                 170                 175

Thr Leu Pro Glu Phe Ala Gly Asn Asn Ile Ser Cys Ala Gly Phe Ser
            180                 185                 190

Thr Pro Ala Ile Ser Gly Gly Gly Ser Phe Val Phe Lys Pro Glu Asp
        195                 200                 205

Leu Asp Glu His Ala Glu Gln Glu Leu Ala Ser His Cys Asp Glu Val
    210                 215                 220

Phe Ser Val Pro Val Arg Ser Glu Ala Leu Gln Gln Ala Ile Val Ser
225                 230                 235                 240

Asn Leu Ser Leu Gly Asp Gly Tyr Gly Glu Leu Leu Arg Gln Gly Ile
                245                 250                 255

Glu Leu Glu Trp Lys Arg Thr Ser Glu Asp Gln Cys Gly Gln Cys Glu
            260                 265                 270

Glu Ser Gly Ser Gly Gly Arg Cys Ala Tyr Ser Gln Lys Arg Glu Phe
        275                 280                 285

Leu Gly Cys Leu Cys Ser Gly Gly Lys Ala Gly Asn Pro Phe Cys Lys
    290                 295                 300

Pro Ser Arg Ser Lys Arg Lys Glu Ala Ser Ile Val Gly Ala Val Ala
305                 310                 315                 320

Val Ala Phe Leu Cys Leu Val Ile Leu Thr Cys Phe Leu Ala Cys Arg
                325                 330                 335

His Gly Ser Leu Pro Phe Lys Ser Glu Asn Lys Pro Gly Thr Arg Ile
            340                 345                 350

Glu Ser Phe Leu Gln Lys Asn Glu Ser Ile His Pro Lys Arg Tyr Thr
        355                 360                 365

Tyr Thr Asp Val Lys Arg Met Thr Lys Ser Phe Ala Val Lys Leu Gly
    370                 375                 380

Gln Gly Gly Phe Gly Ala Val Tyr Lys Gly Ser Leu His Asp Gly Arg
385                 390                 395                 400

Gln Val Ala Val Lys Met Leu Lys Asp Thr Gln Gly Asp Gly Glu Glu
                405                 410                 415

Phe Met Asn Glu Val Ala Ser Ile Ser Arg Thr Ser His Val Asn Val
            420                 425                 430

Val Thr Leu Leu Gly Phe Cys Leu Gln Gly Ser Lys Arg Ala Leu Ile

435                 440                 445

Tyr Glu Tyr Met Pro Asn Gly Ser Leu Glu Arg Tyr Ala Phe Thr Gly
    450                 455                 460

Asp Met Asn Ser Glu Asn Leu Leu Thr Trp Glu Arg Leu Phe Asp Ile
465                 470                 475                 480

Ala Ile Gly Thr Ala Arg Gly Leu Glu Tyr Leu His Arg Gly Cys Asn
                485                 490                 495

Thr Arg Ile Val His Phe Asp Ile Lys Pro His Asn Ile Leu Leu Asp
            500                 505                 510

Gln Asp Phe Cys Pro Lys Ile Ser Asp Phe Gly Leu Ala Lys Leu Cys
        515                 520                 525

Leu Asn Lys Glu Ser Ala Ile Ser Ile Val Gly Ala Arg Gly Thr Ile
    530                 535                 540

Gly Tyr Ile Ala Pro Glu Val Tyr Ser Lys Gln Phe Gly Thr Ile Ser
545                 550                 555                 560

Ser Lys Ser Asp Val Tyr Ser Tyr Gly Met Met Val Leu Glu Met Val
                565                 570                 575

Gly Ala Arg Glu Arg Asn Thr Ser Ala Ser Ala Asp Ser Asp His Ser
            580                 585                 590

Ser Gln Tyr Phe Pro Gln Trp Ile Tyr Glu His Leu Asp Asp Tyr Cys
        595                 600                 605

Val Gly Ala Ser Glu Ile Asn Gly Glu Thr Thr Glu Leu Val Arg Lys
    610                 615                 620

Met Ile Val Val Gly Leu Trp Cys Ile Gln Val Ile Pro Thr Asp Arg
625                 630                 635                 640

Pro Thr Met Thr Arg Val Val Glu Met Leu Glu Gly Ser Thr Ser Asn
                645                 650                 655

Leu Glu Leu Pro Pro Arg Val Leu Leu Ser
            660                 665

<210>  15
<211>  2004
<212>  DNA
<213>  Artificial Sequence

<220>
<223>  mutant Ht2 cDNA (position 1625: G->A)

<400>  15
atggctgctc acctaccacg cctccccgtc ctcctcctcg tcctcctcgc tgcccatgtc      60

gtctccacct ccgcccatgc cgagcctcct cttccgagcc cttacagcac ctccgcccat     120

ggcgagcctc ctcttccgag cacttacaac gtctccatgt gctcggaatc gttctggtgc     180

ggcggcgtcg aaatccgcta cccgttctat cttgccaacg caaccgccga ctacagcggg     240

agctactact cctgcggcta caccgacttg agcgtttcct gcaaactcga ggtcgagggg     300

ccgacgacga catggacccc taccatccgt ctcggcggcg acaactacac cgtcaagaac     360

atcttgtacg actatcatac catctcactg gcggacagcg atgtgctcgg aggcggcgag     420

tgccccgtcg tccaccacaa cgtcagcttc gacgagacgt ggctgcacaa ccccagcgcc     480

ttcgacaacc tcaccttctt cttcggatgc cactgggggc cacgcgatac actgcctgaa     540

tttgccggca acaacatcag ctgcgccggg ttcagtactc cagctatcag cggtggaggc     600

tccttcgtgt tcaagcctga agatcttgac gaacatgcgg agcaggagtt ggcttcacac     660

tgcgacgagg ttttctccgt gccagtgaga agcgaggctc tgcagcaggc gatcgtcagc     720

aacctcagcc tcggggacgg gtacggcgag ctgcttaggc aggggatcga gttggaatgg     780

aaacggacat cggaggatca gtgtggccag tgcgaggaat cgggctccgg cggacggtgc     840

gcctacagcc agaagagaga attccttggc tgcttctgca gcggagggaa ggcgggcaac     900

ccgttctgca aaccatcaag atcaacaaaa aggagagaag cagcatctat tgttggtgct     960

gttgccgttg cattcctgtg tctagtcatt ctcacatgct tcttggcttg tagacattgt    1020

tcgctgccct tcaaatcgaa gaacaaacca gggacaagga ttgagtcctt cctacagaag    1080

aacgagagta gtatacatcc gaaaagatac acctacgcgg acgtgaaaag aatgacaaaa    1140

tccttcgctg tgaagctagg ccaaggtggg tttggtgctg tatacaaagg cagcctccac    1200

gatggccgac aggtagcagt caagatgctg aaggacaccc aaggtgacgg cgaggaattc    1260

atgaacgagg tggctagcat cagcaggact tctcatgtca acgtcgtgac acttctaggg    1320

ttttgcttgc aagggtcgaa aagagcactg atctacgagt acatgcccaa tggttcgctc    1380

gaaaggtatg ccttcaccgg tgacatgaac agtgagaatt tgctaacctg ggaaagacta    1440

tttgacatag cgattggcac ggccagaggg ctcgaatacc tacaccgggg atgcaacact    1500

cggatcgtgc attttgacat caagccacac aacatcctgt tagaccagga tttctgccct    1560

aagatctctg actttggact ggccaagcta tgtctgaaca aagagagcgc tatctccatt    1620

gctgacgcaa gagggacgat agggtatatc gccccggagg tctactcaaa gcaatttgga    1680

acgatcagca gcaagtctga tgtctatagc tatgggatga tggtccttga gatggttgga    1740

gcaagggaca ggaatacaag cgcagatagt gaccatagca gccaatattt ccctcagtgg 1800

ctttatgaac atttggacga ctattgtgtt ggtgcttccg agattaatgg tgagaccaca 1860

gagctcgtga ggaagatgat agttgtaggt ctgtggtgca tacaagtgat tccgactgat 1920

cgaccaacaa tgacgagagt cgtcgagatg ttggaaggaa gcacaagtaa tctagagttg 1980

ccacccagag ttctcttgag ctga 2004

<210> 16
<211> 667
<212> PRT
<213> Artificial Sequence

<220>
<223> mutant Ht2 protein (position 542: G->D)

<400> 16

Met Ala Ala His Leu Pro Arg Leu Pro Val Leu Leu Leu Val Leu Leu
1               5                   10                  15

Ala Ala His Val Val Ser Thr Ser Ala His Ala Glu Pro Pro Leu Pro
            20                  25                  30

Ser Pro Tyr Ser Thr Ser Ala His Gly Glu Pro Pro Leu Pro Ser Thr
        35                  40                  45

Tyr Asn Val Ser Met Cys Ser Glu Ser Phe Trp Cys Gly Gly Val Glu
    50                  55                  60

Ile Arg Tyr Pro Phe Tyr Leu Ala Asn Ala Thr Ala Asp Tyr Ser Gly
65                  70                  75                  80

Ser Tyr Tyr Ser Cys Gly Tyr Thr Asp Leu Ser Val Ser Cys Lys Leu
                85                  90                  95

Glu Val Glu Gly Pro Thr Thr Thr Trp Thr Pro Thr Ile Arg Leu Gly
            100                 105                 110

Gly Asp Asn Tyr Thr Val Lys Asn Ile Leu Tyr Asp Tyr His Thr Ile
        115                 120                 125

Ser Leu Ala Asp Ser Asp Val Leu Gly Gly Gly Glu Cys Pro Val Val
    130                 135                 140

His His Asn Val Ser Phe Asp Glu Thr Trp Leu His Asn Pro Ser Ala
145                 150                 155                 160

Phe Asp Asn Leu Thr Phe Phe Phe Gly Cys His Trp Gly Pro Arg Asp
                165                 170                 175

Thr Leu Pro Glu Phe Ala Gly Asn Asn Ile Ser Cys Ala Gly Phe Ser
        180                 185                 190

Thr Pro Ala Ile Ser Gly Gly Gly Ser Phe Val Phe Lys Pro Glu Asp
    195                 200                 205

Leu Asp Glu His Ala Glu Gln Glu Leu Ala Ser His Cys Asp Glu Val
    210                 215                 220

Phe Ser Val Pro Val Arg Ser Glu Ala Leu Gln Gln Ala Ile Val Ser
225                 230                 235                 240

Asn Leu Ser Leu Gly Asp Gly Tyr Gly Glu Leu Leu Arg Gln Gly Ile
                245                 250                 255

Glu Leu Glu Trp Lys Arg Thr Ser Glu Asp Gln Cys Gly Gln Cys Glu
            260                 265                 270

Glu Ser Gly Ser Gly Gly Arg Cys Ala Tyr Ser Gln Lys Arg Glu Phe
        275                 280                 285

Leu Gly Cys Phe Cys Ser Gly Gly Lys Ala Gly Asn Pro Phe Cys Lys
    290                 295                 300

Pro Ser Arg Ser Thr Lys Arg Arg Glu Ala Ala Ser Ile Val Gly Ala
305                 310                 315                 320

Val Ala Val Ala Phe Leu Cys Leu Val Ile Leu Thr Cys Phe Leu Ala
                325                 330                 335

Cys Arg His Cys Ser Leu Pro Phe Lys Ser Lys Asn Lys Pro Gly Thr
            340                 345                 350

Arg Ile Glu Ser Phe Leu Gln Lys Asn Glu Ser Ser Ile His Pro Lys
        355                 360                 365

Arg Tyr Thr Tyr Ala Asp Val Lys Arg Met Thr Lys Ser Phe Ala Val
    370                 375                 380

Lys Leu Gly Gln Gly Gly Phe Gly Ala Val Tyr Lys Gly Ser Leu His
385                 390                 395                 400

Asp Gly Arg Gln Val Ala Val Lys Met Leu Lys Asp Thr Gln Gly Asp
                405                 410                 415

Gly Glu Glu Phe Met Asn Glu Val Ala Ser Ile Ser Arg Thr Ser His

            420                 425                 430

Val Asn Val Val Thr Leu Leu Gly Phe Cys Leu Gln Gly Ser Lys Arg
        435                 440                 445

Ala Leu Ile Tyr Glu Tyr Met Pro Asn Gly Ser Leu Glu Arg Tyr Ala
    450                 455                 460

Phe Thr Gly Asp Met Asn Ser Glu Asn Leu Leu Thr Trp Glu Arg Leu
465                 470                 475                 480

Phe Asp Ile Ala Ile Gly Thr Ala Arg Gly Leu Glu Tyr Leu His Arg
                485                 490                 495

Gly Cys Asn Thr Arg Ile Val His Phe Asp Ile Lys Pro His Asn Ile
            500                 505                 510

Leu Leu Asp Gln Asp Phe Cys Pro Lys Ile Ser Asp Phe Gly Leu Ala
        515                 520                 525

Lys Leu Cys Leu Asn Lys Glu Ser Ala Ile Ser Ile Ala Asp Ala Arg
    530                 535                 540

Gly Thr Ile Gly Tyr Ile Ala Pro Glu Val Tyr Ser Lys Gln Phe Gly
545                 550                 555                 560

Thr Ile Ser Ser Lys Ser Asp Val Tyr Ser Tyr Gly Met Met Val Leu
                565                 570                 575

Glu Met Val Gly Ala Arg Asp Arg Asn Thr Ser Ala Asp Ser Asp His
            580                 585                 590

Ser Ser Gln Tyr Phe Pro Gln Trp Leu Tyr Glu His Leu Asp Asp Tyr
        595                 600                 605

Cys Val Gly Ala Ser Glu Ile Asn Gly Glu Thr Thr Glu Leu Val Arg
    610                 615                 620

Lys Met Ile Val Val Gly Leu Trp Cys Ile Gln Val Ile Pro Thr Asp
625                 630                 635                 640

Arg Pro Thr Met Thr Arg Val Val Glu Met Leu Glu Gly Ser Thr Ser
                645                 650                 655

Asn Leu Glu Leu Pro Pro Arg Val Leu Leu Ser
            660                 665

<210>  17
<211>  2004
<212>  DNA
<213>  Artificial Sequence

<220>
<223>  mutant Ht2 cDNA (position 95: C->T)

<400>  17
atggctgctc acctaccacg cctccccgtc ctcctcctcg tcctcctcgc tgcccatgtc      60

gtctccacct ccgcccatgc cgagcctcct cttctgagcc cttacagcac ctccgcccat     120

ggcgagcctc ctcttccgag cacttacaac gtctccatgt gctcggaatc gttctggtgc     180

ggcggcgtcg aaatccgcta cccgttctat cttgccaacg caaccgccga ctacagcggg     240

agctactact cctgcggcta caccgacttg agcgtttcct gcaaactcga ggtcgagggg     300

ccgacgacga catggacccc taccatccgt ctcggcggcg acaactacac cgtcaagaac     360

atcttgtacg actatcatac catctcactg gcggacagcg atgtgctcgg aggcggcgag     420

tgccccgtcg tccaccacaa cgtcagcttc gacgagacgt ggctgcacaa ccccagcgcc     480

ttcgacaacc tcaccttctt cttcggatgc cactgggggc cacgcgatac actgcctgaa     540

tttgccggca acaacatcag ctgcgccggg ttcagtactc cagctatcag cggtggaggc     600

tccttcgtgt tcaagcctga agatcttgac gaacatgcgg agcaggagtt ggcttcacac     660

tgcgacgagg ttttctccgt gccagtgaga agcgaggctc tgcagcaggc gatcgtcagc     720

aacctcagcc tcggggacgg gtacggcgag ctgcttaggc aggggatcga gttggaatgg     780

aaacggacat cggaggatca gtgtggccag tgcgaggaat cgggctccgg cggacggtgc     840

gcctacagcc agaagagaga attccttggc tgcttctgca gcggagggaa ggcgggcaac     900

ccgttctgca aaccatcaag atcaacaaaa aggagagaag cagcatctat tgttggtgct     960

gttgccgttg cattcctgtg tctagtcatt ctcacatgct tcttggcttg tagacattgt    1020

tcgctgccct tcaaatcgaa gaacaaacca gggacaagga ttgagtcctt cctacagaag    1080

aacgagagta gtatacatcc gaaaagatac acctacgcgg acgtgaaaag aatgacaaaa    1140

tccttcgctg tgaagctagg ccaaggtggg tttggtgctg tatacaaagg cagcctccac    1200

gatggccgac aggtagcagt caagatgctg aaggacaccc aaggtgacgg cgaggaattc    1260

atgaacgagg tggctagcat cagcaggact tctcatgtca acgtcgtgac acttctaggg    1320

ttttgcttgc aagggtcgaa aagagcactg atctacgagt acatgcccaa tggttcgctc    1380

gaaaggtatg ccttcaccgg tgacatgaac agtgagaatt tgctaacctg ggaaagacta    1440

tttgacatag cgattggcac ggccagaggg ctcgaatacc tacaccgggg atgcaacact    1500

cggatcgtgc attttgacat caagccacac aacatcctgt tagaccagga tttctgccct    1560

aagatctctg actttggact ggccaagcta tgtctgaaca aagagagcgc tatctccatt    1620

gctggcgcaa gagggacgat agggtatatc gccccggagg tctactcaaa gcaatttgga 1680

acgatcagca gcaagtctga tgtctatagc tatgggatga tggtccttga gatggttgga 1740

gcaagggaca ggaatacaag cgcagatagt gaccatagca gccaatattt ccctcagtgg 1800

ctttatgaac atttggacga ctattgtgtt ggtgcttccg agattaatgg tgagaccaca 1860

gagctcgtga ggaagatgat agttgtaggt ctgtggtgca tacaagtgat tccgactgat 1920

cgaccaacaa tgacgagagt cgtcgagatg ttggaaggaa gcacaagtaa tctagagttg 1980

ccacccagag ttctcttgag ctga 2004

<210> 18
<211> 667
<212> PRT
<213> Artificial Sequence

<220>
<223> mutant Ht2 protein (position 32: P->L)

<400> 18

Met Ala Ala His Leu Pro Arg Leu Pro Val Leu Leu Leu Val Leu Leu
1               5                   10                  15

Ala Ala His Val Val Ser Thr Ser Ala His Ala Glu Pro Pro Leu Leu
            20                  25                  30

Ser Pro Tyr Ser Thr Ser Ala His Gly Glu Pro Pro Leu Pro Ser Thr
        35                  40                  45

Tyr Asn Val Ser Met Cys Ser Glu Ser Phe Trp Cys Gly Gly Val Glu
    50                  55                  60

Ile Arg Tyr Pro Phe Tyr Leu Ala Asn Ala Thr Ala Asp Tyr Ser Gly
65                  70                  75                  80

Ser Tyr Tyr Ser Cys Gly Tyr Thr Asp Leu Ser Val Ser Cys Lys Leu
                85                  90                  95

Glu Val Glu Gly Pro Thr Thr Thr Trp Thr Pro Thr Ile Arg Leu Gly
            100                 105                 110

Gly Asp Asn Tyr Thr Val Lys Asn Ile Leu Tyr Asp Tyr His Thr Ile
        115                 120                 125

Ser Leu Ala Asp Ser Asp Val Leu Gly Gly Gly Glu Cys Pro Val Val
    130                 135                 140

His His Asn Val Ser Phe Asp Glu Thr Trp Leu His Asn Pro Ser Ala
145                 150                 155                 160

Phe Asp Asn Leu Thr Phe Phe Phe Gly Cys His Trp Gly Pro Arg Asp
                165                 170                 175

Thr Leu Pro Glu Phe Ala Gly Asn Asn Ile Ser Cys Ala Gly Phe Ser
            180                 185                 190

Thr Pro Ala Ile Ser Gly Gly Gly Ser Phe Val Phe Lys Pro Glu Asp
        195                 200                 205

Leu Asp Glu His Ala Glu Gln Glu Leu Ala Ser His Cys Asp Glu Val
    210                 215                 220

Phe Ser Val Pro Val Arg Ser Glu Ala Leu Gln Gln Ala Ile Val Ser
225                 230                 235                 240

Asn Leu Ser Leu Gly Asp Gly Tyr Gly Glu Leu Leu Arg Gln Gly Ile
                245                 250                 255

Glu Leu Glu Trp Lys Arg Thr Ser Glu Asp Gln Cys Gly Gln Cys Glu
            260                 265                 270

Glu Ser Gly Ser Gly Gly Arg Cys Ala Tyr Ser Gln Lys Arg Glu Phe
        275                 280                 285

Leu Gly Cys Phe Cys Ser Gly Gly Lys Ala Gly Asn Pro Phe Cys Lys
    290                 295                 300

Pro Ser Arg Ser Thr Lys Arg Arg Glu Ala Ala Ser Ile Val Gly Ala
305                 310                 315                 320

Val Ala Val Ala Phe Leu Cys Leu Val Ile Leu Thr Cys Phe Leu Ala
                325                 330                 335

Cys Arg His Cys Ser Leu Pro Phe Lys Ser Lys Asn Lys Pro Gly Thr
            340                 345                 350

Arg Ile Glu Ser Phe Leu Gln Lys Asn Glu Ser Ser Ile His Pro Lys
        355                 360                 365

Arg Tyr Thr Tyr Ala Asp Val Lys Arg Met Thr Lys Ser Phe Ala Val
    370                 375                 380

Lys Leu Gly Gln Gly Gly Phe Gly Ala Val Tyr Lys Gly Ser Leu His
385                 390                 395                 400

Asp Gly Arg Gln Val Ala Val Lys Met Leu Lys Asp Thr Gln Gly Asp

405                 410                 415

Gly Glu Glu Phe Met Asn Glu Val Ala Ser Ile Ser Arg Thr Ser His
            420                 425                 430

Val Asn Val Val Thr Leu Leu Gly Phe Cys Leu Gln Gly Ser Lys Arg
        435                 440                 445

Ala Leu Ile Tyr Glu Tyr Met Pro Asn Gly Ser Leu Glu Arg Tyr Ala
    450                 455                 460

Phe Thr Gly Asp Met Asn Ser Glu Asn Leu Leu Thr Trp Glu Arg Leu
465                 470                 475                 480

Phe Asp Ile Ala Ile Gly Thr Ala Arg Gly Leu Glu Tyr Leu His Arg
                485                 490                 495

Gly Cys Asn Thr Arg Ile Val His Phe Asp Ile Lys Pro His Asn Ile
            500                 505                 510

Leu Leu Asp Gln Asp Phe Cys Pro Lys Ile Ser Asp Phe Gly Leu Ala
        515                 520                 525

Lys Leu Cys Leu Asn Lys Glu Ser Ala Ile Ser Ile Ala Gly Ala Arg
    530                 535                 540

Gly Thr Ile Gly Tyr Ile Ala Pro Glu Val Tyr Ser Lys Gln Phe Gly
545                 550                 555                 560

Thr Ile Ser Ser Lys Ser Asp Val Tyr Ser Tyr Gly Met Met Val Leu
                565                 570                 575

Glu Met Val Gly Ala Arg Asp Arg Asn Thr Ser Ala Asp Ser Asp His
            580                 585                 590

Ser Ser Gln Tyr Phe Pro Gln Trp Leu Tyr Glu His Leu Asp Asp Tyr
        595                 600                 605

Cys Val Gly Ala Ser Glu Ile Asn Gly Glu Thr Thr Glu Leu Val Arg
    610                 615                 620

Lys Met Ile Val Val Gly Leu Trp Cys Ile Gln Val Ile Pro Thr Asp
625                 630                 635                 640

Arg Pro Thr Met Thr Arg Val Val Glu Met Leu Glu Gly Ser Thr Ser
                645                 650                 655

Asn Leu Glu Leu Pro Pro Arg Val Leu Leu Ser
            660                 665

<210>  19
<211>  2004
<212>  DNA
<213>  Artificial Sequence

<220>
<223>  mutant Ht2 cDNA (position 115: G->A)

<400>  19
atggctgctc acctaccacg cctccccgtc ctcctcctcg tcctcctcgc tgcccatgtc      60

gtctccacct ccgcccatgc cgagcctcct cttccgagcc cttacagcac ctccacccat     120

ggcgagcctc ctcttccgag cacttacaac gtctccatgt gctcggaatc gttctggtgc     180

ggcggcgtcg aaatccgcta cccgttctat cttgccaacg caaccgccga ctacagcggg     240

agctactact cctgcggcta caccgacttg agcgtttcct gcaaactcga ggtcgagggg     300

ccgacgacga catggacccc taccatccgt ctcggcggcg acaactacac cgtcaagaac     360

atcttgtacg actatcatac catctcactg gcggacagcg atgtgctcgg aggcggcgag     420

tgccccgtcg tccaccacaa cgtcagcttc gacgagacgt ggctgcacaa ccccagcgcc     480

ttcgacaacc tcaccttctt cttcggatgc cactgggggc cacgcgatac actgcctgaa     540

tttgccggca acaacatcag ctgcgccggg ttcagtactc cagctatcag cggtggaggc     600

tccttcgtgt tcaagcctga agatcttgac gaacatgcgg agcaggagtt ggcttcacac     660

tgcgacgagg ttttctccgt gccagtgaga agcgaggctc tgcagcaggc gatcgtcagc     720

aacctcagcc tcggggacgg gtacggcgag ctgcttaggc aggggatcga gttggaatgg     780

aaacggacat cggaggatca gtgtggccag tgcgaggaat cgggctccgg cggacggtgc     840

gcctacagcc agaagagaga attccttggc tgcttctgca gcggagggaa ggcgggcaac     900

ccgttctgca aaccatcaag atcaacaaaa aggagagaag cagcatctat tgttggtgct     960

gttgccgttg cattcctgtg tctagtcatt ctcacatgct tcttggcttg tagacattgt    1020

tcgctgccct tcaaatcgaa gaacaaacca gggacaagga ttgagtcctt cctacagaag    1080

aacgagagta gtatacatcc gaaaagatac acctacgcgg acgtgaaaag aatgacaaaa    1140

tccttcgctg tgaagctagg ccaaggtggg tttggtgctg tatacaaagg cagcctccac    1200

gatggccgac aggtagcagt caagatgctg aaggacaccc aaggtgacgg cgaggaattc    1260

atgaacgagg tggctagcat cagcaggact tctcatgtca acgtcgtgac acttctaggg    1320

ttttgcttgc aagggtcgaa aagagcactg atctacgagt acatgcccaa tggttcgctc    1380

gaaaggtatg ccttcaccgg tgacatgaac agtgagaatt tgctaacctg ggaaagacta    1440

tttgacatag cgattggcac ggccagaggg ctcgaatacc tacaccgggg atgcaacact    1500

cggatcgtgc attttgacat caagccacac aacatcctgt tagaccagga tttctgccct 1560

aagatctctg actttggact ggccaagcta tgtctgaaca aagagagcgc tatctccatt 1620

gctggcgcaa gagggacgat agggtatatc gccccggagg tctactcaaa gcaatttgga 1680

acgatcagca gcaagtctga tgtctatagc tatgggatga tggtccttga gatggttgga 1740

gcaagggaca ggaatacaag cgcagatagt gaccatagca gccaatattt ccctcagtgg 1800

ctttatgaac atttggacga ctattgtgtt ggtgcttccg agattaatgg tgagaccaca 1860

gagctcgtga ggaagatgat agttgtaggt ctgtggtgca tacaagtgat tccgactgat 1920

cgaccaacaa tgacgagagt cgtcgagatg ttggaaggaa gcacaagtaa tctagagttg 1980

ccacccagag ttctcttgag ctga 2004

<210> 20
<211> 667
<212> PRT
<213> Artificial Sequence

<220>
<223> mutant Ht2 protein (position 39: A->T)

<400> 20

Met Ala Ala His Leu Pro Arg Leu Pro Val Leu Leu Leu Val Leu Leu
1               5                   10                  15

Ala Ala His Val Val Ser Thr Ser Ala His Ala Glu Pro Pro Leu Pro
            20                  25                  30

Ser Pro Tyr Ser Thr Ser Thr His Gly Glu Pro Pro Leu Pro Ser Thr
        35                  40                  45

Tyr Asn Val Ser Met Cys Ser Glu Ser Phe Trp Cys Gly Gly Val Glu
    50                  55                  60

Ile Arg Tyr Pro Phe Tyr Leu Ala Asn Ala Thr Ala Asp Tyr Ser Gly
65                  70                  75                  80

Ser Tyr Tyr Ser Cys Gly Tyr Thr Asp Leu Ser Val Ser Cys Lys Leu
                85                  90                  95

Glu Val Glu Gly Pro Thr Thr Thr Trp Thr Pro Thr Ile Arg Leu Gly
            100                 105                 110

Gly Asp Asn Tyr Thr Val Lys Asn Ile Leu Tyr Asp Tyr His Thr Ile
        115                 120                 125

Ser Leu Ala Asp Ser Asp Val Leu Gly Gly Gly Glu Cys Pro Val Val
    130                 135                 140

His His Asn Val Ser Phe Asp Glu Thr Trp Leu His Asn Pro Ser Ala
145                 150                 155                 160

Phe Asp Asn Leu Thr Phe Phe Phe Gly Cys His Trp Gly Pro Arg Asp
                165                 170                 175

Thr Leu Pro Glu Phe Ala Gly Asn Asn Ile Ser Cys Ala Gly Phe Ser
            180                 185                 190

Thr Pro Ala Ile Ser Gly Gly Gly Ser Phe Val Phe Lys Pro Glu Asp
        195                 200                 205

Leu Asp Glu His Ala Glu Gln Glu Leu Ala Ser His Cys Asp Glu Val
    210                 215                 220

Phe Ser Val Pro Val Arg Ser Glu Ala Leu Gln Gln Ala Ile Val Ser
225                 230                 235                 240

Asn Leu Ser Leu Gly Asp Gly Tyr Gly Glu Leu Leu Arg Gln Gly Ile
                245                 250                 255

Glu Leu Glu Trp Lys Arg Thr Ser Glu Asp Gln Cys Gly Gln Cys Glu
            260                 265                 270

Glu Ser Gly Ser Gly Gly Arg Cys Ala Tyr Ser Gln Lys Arg Glu Phe
        275                 280                 285

Leu Gly Cys Phe Cys Ser Gly Gly Lys Ala Gly Asn Pro Phe Cys Lys
    290                 295                 300

Pro Ser Arg Ser Thr Lys Arg Arg Glu Ala Ala Ser Ile Val Gly Ala
305                 310                 315                 320

Val Ala Val Ala Phe Leu Cys Leu Val Ile Leu Thr Cys Phe Leu Ala
                325                 330                 335

Cys Arg His Cys Ser Leu Pro Phe Lys Ser Lys Asn Lys Pro Gly Thr
            340                 345                 350

Arg Ile Glu Ser Phe Leu Gln Lys Asn Glu Ser Ser Ile His Pro Lys
        355                 360                 365

Arg Tyr Thr Tyr Ala Asp Val Lys Arg Met Thr Lys Ser Phe Ala Val
    370                 375                 380

Lys Leu Gly Gln Gly Gly Phe Gly Ala Val Tyr Lys Gly Ser Leu His

385                 390                 395                 400

Asp Gly Arg Gln Val Ala Val Lys Met Leu Lys Asp Thr Gln Gly Asp
                405                 410                 415

Gly Glu Glu Phe Met Asn Glu Val Ala Ser Ile Ser Arg Thr Ser His
            420                 425                 430

Val Asn Val Val Thr Leu Leu Gly Phe Cys Leu Gln Gly Ser Lys Arg
        435                 440                 445

Ala Leu Ile Tyr Glu Tyr Met Pro Asn Gly Ser Leu Glu Arg Tyr Ala
    450                 455                 460

Phe Thr Gly Asp Met Asn Ser Glu Asn Leu Leu Thr Trp Glu Arg Leu
465                 470                 475                 480

Phe Asp Ile Ala Ile Gly Thr Ala Arg Gly Leu Glu Tyr Leu His Arg
                485                 490                 495

Gly Cys Asn Thr Arg Ile Val His Phe Asp Ile Lys Pro His Asn Ile
            500                 505                 510

Leu Leu Asp Gln Asp Phe Cys Pro Lys Ile Ser Asp Phe Gly Leu Ala
        515                 520                 525

Lys Leu Cys Leu Asn Lys Glu Ser Ala Ile Ser Ile Ala Gly Ala Arg
    530                 535                 540

Gly Thr Ile Gly Tyr Ile Ala Pro Glu Val Tyr Ser Lys Gln Phe Gly
545                 550                 555                 560

Thr Ile Ser Ser Lys Ser Asp Val Tyr Ser Tyr Gly Met Met Val Leu
                565                 570                 575

Glu Met Val Gly Ala Arg Asp Arg Asn Thr Ser Ala Asp Ser Asp His
            580                 585                 590

Ser Ser Gln Tyr Phe Pro Gln Trp Leu Tyr Glu His Leu Asp Asp Tyr
        595                 600                 605

Cys Val Gly Ala Ser Glu Ile Asn Gly Glu Thr Thr Glu Leu Val Arg
    610                 615                 620

Lys Met Ile Val Val Gly Leu Trp Cys Ile Gln Val Ile Pro Thr Asp
625                 630                 635                 640

Arg Pro Thr Met Thr Arg Val Val Glu Met Leu Glu Gly Ser Thr Ser
                645                 650                 655

Asn Leu Glu Leu Pro Pro Arg Val Leu Leu Ser
            660                 665

<210>  21
<211>  2003
<212>  DNA
<213>  Artificial Sequence

<220>
<223>  cDNA of RLK1 allele from A188

<220>
<221>  misc_feature
<222>  (190)..(190)
<223>  n is a, c, g, or t

<220>
<221>  misc_feature
<222>  (262)..(262)
<223>  n is a, c, g, or t

<400>  21
atggctgctc acctaccacg cctccccgtc ctcctcctcg tcctcctcgc tgcccatgtc      60

gtctccacct ccgcccatgc cgagcctcct cttccgagcc cttacagcac ctccgcccat     120

ggcgagcctc ctcttccgag cacttacaac gtctccatgt gctcggaatc gttctggtgc     180

ggcggcgtcn aaatccgcta cccgttctat cttgccaacg caaccgccga ctacagcggg     240

agctactact cctgcggcta cnccgacttg agcgtttcct gcaaactcga ggtcgagggg     300

ccgacgacga catggacccc taccatccgt ctcggcggcg acaactacac cgtcaagaac     360

atcttgtacg actatcatac catctcactg gcggacagcg atgtgctcgg aggcggcgag     420

tgccccgtcg tccaccacaa cgtcagcttc gacgagacgt ggctgcacaa ccccagcgcc     480

ttcgacaacc tcaccttctt cttcggatgc cactgggggc cacgcgatac actgcctgaa     540

tttgccggca acaacatcag ctgcgccggg ttcagtactc cagctatcag cggtggaggc     600

tccttcgtgt tcaagcctga agatcttgac gaacatgcgg agcaggagtt ggcttcacac     660

tgcgacgagg ttttctccgt gccagtgaga agcgaggctc tgcagcaggc gatcgtcagc     720

aacctcagcc tcggggacgg gtacggcgag ctgcttaggc aggggatcga gttggaatgg     780

aaacggacat cggaggatca gtgtggccag tgcgaggaat cgggctccgg cggacggtgc     840

gcctacagcc agaagagaga attccttggc tgcttgtgca gcggagggaa ggcgggcaac     900

ccgttctgca aaccatcaag atcaacaaaa aggagagaag cagcatctat tgttggtgct     960

gttgccgttg cattcctgtg tctagtcatt ctcacatgct tcttggcttg tagacatggt    1020

tcgctgccct tcaaatcgga gaacaaacca gggacaagga ttgagtcctt cctacagaag    1080

aacgagagta tacatccgaa aagatacacc tacgcggacg tgaaaagaat gacaaaatcc 1140

ttcgctgtga agctaggcca aggtgggttt ggtgctgtat acaaaggcag cctccacgat 1200

ggccgacagg tagcagtcaa gatgctcaag gacacccaag gtgacggcga ggaattcatg 1260

aacgaggtgg ctagcatcag caggacttct catgtcaacg tcgtgacact tctagggttt 1320

tgcttgcaag ggtcgaaaag agcactgatc tacgagtaca tgcccaatgg ttcgctcgaa 1380

aggtatgcct tcaccggtga catgaacagt gagaatttgc taacctggga aagactattt 1440

gacatagcaa ttggcacacg gccagagggc tcgaatacct acaccgggga tgcaacactc 1500

ggatcgtgca ttttgacatc aagccacaca acatcctgtt agaccaggat ttctgcccta 1560

agatctctga ctttggactg gccaagctat gtctgaacaa agagagcgct atctccattg 1620

ctggcgcaag agggacgata gggtatatcg ccccggaggt ctactcaaag caatttggaa 1680

caataagcag caagtctgat gtctatagct atgggatgat ggtccttgag atggttggag 1740

caagggacag gaatacaagc gcagatagtg accatagcag ccaatatttc cctcagtggc 1800

tttatgagca tttggacgac tattgtgttg gtgcttccga gattaatggt gagaccacag 1860

agctcgtgag gaagatgata gttgtaggtc tgtggtgcat acaagtgatt ccgactgatc 1920

gaccaacaat gacgagagtc gtcgagatgt tggaaggaag cacaagtaat ctagagttgc 1980

cacccagagt tctcttgagc tga 2003

<210> 22
<211> 513
<212> PRT
<213> Zea mays

<220>
<221> misc_feature
<222> (64)..(64)
<223> Xaa can be any naturally occurring amino acid

<220>
<221> misc_feature
<222> (88)..(88)
<223> Xaa can be any naturally occurring amino acid

<400> 22

Met Ala Ala His Leu Pro Arg Leu Pro Val Leu Leu Leu Val Leu Leu
1               5                   10                  15

Ala Ala His Val Val Ser Thr Ser Ala His Ala Glu Pro Pro Leu Pro
            20                  25                  30

Ser Pro Tyr Ser Thr Ser Ala His Gly Glu Pro Pro Leu Pro Ser Thr
        35                  40                  45

Tyr Asn Val Ser Met Cys Ser Glu Ser Phe Trp Cys Gly Gly Val Xaa
    50                  55                  60

Ile Arg Tyr Pro Phe Tyr Leu Ala Asn Ala Thr Ala Asp Tyr Ser Gly
65                  70                  75                  80

Ser Tyr Tyr Ser Cys Gly Tyr Xaa Asp Leu Ser Val Ser Cys Lys Leu
                85                  90                  95

Glu Val Glu Gly Pro Thr Thr Thr Trp Thr Pro Thr Ile Arg Leu Gly
            100                 105                 110

Gly Asp Asn Tyr Thr Val Lys Asn Ile Leu Tyr Asp Tyr His Thr Ile
        115                 120                 125

Ser Leu Ala Asp Ser Asp Val Leu Gly Gly Gly Glu Cys Pro Val Val
    130                 135                 140

His His Asn Val Ser Phe Asp Glu Thr Trp Leu His Asn Pro Ser Ala
145                 150                 155                 160

Phe Asp Asn Leu Thr Phe Phe Phe Gly Cys His Trp Gly Pro Arg Asp
                165                 170                 175

Thr Leu Pro Glu Phe Ala Gly Asn Asn Ile Ser Cys Ala Gly Phe Ser
            180                 185                 190

Thr Pro Ala Ile Ser Gly Gly Gly Ser Phe Val Phe Lys Pro Glu Asp
        195                 200                 205

Leu Asp Glu His Ala Glu Gln Glu Leu Ala Ser His Cys Asp Glu Val
    210                 215                 220

Phe Ser Val Pro Val Arg Ser Glu Ala Leu Gln Gln Ala Ile Val Ser
225                 230                 235                 240

Asn Leu Ser Leu Gly Asp Gly Tyr Gly Glu Leu Leu Arg Gln Gly Ile
                245                 250                 255

Glu Leu Glu Trp Lys Arg Thr Ser Glu Asp Gln Cys Gly Gln Cys Glu
            260                 265                 270

Glu Ser Gly Ser Gly Gly Arg Cys Ala Tyr Ser Gln Lys Arg Glu Phe
        275                 280                 285

Leu Gly Cys Leu Cys Ser Gly Gly Lys Ala Gly Asn Pro Phe Cys Lys
    290                 295                 300

Pro Ser Arg Ser Thr Lys Arg Arg Glu Ala Ala Ser Ile Val Gly Ala
305                 310                 315                 320

Val Ala Val Ala Phe Leu Cys Leu Val Ile Leu Thr Cys Phe Leu Ala
                325                 330                 335

Cys Arg His Gly Ser Leu Pro Phe Lys Ser Glu Asn Lys Pro Gly Thr
            340                 345                 350

Arg Ile Glu Ser Phe Leu Gln Lys Asn Glu Ser Ile His Pro Lys Arg
        355                 360                 365

Tyr Thr Tyr Ala Asp Val Lys Arg Met Thr Lys Ser Phe Ala Val Lys
    370                 375                 380

Leu Gly Gln Gly Gly Phe Gly Ala Val Tyr Lys Gly Ser Leu His Asp
385                 390                 395                 400

Gly Arg Gln Val Ala Val Lys Met Leu Lys Asp Thr Gln Gly Asp Gly
                405                 410                 415

Glu Glu Phe Met Asn Glu Val Ala Ser Ile Ser Arg Thr Ser His Val
            420                 425                 430

Asn Val Val Thr Leu Leu Gly Phe Cys Leu Gln Gly Ser Lys Arg Ala
        435                 440                 445

Leu Ile Tyr Glu Tyr Met Pro Asn Gly Ser Leu Glu Arg Tyr Ala Phe
    450                 455                 460

Thr Gly Asp Met Asn Ser Glu Asn Leu Leu Thr Trp Glu Arg Leu Phe
465                 470                 475                 480

Asp Ile Ala Ile Gly Thr Arg Pro Glu Gly Ser Asn Thr Tyr Thr Gly
                485                 490                 495

Asp Ala Thr Leu Gly Ser Cys Ile Leu Thr Ser Ser His Thr Thr Ser
            500                 505                 510

Cys

<210>  23
<211>  2001
<212>  DNA
<213>  Artificial Sequence

<220>
<223>  cDNA of modified RLK1 allele from A188

<220>
<221>  misc_feature
<222>  (190)..(190)
<223>  n is a, c, g, or t

<220>
<221>  misc_feature
<222>  (262)..(262)
<223>  n is a, c, g, or t

<400>  23
atggctgctc acctaccacg cctccccgtc ctcctcctcg tcctcctcgc tgcccatgtc      60

gtctccacct ccgcccatgc cgagcctcct cttccgagcc cttacagcac ctccgcccat     120

ggcgagcctc ctcttccgag cacttacaac gtctccatgt gctcggaatc gttctggtgc     180

ggcggcgtcn aaatccgcta cccgttctat cttgccaacg caaccgccga ctacagcggg     240

agctactact cctgcggcta cnccgacttg agcgtttcct gcaaactcga ggtcgagggg     300

ccgacgacga catggacccc taccatccgt ctcggcggcg acaactacac cgtcaagaac     360

atcttgtacg actatcatac catctcactg gcggacagcg atgtgctcgg aggcggcgag     420

tgccccgtcg tccaccacaa cgtcagcttc gacgagacgt ggctgcacaa ccccagcgcc     480

ttcgacaacc tcaccttctt cttcggatgc cactgggggc cacgcgatac actgcctgaa     540

tttgccggca acaacatcag ctgcgccggg ttcagtactc cagctatcag cggtggaggc     600

tccttcgtgt tcaagcctga agatcttgac gaacatgcgg agcaggagtt ggcttcacac     660

tgcgacgagg ttttctccgt gccagtgaga agcgaggctc tgcagcaggc gatcgtcagc     720

aacctcagcc tcggggacgg gtacggcgag ctgcttaggc aggggatcga gttggaatgg     780

aaacggacat cggaggatca gtgtggccag tgcgaggaat cgggctccgg cggacggtgc     840

gcctacagcc agaagagaga attccttggc tgcttgtgca gcggagggaa ggcgggcaac     900

ccgttctgca aaccatcaag atcaacaaaa aggagagaag cagcatctat tgttggtgct     960

gttgccgttg cattcctgtg tctagtcatt ctcacatgct tcttggcttg tagacatggt    1020

tcgctgccct tcaaatcgga gaacaaacca gggacaagga ttgagtcctt cctacagaag    1080

aacgagagta tacatccgaa aagatacacc tacgcggacg tgaaaagaat gacaaaatcc    1140

ttcgctgtga agctaggcca aggtgggttt ggtgctgtat acaaaggcag cctccacgat    1200

ggccgacagg tagcagtcaa gatgctcaag gacacccaag gtgacggcga ggaattcatg    1260

aacgaggtgg ctagcatcag caggacttct catgtcaacg tcgtgacact tctagggttt    1320

tgcttgcaag ggtcgaaaag agcactgatc tacgagtaca tgcccaatgg ttcgctcgaa    1380

aggtatgcct tcaccggtga catgaacagt gagaatttgc taacctggga aagactattt    1440

gacatagcaa ttggcacggc cagagggctc gaatacctac accggggatg caacactcgg     1500

atcgtgcatt ttgacatcaa gccacacaac atcctgttag accaggattt ctgccctaag     1560

atctctgact ttggactggc caagctatgt ctgaacaaag agagcgctat ctccattgct     1620

ggcgcaagag ggacgatagg gtatatcgcc ccggaggtct actcaaagca atttggaaca     1680

ataagcagca agtctgatgt ctatagctat gggatgatgg tccttgagat ggttggagca     1740

agggacagga atacaagcgc agatagtgac catagcagcc aatatttccc tcagtggctt     1800

tatgagcatt tggacgacta ttgtgttggt gcttccgaga ttaatggtga gaccacagag     1860

ctcgtgagga agatgatagt tgtaggtctg tggtgcatac aagtgattcc gactgatcga     1920

ccaacaatga cgagagtcgt cgagatgttg gaaggaagca caagtaatct agagttgcca     1980

cccagagttc tcttgagctg a                                               2001


<210>  24
<211>  666
<212>  PRT
<213>  Artificial Sequence

<220>
<223>  modified RLK1 protein from A188


<220>
<221>  misc_feature
<222>  (64)..(64)
<223>  Xaa can be any naturally occurring amino acid

<220>
<221>  misc_feature
<222>  (88)..(88)
<223>  Xaa can be any naturally occurring amino acid

<400>  24

Met Ala Ala His Leu Pro Arg Leu Pro Val Leu Leu Leu Val Leu Leu
1               5                   10                  15


Ala Ala His Val Val Ser Thr Ser Ala His Ala Glu Pro Pro Leu Pro
            20                  25                  30


Ser Pro Tyr Ser Thr Ser Ala His Gly Glu Pro Pro Leu Pro Ser Thr
        35                  40                  45


Tyr Asn Val Ser Met Cys Ser Glu Ser Phe Trp Cys Gly Gly Val Xaa
    50                  55                  60


Ile Arg Tyr Pro Phe Tyr Leu Ala Asn Ala Thr Ala Asp Tyr Ser Gly
65                  70                  75                  80


Ser Tyr Tyr Ser Cys Gly Tyr Xaa Asp Leu Ser Val Ser Cys Lys Leu

                85                  90                  95

Glu Val Glu Gly Pro Thr Thr Thr Trp Thr Pro Thr Ile Arg Leu Gly
            100                 105                 110

Gly Asp Asn Tyr Thr Val Lys Asn Ile Leu Tyr Asp Tyr His Thr Ile
        115                 120                 125

Ser Leu Ala Asp Ser Asp Val Leu Gly Gly Gly Glu Cys Pro Val Val
    130                 135                 140

His His Asn Val Ser Phe Asp Glu Thr Trp Leu His Asn Pro Ser Ala
145                 150                 155                 160

Phe Asp Asn Leu Thr Phe Phe Phe Gly Cys His Trp Gly Pro Arg Asp
                165                 170                 175

Thr Leu Pro Glu Phe Ala Gly Asn Asn Ile Ser Cys Ala Gly Phe Ser
            180                 185                 190

Thr Pro Ala Ile Ser Gly Gly Gly Ser Phe Val Phe Lys Pro Glu Asp
        195                 200                 205

Leu Asp Glu His Ala Glu Gln Glu Leu Ala Ser His Cys Asp Glu Val
    210                 215                 220

Phe Ser Val Pro Val Arg Ser Glu Ala Leu Gln Gln Ala Ile Val Ser
225                 230                 235                 240

Asn Leu Ser Leu Gly Asp Gly Tyr Gly Glu Leu Leu Arg Gln Gly Ile
                245                 250                 255

Glu Leu Glu Trp Lys Arg Thr Ser Glu Asp Gln Cys Gly Gln Cys Glu
            260                 265                 270

Glu Ser Gly Ser Gly Gly Arg Cys Ala Tyr Ser Gln Lys Arg Glu Phe
        275                 280                 285

Leu Gly Cys Leu Cys Ser Gly Gly Lys Ala Gly Asn Pro Phe Cys Lys
    290                 295                 300

Pro Ser Arg Ser Thr Lys Arg Arg Glu Ala Ala Ser Ile Val Gly Ala
305                 310                 315                 320

Val Ala Val Ala Phe Leu Cys Leu Val Ile Leu Thr Cys Phe Leu Ala
                325                 330                 335

Cys Arg His Gly Ser Leu Pro Phe Lys Ser Glu Asn Lys Pro Gly Thr
        340                 345                 350

Arg Ile Glu Ser Phe Leu Gln Lys Asn Glu Ser Ile His Pro Lys Arg
    355                 360                 365

Tyr Thr Tyr Ala Asp Val Lys Arg Met Thr Lys Ser Phe Ala Val Lys
    370                 375                 380

Leu Gly Gln Gly Gly Phe Gly Ala Val Tyr Lys Gly Ser Leu His Asp
385                 390                 395                 400

Gly Arg Gln Val Ala Val Lys Met Leu Lys Asp Thr Gln Gly Asp Gly
                405                 410                 415

Glu Glu Phe Met Asn Glu Val Ala Ser Ile Ser Arg Thr Ser His Val
            420                 425                 430

Asn Val Val Thr Leu Leu Gly Phe Cys Leu Gln Gly Ser Lys Arg Ala
        435                 440                 445

Leu Ile Tyr Glu Tyr Met Pro Asn Gly Ser Leu Glu Arg Tyr Ala Phe
    450                 455                 460

Thr Gly Asp Met Asn Ser Glu Asn Leu Leu Thr Trp Glu Arg Leu Phe
465                 470                 475                 480

Asp Ile Ala Ile Gly Thr Ala Arg Gly Leu Glu Tyr Leu His Arg Gly
                485                 490                 495

Cys Asn Thr Arg Ile Val His Phe Asp Ile Lys Pro His Asn Ile Leu
            500                 505                 510

Leu Asp Gln Asp Phe Cys Pro Lys Ile Ser Asp Phe Gly Leu Ala Lys
        515                 520                 525

Leu Cys Leu Asn Lys Glu Ser Ala Ile Ser Ile Ala Gly Ala Arg Gly
    530                 535                 540

Thr Ile Gly Tyr Ile Ala Pro Glu Val Tyr Ser Lys Gln Phe Gly Thr
545                 550                 555                 560

Ile Ser Ser Lys Ser Asp Val Tyr Ser Tyr Gly Met Met Val Leu Glu
                565                 570                 575

Met Val Gly Ala Arg Asp Arg Asn Thr Ser Ala Asp Ser Asp His Ser
            580                 585                 590

Ser Gln Tyr Phe Pro Gln Trp Leu Tyr Glu His Leu Asp Asp Tyr Cys
        595                 600                 605

Val Gly Ala Ser Glu Ile Asn Gly Glu Thr Thr Glu Leu Val Arg Lys
    610                 615                 620

Met Ile Val Val Gly Leu Trp Cys Ile Gln Val Ile Pro Thr Asp Arg
625                 630                 635                 640

Pro Thr Met Thr Arg Val Val Glu Met Leu Glu Gly Ser Thr Ser Asn
                645                 650                 655

Leu Glu Leu Pro Pro Arg Val Leu Leu Ser
            660                 665

<210>  25
<211>  47
<212>  DNA
<213>  Artificial Sequence

<220>
<223>  SYN14136 Primer Allele X

<400>  25
gaaggtgacc aagttcatgc tcatcttgtt cggccttttc tacacta                47

<210>  26
<211>  46
<212>  DNA
<213>  Artificial Sequence

<220>
<223>  SYN14136 Primer Allele Y

<400>  26
gaaggtcgga gtcaacggat tatcttgttc ggccttttct acactc                 46

<210>  27
<211>  28
<212>  DNA
<213>  Artificial Sequence

<220>
<223>  SYN14136 Common Primer

<400>  27
agaaccaaaa attcaccagc tgtgagaa                                     28

<210>  28
<211>  36
<212>  DNA
<213>  Artificial Sequence

<220>

<223>  PZE-108077560 Primer Allele X

<400>  28
gaaggtgacc aagttcatgc tgcagtggcc tgcgca                                36


<210>  29
<211>  39
<212>  DNA
<213>  PZE-108077560 Primer Allele Y

<400>  29
gaaggtcgga gtcaacggat tgctgcagtg gcctgcgcg                             39


<210>  30
<211>  28
<212>  DNA
<213>  Artificial Sequence

<220>
<223>  PZE-108077560 Common Primer

<400>  30
agataatgag tcatcaggct atcagcaa                                         28


<210>  31
<211>  49
<212>  DNA
<213>  Artificial Sequence

<220>
<223>  PZE-108093423 Primer Allele X

<400>  31
gaaggtgacc aagttcatgc tgtaatccat tgtgaacata tcgctatca                  49


<210>  32
<211>  47
<212>  DNA
<213>  Artificial Sequence

<220>
<223>  PZE-108093423 Primer Allele Y

<400>  32
gaaggtcgga gtcaacggat taatccattg tgaacatatc gctatcg                    47


<210>  33
<211>  25
<212>  DNA
<213>  Artificial Sequence

<220>
<223>  PZE-108093423 Common Primer

<400>  33
gaaacggttc tgctgcagtt tggta                                            25

<210>  34
<211>  42
<212>  DNA
<213>  Artificial Sequence

<220>
<223>  MA0021 Primer Allele X

<400>  34
gaaggtgacc aagttcatgc tgagctcatc tcgtccaagc cc                          42


<210>  35
<211>  42
<212>  DNA
<213>  Artificial Sequence

<220>
<223>  MA0021 Primer Allele Y

<400>  35
gaaggtcgga gtcaacggat tgagctcatc tcgtccaagc cg                          42


<210>  36
<211>  19
<212>  DNA
<213>  Artificial Sequence

<220>
<223>  MA0021 Common Primer

<400>  36
cgctgcggtg ccgggtgat                                                    19


<210>  37
<211>  47
<212>  DNA
<213>  Artificial Sequence

<220>
<223>  MA0022 Primer Allele X

<400>  37
gaaggtgacc aagttcatgc tcaccaacac aatagtcgtc caaatgt                     47


<210>  38
<211>  46
<212>  DNA
<213>  Artificial Sequence

<220>
<223>  MA0022 Primer Allele Y

<400>  38
gaaggtcgga gtcaacggat taccaacaca atagtcgtcc aaatgc                      46


<210>  39
<211>  25
<212>  DNA

<213>  Artificial Sequence

<220>
<223>  MA0022 Common Primer

<400>  39
cagccaatat ttccctcagt ggctt                                          25


<210>  40
<211>  47
<212>  DNA
<213>  Artificial Sequence

<220>
<223>  SYN4196 Primer Allele X

<400>  40
gaaggtgacc aagttcatgc tctgcactct ggaatatcta taacaga                  47


<210>  41
<211>  47
<212>  DNA
<213>  Artificial Sequence

<220>
<223>  SYN4196 Primer Allele Y

<400>  41
gaaggtcgga gtcaacggat tctgcactct ggaatatcta taacagc                  47


<210>  42
<211>  25
<212>  DNA
<213>  Artificial Sequence

<220>
<223>  SYN4196 Common Primer

<400>  42
tggtttatac caggaaggga cgcat                                          25